# EUROPEAN PATENT APPLICATION

(11) **EP 4 563 692 A1**
(43) Date of publication of application: **04.06.2025**
(21) Application number: 23846697.3
(22) Date of filing: 28.07.2023
(51) Int. Cl.: C12M 3/00, C12M 1/02, C12M 3/06

(54) **CULTURE SYSTEM**

(30) Priority: 29.07.2022 JP 2022121934
(71) Applicant: Nikkiso Co., Ltd., Tokyo 150-6022 (JP)
(72) Inventor: KONDO, Masayuki, Kanazawa-shi, Ishikawa 920-0177 (JP); JIMBO, Yoichi, Kanazawa-shi, Ishikawa 920-0177 (JP); KITAGAWA, Fumihiko, Kanazawa-shi, Ishikawa 920-0177 (JP); HORIOKA, Satoru, Kanazawa-shi, Ishikawa 920-0177 (JP); MAEDA, Hayata, Kanazawa-shi, Ishikawa 920-0177 (JP)
(74) Representative: Hoffmann Eitle
(86) International application number: PCT/JP2023/027842
(87) International publication number: WO 2024/024967

(57) **Abstract**

Provided is a culture system capable of generating a sufficiently grown cell aggregate. A culture system that forms a cell aggregate by liquid culture includes: a culture portion that contains a liquid and has an outlet through which the liquid is discharged and an inlet through which the liquid is introduced; a path portion that allows the liquid to flow between the outlet and the inlet; a monitoring portion capable of monitoring a component in the liquid and/or properties of the liquid in at least one of the culture portion and the path portion; an adjustment portion capable of adjusting the component in the liquid and/or the properties of the liquid in at least one of the culture portion and the path portion; and a control portion capable of controlling the adjustment portion on the basis of a monitoring result of the monitoring portion.

## Description

### Technical Field

The present invention relates to a culture system.

### Background Art

A system that cultures cells is known (see, for example, Patent Literature 1).

### Citation List

### Patent Literature

Patent Literature 1: WO 2013/161885 A

### Summary of Invention

### Technical Problem

The conventional cell culture system described above is on the basis of use of a filter that allows a cell culture solution to pass therethrough and does not allow cells or cell aggregates to pass therethrough. For this reason, there is a possibility that the cells or the cell aggregates are locally pressed against the filter, and the cells or the cell aggregates are damaged or divided.

The present invention has been made in view of the above points. An object of the present invention is to provide a culture system capable of accurately growing a cell aggregate.

### Solution to Problem

A culture system according to the present invention is characterized by
a culture system that forms a cell aggregate by liquid culture, the culture system including:
a culture portion that contains a liquid and has an outlet through which the liquid is led out and an inlet through which the liquid is introduced;
a path portion that allows the liquid to flow between the outlet and the inlet;
a monitoring portion capable of monitoring a component in the liquid and/or properties of the liquid in at least one of the culture portion and the path portion;
an adjustment portion capable of adjusting the component in the liquid and/or the properties of the liquid in at least one of the culture portion and the path portion; and
a control portion capable of controlling the adjustment portion on the basis of a monitoring result of the monitoring portion.

### Advantageous Effects of Invention

A cell aggregate can be accurately grown.

### Brief Description of Drawings

[FIG. 1] Fig. 1 is a block diagram illustrating an outline of a culture system 1.
[FIG. 2] Fig. 2 is a block diagram illustrating details of a culture system 1-1 according to a first embodiment.
[FIG. 3] Fig. 3 is a block diagram illustrating details of a culture system 1-2 according to a second embodiment.
[FIG. 4] Fig. 4 is a schematic diagram illustrating positions of an inlet 227-2 and an outlet 231-2 in a culture vessel 110-2.
[FIG. 5] Fig. 5 is a block diagram illustrating various sensors included in a monitoring device 300-2.
[FIG. 6] Fig. 6 is a time chart illustrating an outline of treatment of the culture system 1-2.
[FIG. 7] Fig. 7 is a schematic diagram illustrating a state of cell aggregates that grow by the treatment of the culture system 1-2.
[FIG. 8] Fig. 8 is a block diagram illustrating details of a culture system 1-3 according to a third embodiment.
[FIG. 9] Fig. 9 is a time chart illustrating an outline of treatment of the culture system 1-3.
[FIG. 10] Fig. 10 is a graph illustrating a process of subjecting cell aggregates to a classification treatment and then subjecting the cell aggregates to a concentration recovery treatment by a change in a particle size distribution of the aggregates.
[FIG. 11] Fig. 11 is a schematic diagram illustrating a configuration of a classification system 700 for classifying cell aggregates.
[FIG. 12] Fig. 12 is a schematic diagram illustrating a configuration of a concentration recovery system 800 for concentrating and recovering cell aggregates.
[FIG. 13] Fig. 13 is a block diagram illustrating a culture classification system 4-1 in which a classification module 720 for classifying cell aggregates is incorporated into the culture system 1-2 (Fig. 3).
[FIG. 14] Fig. 14 is a time chart illustrating a classification treatment in the culture classification system 4-1 illustrated in Fig. 13.
[FIG. 15] Fig. 15 is a block diagram illustrating a configuration of a culture classification concentration recovery system 4-2.
[FIG. 16] Fig. 16 is a block diagram illustrating a concentration recovery system 5-1 for culturing and classifying cell aggregates by the culture classification system 4-1 illustrated in Fig. 13, then moving a culture tank 110, and concentrating and recovering the cell aggregates.
[FIG. 17] Fig. 17 is a time chart illustrating a procedure of a concentration recovery treatment.
[FIG. 18] Fig. 18 is a block diagram illustrating a culture classification concentration recovery system 6-1 in which the classification system 700 and the concentration recovery system 800 are incorporated into the culture system 1-3 (Fig. 8).

### Description of Embodiments

### <<<<Outline of culture system 1>>>

A culture system 1 is a culture system that cultures cells. Specifically, the culture system 1 is a system that forms cell aggregates with cells while growing the cells. Note that not only cells are sequentially aggregated, but also some of cells that have been once aggregated may be separated. As a whole, it is only required to be able to finally form a cell aggregate having a desired size. Note that a too large cell aggregate is not good. A size with which nutrients can be supplied to cells inside the cell aggregate is preferable.

### <<<Cell>>>

A cell is an object to be cultured by the culture system 1. By culturing a cell, the cell is divided to increase the number of cells. By contact between cells, a cell aggregate (cell aggregated body) is formed. The culture system 1 is a system that forms a cell aggregate while growing the cell.

The cell is not particularly limited as long as it forms a cell aggregate in a medium. The cell is preferably derived from a mammal, and is preferably derived from a species generally used for studies of primates such as humans and monkeys, mice, and the like. Examples of the cell include a cell used for studies on regenerative medicine and a cell used as a cell preparation, and specific examples thereof include pluripotent stem cells such as an ES cell and an iPS cell, various precursor cells such as a nephron progenitor cell, a ureteric bud cell, and a stromal precursor cell, and various stem cells such as a mesenchymal stem cell, a neural stem cell, and an adipose stem cell. The cell may be a cell derived from a pluripotent stem cell, an immortalized cell, or an established cell, or may be a primary cultured cell isolated from a tissue. In addition, the cell may be a normal cell or a cell having a disease, a genetic abnormality, or a transgene depending on a purpose.

### <<<Medium>>>

The medium used in the culture system 1 includes a new medium and a circulation feed medium. The new medium is a medium newly supplied to a culture vessel 110-1 (reactor) described later or the like. The circulation feed medium is a medium that is fed in a circulation manner for circulation or disposal among media that have already been supplied to the culture vessel 110-1 or the like and used for culture.

An appropriate medium can be selected and used depending on a cell, and the medium is not particularly limited. In addition, conventionally known materials and additives useful for cell culture can be appropriately used. Examples of a basal medium include DMEM, DMEMHG, EMEM, Iscove's Modified Dulbecco's Medium (IMDM), Glasgow's MEM (GMEM), RPMI-1640, α-MEM, Ham's Medium F-12, Ham's Medium F-10, Ham's Medium F12K, etc. It is preferable to add, as additives, an amino acid, a vitamin, an inorganic salt, a protein (growth factor), glucose, an antibiotic, a signal transduction inhibitor, a reducing agent, a buffer, and the like. Furthermore, serum (preferably serum derived from a mammal (for example, fetal bovine serum, human serum, or the like)) may be added. As an alternative to serum, a supplement can also be used.

### <<<Cell suspension>>>

A cell suspension is a system in which cells and cell aggregates are dispersed in a medium. In the culture vessel 110-1, cells, cell aggregates, and a medium are stored as a cell suspension.

### <<<Stirring>>>

Stirring means displacing cells, cell aggregates, and a medium in a cell suspension. By stirring, cells and cell aggregates are displaced together with displacement of a medium. The cells and the cell aggregates are also displaced mainly along with the displacement (convection) of the medium. Note that operation on the cell suspension only needs to be able to displace the cells, and may be operation such as shaking as well as stirring.

### <<<Convection>>>

Convection refers to a phenomenon in which cells and cell aggregates are carried by a flow of a medium.

### <<<Floating>>

Floating mainly means a state in which cells and cell aggregates are not in contact with a wall surface of a culture vessel (for example, the culture vessel 110-1 described later). The wall surface of the culture vessel includes all surfaces with which cells and cell aggregates may come into contact. For example, when the culture vessel has a cylindrical shape or a square cylindrical shape, the wall surface is a bottom surface and an inner surface of a side surface. When the culture vessel has a spherical shape, the wall surface is an inner surface of a spherical surface. Note that a state in which a part of cells and cell aggregates can be easily separated from a wall surface of a culture vessel due to displacement of a medium even while the part of cells and cell aggregates is in contact with the wall surface is also included in floating.

### <<<Shear stress>>>

Shear stress refers to a stress caused by a difference in relative displacement between a displaced medium and cells or cell aggregates.

### <<<Aggregation>>>

Aggregation refers to formation of a large mass by aggregation of cells. Note that even when cells are once aggregated, cells and small aggregates may be separated by shear stress or the like in a culture process. Even when a case of separation is included, it is only required to be able to perform culture such that a cell aggregate finally has a large size. Note that the size of the cell aggregate is not limited. A state in which at least two cells are aggregated is included in the cell aggregate. A state in which cells are aggregated may be referred to as a cell aggregated body.

### <<<<Configuration of culture system 1>>>>

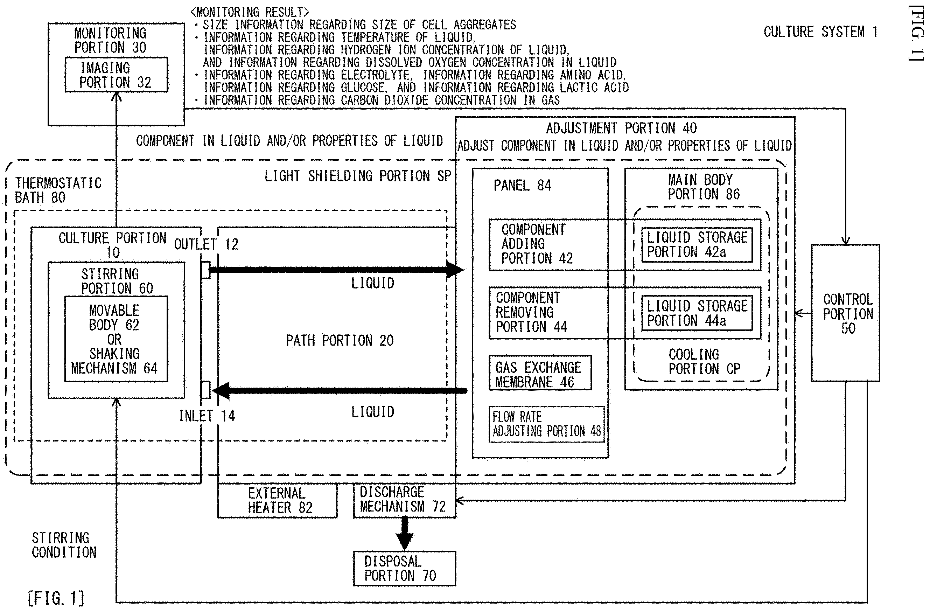

Fig. 1 is a block diagram illustrating an outline of a configuration of the culture system 1.

### <<Culture system 1 according to first aspect>>

A culture system 1 according to a first aspect provides a culture system 1 that forms a cell aggregate by liquid culture, the culture system 1 including:
a culture portion 10 that contains a liquid and has an outlet 12 through which the liquid is led out and an inlet 14 through which the liquid is introduced;
a path portion 20 that allows the liquid to flow between the outlet 12 and the inlet 14;
a monitoring portion 30 capable of monitoring a component in the liquid and/or properties of the liquid in at least one of the culture portion 10 and the path portion 20;
an adjustment portion 40 capable of adjusting the component in the liquid and/or the properties of the liquid in at least one of the culture portion 10 and the path portion 20; and
a control portion 50 capable of controlling the adjustment portion 40 on the basis of a monitoring result of the monitoring portion 30.

The culture system 1 is a system for forming a cell aggregate by liquid culture.

The culture system 1 includes:
the culture portion 10;
the path portion 20;
the monitoring portion 30;
the adjustment portion 40; and
the control portion 50.

### <Culture portion 10>

The culture portion 10 contains a liquid. The culture portion 10 includes the outlet 12 and the inlet 14. The outlet 12 is a port through which a liquid is discharged from the culture portion 10. The inlet 14 is a port for introducing a liquid into the culture portion 10.

### <Liquid>

The liquid only needs to be a flowable medium that can be contained in the culture portion 10, can be led out from the outlet 12, and can be introduced from the inlet 14. For example, the liquid can be a medium, a cell suspension, or the like.

### <Path portion 20>

The path portion 20 allows a liquid to flow between the outlet 12 and the inlet 14. The path portion 20 allows the outlet 12 and the inlet 14 to communicate with each other. The path portion 20 can allow a liquid to flow between the outlet 12 and the inlet 14.

### <Monitoring portion 30>

The monitoring portion 30 can monitor a component in a liquid and/or properties of the liquid. The monitoring portion 30 can monitor at least one of the component in the liquid and the properties of the liquid. The monitoring portion 30 can monitor the component in the liquid or the properties of the liquid in at least one of the culture portion 10 and the path portion 20.

### <Adjustment portion 40>

The adjustment portion 40 can adjust a component in a liquid and/or properties of the liquid. The adjustment portion 40 can adjust at least one of the component in the liquid and/or the properties of the liquid. The adjustment portion 40 can adjust the component in the liquid or the properties of the liquid in at least one of the culture portion 10 and the path portion 20.

### <Control portion 50>

The control portion 50 can control the adjustment portion 40 on the basis of a monitoring result of the monitoring portion 30.

Since a component in a liquid or properties of the liquid is monitored and the component in the liquid or the properties of the liquid is adjusted on the basis of a monitoring result thereof, it is possible to accurately grow cells to form cell aggregates.

### <<Culture system 1 according to second aspect>>

A culture system 1 according to a second aspect is the culture system 1 according to the first aspect,
further including a stirring portion 60 that stirs the liquid in the culture portion 10, in which
the control portion 50 controls the stirring portion 60 on the basis of a predetermined stirring condition.

### <<Culture system 1 according to third aspect>>

A culture system 1 according to a third aspect is the culture system 1 according to the second aspect, in which
the liquid culture is floating culture in which cells in the liquid are floated by stirring by the stirring portion 60.

### <<Culture system 1 according to fourth aspect>>

A culture system 1 according to a fourth aspect is the culture system 1 according to the second aspect, in which
the stirring portion 60 includes a movable body 62 that causes a liquid to flow.

### <<Culture system 1 according to fifth aspect>>

A culture system 1 according to a fifth aspect is the culture system 1 according to the second aspect, in which
the stirring portion 60 includes a shaking mechanism 64 that shakes the culture portion 10.

### <<Culture system 1 according to sixth aspect>>

A culture system 1 according to a sixth aspect is the culture system 1 according to the second aspect, in which
the predetermined stirring condition is a condition determined in advance on the basis of nucleation of cells and the size of the cell aggregate.

As described above, the monitoring portion 30 monitors at least one of a component in a liquid and properties of the liquid. As a result of monitoring, the monitoring portion 30 outputs various types of information such as at least one piece of properties information, at least one piece of component information, and size information regarding the size of the cell aggregate. The predetermined stirring condition is determined on the basis of various types of information output from the monitoring portion 30.

For example, as described later, the monitoring portion 30 includes an imaging portion 32 that images a cell aggregate. The imaging portion 32 outputs size information capable of determining the size of the cell aggregate. The control portion 50 acquires the size information output from monitoring portion 30. The control portion 50 can determine the size of the cell aggregate from the size information. The control portion 50 determines a predetermined stirring condition on the basis of the size of the cell aggregate. For example, a stirring rate is determined depending on the size of the cell aggregate.

### <Stirring portion 60>

The culture system 1 further includes the stirring portion 60. The stirring portion 60 stirs a liquid in the culture portion 10. The stirring portion 60 stirs the liquid in the culture portion 10 under a predetermined stirring condition. The control portion 50 controls and operates the stirring portion 60 so as to satisfy the predetermined stirring condition.

The stirring portion 60 may include the movable body 62 that causes a liquid to flow. The stirring portion 60 may include the shaking mechanism 64 that shakes the culture portion 10. The stirring portion 60 only needs to be a portion that fluctuates (vibrates, displaces, or the like) a liquid in order to appropriately form a cell aggregate.

The predetermined stirring condition is a condition for appropriately forming a cell aggregate. Note that the predetermined stirring condition varies depending on a stage of culture. The predetermined stirring condition is a condition for increasing a possibility of contact between cells, dispersing formed cell aggregates so as not to adhere to each other (enlarge), and preventing shear stress applied to the formed cell aggregates from increasing. By setting the predetermined stirring condition in this way, first, the possibility of contact between cells can be increased, and cell aggregates can be easily formed. Furthermore, by appropriately dispersing the formed cell aggregates, it is possible to prevent adhesion between the cell aggregates (enlargement). Furthermore, by preventing the shear stress applied to the cell aggregates from increasing, the formed cell aggregates can be prevented from being divided.

The predetermined stirring condition is determined in advance on the basis of nucleation of cells and the size of the cell aggregate. The predetermined stirring condition can be varied depending on the degree of nucleation of cells and the size of the cell aggregate. An appropriate stirring condition according to growth of the cell aggregates can be adopted. The predetermined stirring condition is determined in advance by a preliminary experiment or the like, and is stored in an auxiliary storage device or the like (not illustrated). The control portion 50 reads the predetermined stirring condition from the auxiliary storage device and controls the stirring portion 60.

The monitoring portion 30 outputs various types of information such as at least one piece of properties information, at least one piece of component information, and size information regarding the size of the cell aggregate. The predetermined stirring condition is determined on the basis of various types of information output from the monitoring portion 30. For example, as described later, the monitoring portion 30 includes the imaging portion 32 that images a cell aggregate. The imaging portion 32 outputs size information capable of determining the size of the cell aggregate. In addition, the monitoring portion 30 measures a concentration of glucose or lactic acid by optical analysis, for example, Raman spectroscopy. The control portion 50 acquires the size information output from the monitoring portion 30. The control portion 50 can determine the size of the cell aggregate from the size information. The control portion 50 determines the predetermined stirring condition on the basis of the size of the cell aggregate. For example, when the size of the cell aggregate is equal to or larger than a predetermined size, the stirring rate is determined depending on the size of the cell aggregate.

### <<Culture system 1 according to seventh aspect>>

A culture system 1 according to a seventh aspect is the culture system 1 according to any one of the first to sixth aspects, further including
a thermostatic bath 80 capable of housing at least a part of the culture portion 10 and the path portion 20 therein.

### <Thermostatic bath 80>

The thermostatic bath 80 can house at least a part of at least one of the culture portion 10 and the path portion 20. By storing the culture portion 10 or the path portion 20 in the thermostatic bath 80, a cell aggregate can be formed in a stable environment. Note that the thermostatic bath 80 does not have to be used as long as a cell aggregate can be formed in a stable environment.

### <<Culture system 1 according to eighth aspect>>

A culture system 1 according to an eighth aspect is the culture system 1 according to any one of the first to seventh aspects, further including
an external heater 82 capable of heating the liquids in the culture portion 10 and the path portion 20 from the outside.

### <External heater 82>

The external heater 82 can heat the liquid in the culture portion 10 and the liquid in the path portion 20 from the outside. By heating the liquid by the external heater 82, an environment suitable for forming the cell aggregate can be obtained.

### <<Culture system 1 according to ninth aspect>>

A culture system 1 according to a ninth aspect is the culture system 1 according to any one of the first to eighth aspects, in which
the outlet 12 is higher than a liquid level of the liquid at the time of start of culture.

At the time of starting culture, the outlet 12 is higher than a liquid level of the liquid contained in the culture portion 10. In other words, at the time of starting culture, the liquid is contained in the culture portion 10 such that the liquid level of the liquid is lower than the outlet 12.

By a filter, a physical mechanism, or the like, it is possible to prevent ungrown cells and small cell aggregates from being discharged from the outlet 12.

### <<Culture system 1 according to tenth aspect>>

A culture system 1 according to a tenth aspect is the culture system 1 according to any one of the first to ninth aspects, in which
the monitoring portion 30 is capable of outputting, as information regarding the properties of the liquid, at least one piece of properties information among information regarding a temperature of the liquid, information regarding a hydrogen ion concentration of the liquid, and information regarding a dissolved oxygen concentration in the liquid, and
the control portion 50 acquires the at least one piece of properties information output from the monitoring portion 30.

### <Information regarding properties of liquid (properties information)>

Examples of the information regarding the properties of the liquid include:
information regarding a temperature of the liquid;
information regarding a hydrogen ion concentration of the liquid; and
information regarding a dissolved oxygen concentration in the liquid.

### <Control of control portion 50>

The monitoring portion 30 can output at least one piece of properties information among these pieces of information. The properties information output from the monitoring portion 30 is supplied to the control portion 50. The control portion 50 can acquire at least one piece of properties information among these pieces of information. The control portion 50 can control the adjustment portion 40, the stirring portion 60, and the like using the acquired at least one piece of properties information.

### <<Culture system 1 according to eleventh aspect>>

A culture system 1 according to an eleventh aspect is the culture system 1 according to any one of the first to tenth aspects, in which
the monitoring portion 30 is capable of outputting, as information regarding the component in the liquid, at least one piece of component information among information regarding an electrolyte, information regarding an amino acid, information regarding glucose, and information regarding lactic acid, and
the control portion 50 acquires the at least one piece of component information output from the monitoring portion 30.

The monitoring portion 30 may be able to output information regarding ammonia as the information regarding the component in the liquid.

### <Information regarding component in liquid>

Examples of the information regarding the component in the liquid (component information) include:
information regarding an electrolyte;
information regarding an amino acid;
information regarding glucose; and
information regarding lactic acid.

### <Control of control portion 50>

The monitoring portion 30 can output at least one piece of component information among these pieces of information. The component information output from the monitoring portion 30 is supplied to the control portion 50. The control portion 50 can acquire at least one piece of component information among these pieces of information. The control portion 50 can control the adjustment portion 40, the stirring portion 60, and the like using the acquired at least one piece of component information.

### <<Culture system 1 according to twelfth aspect>>

A culture system 1 according to a twelfth aspect is the culture system 1 according to any one of the first to eleventh aspects, in which
the monitoring portion 30 is capable of outputting size information regarding the size of the cell aggregate in the culture portion 10, and
the control portion 50 acquires the size information output from the monitoring portion 30.

The monitoring portion 30 can output size information regarding the size of the cell aggregate present in the culture portion 10. The size information output from the monitoring portion 30 is supplied to the control portion 50. The control portion 50 can acquire the size information. The control portion 50 can control the adjustment portion 40, the stirring portion 60, and the like using the acquired size information.

<<Culture system 1 according to thirteenth aspect>>

A culture system 1 according to a thirteenth aspect is the culture system 1 according to the twelfth aspect, in which
the monitoring portion 30 includes the imaging portion 32 that images a cell aggregate in the culture portion 10, and
the control portion 50 acquires the size of the cell aggregate as the size information from imaging data of the cell aggregate.

The monitoring portion 30 includes the imaging portion 32. The imaging portion 32 images a cell aggregate in the culture portion 10. The imaging portion 32 can image not only the cell aggregate but also a liquid or the like together with the cell aggregate. The imaging portion 32 outputs the size information. The size information can be, for example, imaging data obtained by the imaging portion 32 imaging the cell aggregate or the like. The size information only needs to be information including data that can determine the size of the cell aggregate.

The control portion 50 acquires the size information output from the monitoring portion 30. The control portion 50 determines the size of the cell aggregate by performing various types of image processing and the like on the acquired size information. For example, the control portion 50 can determine the size of the cell aggregate by extracting a contour of the cell aggregate or the like by various types of image processing.

In addition, the control portion 50 may determine the size of the cell aggregate by performing machine learning in advance using various pieces of imaging data obtained by imaging the culture portion 10 and an actual measurement value of the size of the cell aggregate corresponding to each of these pieces of imaging data as teacher data, and referring to a learning result from the imaging data.

Note that, in a case where the imaging portion 32 has a function of being able to execute various types of image processing, various types of arithmetic processing, and the like (for example, a smart camera), the imaging portion 32 can determine the size of the cell aggregate from the imaging data and can output the size of the cell aggregate. In this case, the size information is information indicating the size of the cell aggregate. The control portion 50 can directly acquire the size of the cell aggregate.

### <<Culture system 1 according to fourteenth aspect>>

A culture system 1 according to a fourteenth aspect is the culture system 1 according to any one of the first to thirteenth aspects, in which
the adjustment portion 40 includes a component adding portion 42 that adds a necessary component to a liquid and/or a component removing portion 44 that removes an unnecessary component from the liquid, and
the control portion 50 controls the component adding portion 42 and/or the component removing portion 44 on the basis of the monitoring result of the monitoring portion 30 to adjust the component in the liquid and/or the properties of the liquid.

The adjustment portion 40 includes the component adding portion 42 and/or the component removing portion 44. The adjustment portion 40 includes at least one of the component adding portion 42 and the component removing portion 44. The component adding portion 42 adds a necessary component to the liquid. The component removing portion 44 removes an unnecessary component from the liquid.

The control portion 50 controls the component adding portion 42 and the component removing portion 44 on the basis of the monitoring result output from the monitoring portion 30. By control of the component adding portion 42 and the component removing portion 44, the component in the liquid or the properties of the liquid can be adjusted. By adjusting the component in the liquid or the properties of the liquid, an environment of the culture portion 10 can be brought into a state appropriate for growth of the cell aggregate.

### <<Culture system 1 according to fifteenth aspect>>

A culture system 1 according to a fifteenth aspect is the culture system 1 according to any one of the first to fourteenth aspects, in which
the adjustment portion 40 includes a gas exchange membrane 46 capable of supplying a gas component necessary for culture to the liquid.

The adjustment portion 40 includes the gas exchange membrane 46 (for example, a gas exchange module 416-1 described later). The gas exchange membrane 46 can supply a gas component necessary for culture to the liquid. By supply of the gas component necessary for culture, an environment of the culture portion 10 can be brought into a state appropriate for growth of the cell aggregate.

### <<Culture system 1 according to sixteenth aspect>>

A culture system 1 according to a sixteenth aspect is the culture system 1 according to the fifteenth aspect, in which
the gas exchange membrane 46 is a hollow fiber gas exchange membrane containing any one of silicone, polypropylene, polytetrafluoroethylene, and polymethylpentene.

Since the hollow fiber gas exchange membrane is used, use for a long period of time is possible due to the homogeneous membrane that is less likely to be clogged, and the system can be downsized.

### <<Culture system 1 according to seventeenth aspect>>

A culture system 1 according to a seventeenth aspect is the culture system 1 according to any one of the first to sixteenth aspects, in which
the adjustment portion 40 includes a dialysis membrane.

The adjustment portion 40 includes the dialysis membrane (for example, a dialysis module 412-1 or a dialysis module 472-3 described later), and therefore adjusts a medium by continuously correcting a composition of nutrients or metabolites or reusing growth factors, and can use the medium for a long period of time.

### <<Culture system 1 according to eighteenth aspect>>

A culture system 1 according to an eighteenth aspect is the culture system 1 according to any one of the first to seventeenth aspects, in which
the control portion 50 controls the adjustment portion 40 on the basis of the monitoring result of the monitoring portion 30, and adjusts a hydrogen ion concentration of the liquid by adding a pH adjuster to the liquid.

A hydrogen ion concentration range appropriate for cell culture can be maintained. The appropriate hydrogen ion concentration range varies depending on the type of cell, and in cells derived from mammals, for example, pH is preferably controlled to 7.2 or more and 7.4 or less.

### <<Culture system 1 according to nineteenth aspect>>

A culture system 1 according to a nineteenth aspect is the culture system 1 according to any one of the first to eighteenth aspects, in which
the monitoring portion 30 is further capable of outputting information regarding a carbon dioxide concentration in a gas in contact with the liquid, and
the control portion 50 controls the adjustment portion 40 on the basis of the information regarding the carbon dioxide concentration of the monitoring portion 30, supplies carbon dioxide, and adjusts a hydrogen ion concentration of the liquid.

By also using the information regarding the carbon dioxide concentration, an environment in which cells are cultured can be accurately maintained.

### <<Culture system 1 according to twentieth aspect>>

A culture system 1 according to a twentieth aspect is the culture system 1 according to any one of the first to nineteenth aspects, in which
the adjustment portion 40 includes a flow rate adjusting portion 48 that adjusts a flow rate of each component for adjustment, and
the control portion 50 controls the flow rate adjusting portion 48 on the basis of the monitoring result of the monitoring portion 30.

The adjustment portion 40 includes the flow rate adjusting portion 48. The flow rate adjusting portion 48 adjusts the flow rate of each component for adjustment. The flow rate adjusting portion 48 can be, for example, a valve or a pump. Note that the pump only needs to be able to adjust a flow rate, and can be, for example, a roller pump, a reciprocating pump, a centrifugal pump, or a propeller pump. The flow rate can be adjusted by a frequency of opening and closing of a valve or the like. The flow rate can be adjusted by a rotation speed of the pump, a frequency of driving of the pump, or the like. The flow rate adjusting portion 48 can adjust the flow rate separately for each component. Since the flow rate can be adjusted for each component, components necessary for growth of a cell aggregate can be supplied to the culture portion 10 without excess or deficiency.

### <<Culture system 1 according to twenty-first aspect>>

A culture system 1 according to a twenty-first aspect is the culture system 1 according to any one of the first to twentieth aspects, in which
the adjustment portion 40 includes a main body portion 86 and a panel 84 attachable to and detachable from the main body portion 86.

The main body portion 86 is mainly a component or a member that is fixedly installed or disposed. The main body portion 86 includes a housing and the like (not illustrated). An outer surface portion of the housing of the main body portion 86 has a latching member such as a hook. The panel 84 is latched or locked to the latching member. The panel 84 can be detached from the main body portion 86 or attached to the main body portion 86. Note that the panel 84 only needs to be attachable to and detachable from a certain position of the main body portion 86, and various locking mechanisms such as a magnet can be used in addition to a mechanistic member such as a hook.

### <Main body portion 86 and non-wetted portion>

The adjustment portion 40 includes a non-wetted portion and a wetted portion. The non-wetted portion is mainly a component, a member, or the like that is not in contact with a flowing liquid. For example, the non-wetted portion is a non-consumable item such as an electric motor for operating a pump for causing a liquid to flow, a drive circuit for driving the electric motor, a liquid storage portion 42a or 44a (for example, a tank) for storing a liquid, and a cooling device for cooling the storage portion. A component or a member constituting the non-wetted portion is housed or installed in the main body portion 86. The liquid storage portions 42a and 44a will be described later.

### <Panel 84 and wetted portion>

The wetted portion is mainly a component, a member, or the like that comes into contact with a flowing liquid. For example, the wetted portion is the component adding portion 42, the component removing portion 44, the gas exchange membrane 46, the dialysis membrane, or the like described above in addition to a circulation feed pipe, a supply pipe, or the like. The wetted portion is mainly constituted by a component or a member handled as a consumable item or the like. A component or a member constituting the wetted portion is attached to the panel 84. By disposing the component or the member on the panel 84, operation of exchanging a component or a member related to the adjustment portion 40 can be easily and simply performed.

Specifically, among the members and components used in the adjustment portion 40, a member or a component to be come into contact with a liquid (requiring sterilization) is disposed on the panel 84 that is attachable and detachable. In particular, a member, a component, or the like that is handled as a consumable component or a disposable component, such as a circulation feed pipe, a supply pipe, or a connector for connecting the circulation feed pipe and the supply pipe is disposed on the panel 84, and the member, the component, or the like is disposed on the panel 84 in advance, whereby operation of exchanging the panel 84 or the like can be quickly and easily performed. Note that a module such as an oxygen supply module may be disposed on the panel 84 from an exchange frequency or the like.

Components such as pipes and connectors are connected to each other in advance and disposed on the panel 84. In this way, a component such as a pipe or a connector can be formed on the panel 84 in advance in a state communicable with a pump or a valve.

On the panel 84, a plurality of components such as pipes and connectors can be disposed so as not to interfere with each other, and operability can be improved. Furthermore, since states of the plurality of components are easily visually recognized, necessity of exchange or the like can be easily recognized.

Note that the size, shape, and the like of the panel 84 are not limited. The panel 84 only needs to be a panel that can mount a plurality of components such as a pipe and a connector thereon and can be attached to and detached from another member such as a pump or a valve.

### <<Culture system 1 according to twenty-second aspect>>

A culture system 1 according to a twenty-second aspect is the culture system 1 according to any one of the first to twenty-first aspects, further including a disposal portion 70 capable of discharging the liquid from the path portion 20.

Since the liquid can be discharged to the disposal portion 70, a component in the liquid contained in the culture portion 10 or properties of the liquid can be adjusted only to those necessary for growth of the cell aggregate. The amount of the liquid contained in the culture portion 10 can be adjusted to an appropriate amount.

### <<Culture system 1 according to twenty-third aspect>>

A culture system 1 according to a twenty-third aspect is the culture system 1 according to the twenty-second aspect, further including a discharge mechanism 72 that discharges a liquid from the path portion 20 to the disposal portion 70, in which
the control portion 50 controls the discharge mechanism 72 to cause the liquid in the culture portion 10 to flow to the path portion 20 as circulation priming, and then to be discharged to the disposal portion 70. The circulation priming is a treatment performed for rinsing the path portion 20 and filling the path portion 20 with a medium. Furthermore, in a case where there are various modules such as a gas exchange module, a dialysis module, and an oxygen supply module, the modules can also be rinsed by the circulation priming.

Since the liquid of the culture portion 10 is discharged by being caused to flow to the path portion 20 as circulation priming,
the liquid in the culture portion 10 can be effectively utilized.

### <<Culture system 1 according to twenty-fourth aspect>>

A culture system 1 according to a twenty-fourth aspect is the culture system 1 according to any one of the first to twenty-third aspects, in which
the control portion 50 controls the component in the liquid and/or the properties of the liquid to a predetermined value or a predetermined range on the basis of the monitoring result of the monitoring portion 30.

The cell aggregate can be continuously grown accurately depending on the degree of growth of the cell aggregate and a state of the liquid.

### <<Culture system 1 according to twenty-fifth aspect>>

A culture system 1 according to a twenty-fifth aspect is the culture system 1 according to any one of the first to twenty-fourth aspects, in which
the control portion 50 controls the adjustment portion 40 to make the amount of the liquid in the culture portion 10 larger than the amount of the liquid at the time of start of culture on the basis of progress of culture.

Since the amount of the liquid is determined depending on the size of the cell and the degree of growth of the cell aggregate, it is possible to prevent the cell and the cell aggregate from being led out from the culture portion 10 and to cause the cell and the cell aggregate to stay in the culture portion 10 to grow the cell aggregate.

### <<Culture system 1 according to twenty-sixth aspect>>

A culture system 1 according to a twenty-sixth aspect is the culture system 1 according to the twenty-fifth aspect, in which
the control portion 50 performs
a first treatment of controlling the stirring portion by alternately switching between a first stirring control and a second stirring control different from the first stirring control when the amount of the liquid in the culture portion 10 is the amount of the liquid at the time of start of culture.

By repeating both a state in which the cells are easily brought into contact with each other and the cell aggregate is easily formed and a state in which the formed cell aggregate is stabilized, the cell aggregate can be accurately grown in the culture portion 10.

### <<Culture system 1 according to twenty-seventh aspect>>

A culture system 1 according to a twenty-seventh aspect is the culture system 1 according to the twenty-sixth aspect, in which
the control portion 50
performs, after the first treatment, a second treatment of operating the stirring portion by the first stirring control and causing the adjustment portion 40 to stepwise adjust the amount of the liquid in the culture portion 10.

The cell aggregate can be gradually grown depending on the size of the cell aggregate so as to approach a size that is not led out from the culture portion 10.

### <<Culture system 1 according to twenty-eighth aspect>>

A culture system 1 according to a twenty-eighth aspect is the culture system 1 according to the twenty-seventh aspect, in which
the control portion 50 controls the adjustment portion 40, and
performs, after the second treatment, a third treatment of exchanging the liquid via the path portion 20 when a liquid level of the liquid in the culture portion 10 is higher than the outlet 12.

The cell aggregate can be grown to a size that is not led out from the culture portion 10, and the cell aggregate can be finally grown to a desired size by circulating the liquid.

### <<Light shielding portion SP>>

As illustrated in Fig. 1, the thermostatic bath 80, the path portion 20, and the adjustment portion 40 are shielded from light by a light shielding member (light shielding portion SP). By shielding the thermostatic bath 80, the path portion 20, and the adjustment portion 40 from light, photodegradation and decomposition of an additive or the like can be prevented to prolong a life of the liquid (for example, a medium), and accuracy of the monitoring result by the monitoring portion 30, for example, accuracy of measurement of a medium component, imaging, or the like can be improved.

### <<Cooling portion CP>>

As illustrated in Fig. 1, the component adding portion 42 of the adjustment portion 40 includes the liquid storage portion 42a that stores a liquid related to component addition, and the component removing portion 44 includes the liquid storage portion 44a that stores a liquid related to component removal. The liquid storage portion 42a of the component adding portion 42 and the liquid storage portion 44a of the component removing portion 44 are cooled by a cooling member (cooling portion CP). By cooling the liquid storage portions 42a and 44a, degradation and decomposition of an additive or the like can be prevented.

### <<<<First to third embodiments>>>>

Hereinafter, culture systems according to first to third embodiments will be described. Note that the following are merely examples, and the first to third embodiments, modification examples, and the like described as examples in the present specification should not be limited to being applied to specific ones, and may be any combination. For example, a modification of one embodiment is also a modification of another embodiment. In addition, even when one modification and another modification are described independently, it should be understood that a combination of the one modification and the another modification is also described.

### <<<<Details of first embodiment>>>>

In a case where a cell aggregate is cultured using a culture system in which a medium is caused to flow by a liquid feeding pump or the like, when the cell aggregate passes through a separation filter along with a flow of the medium, there is a possibility that the cell aggregate is separated. Therefore, unless the cell aggregate is grown to a certain size (pore size of a cell separation filter or more), it may be difficult to apply this culture system. For this reason, pre-culture of the cell aggregate may be required separately.

In addition, it is known that a proliferation rate (aggregate growth rate) varies depending on the type or lot of the cell. Therefore, in a culture process, it is necessary to monitor a culture environment and to control culture conditions. However, in a conventional culture system, only control for making a flow rate in a culture tank constant is performed. Therefore, when the type or lot of cell is different, it may take time to optimize the culture conditions (such as a circulation flow rate) in the conventional culture system.

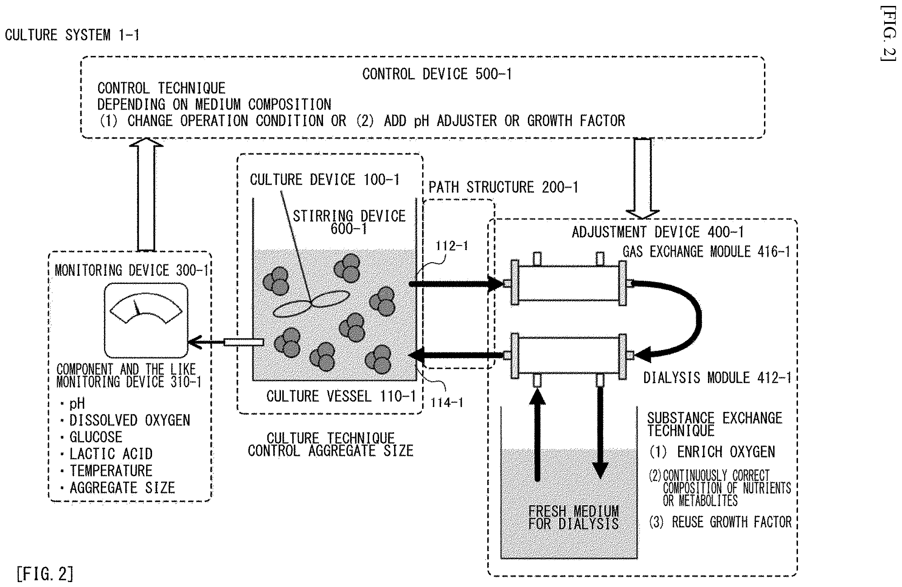

Hereinafter, a first embodiment will be described with reference to the drawings. Fig. 2 is a configuration diagram illustrating a schematic configuration of a culture system 1-1 according to the first embodiment.

The culture system 1-1 mainly includes:
a culture device 100-1;
a path structure 200-1;
a monitoring device 300-1;
an adjustment device 400-1;
a control device 500-1; and
a stirring device 600-1.

### <<Culture device 100-1>>

The culture device 100-1 includes a culture vessel 110-1. A medium and cells are contained in the culture vessel 110-1. The cells are cultured in the culture vessel 110-1. In the culture vessel 110-1, the cells are cultured, and a cell aggregate is grown by contact between the cells. In the culture device 100-1, the cell aggregate is grown while the size of the cell aggregate is controlled.

### <<Path structure 200-1>>

The path structure 200-1 is interposed between the culture device 100-1 and the adjustment device 400-1. The culture vessel 110-1 has an outlet 112-1 and an inlet 114-1. The outlet 112-1 and the inlet 114-1 are connected to the path structure 200-1. The path structure 200-1 communicates with the outlet 112-1 and the inlet 114-1.

The medium contained in the culture vessel 110-1 is led out from the outlet 112-1 and supplied to adjustment device 400-1 via the path structure 200-1. The medium of the adjustment device 400-1 is introduced into the culture vessel 110-1 from the inlet 114-1 via the path structure 200-1. The path structure 200-1 can cause the medium to flow between the culture device 100-1 and the adjustment device 400-1.

### <<Monitoring device 300-1>>

The monitoring device 300-1 monitors states of the cells and the medium in the culture device 100-1. For example, in the culture system 1-1 according to the first embodiment, the monitoring device 300-1 is constituted by a component and the like monitoring device 310-1. The component and the like monitoring device 310-1 detects pH, dissolved oxygen, glucose, lactic acid, a temperature, and an aggregate size. The component and the like monitoring device 310-1 outputs information indicating the detected pH, dissolved oxygen, glucose, lactic acid, temperature, and aggregate size (information regarding a component and the like). The information regarding the component output from the component and the like monitoring device 310-1 is supplied to the control device 500-1.

### <<Adjustment device 400-1>>

The adjustment device 400-1 controls a gas exchange module 416-1 and a dialysis module 412-1 according to instruction information (such as a command or an instruction amount) issued from the control device 500-1. The medium supplied from the culture vessel 110-1 to the adjustment device 400-1 is adjusted by the gas exchange module 416-1 and the dialysis module 412-1. The adjusted medium is returned to the culture vessel 110-1.

The adjustment device 400-1 adjusts the medium by a substance exchange technique. For example, the adjustment device 400-1 adjusts the medium by enriching oxygen, continuously correcting a composition of nutrients or metabolites, or reusing growth factors remaining in the medium. Note that the remaining growth factors include both a growth factor added to a new medium and a growth factor produced by cells.

### <<Control device 500-1>>

The control device 500-1 receives the information regarding a component output from the component and the like monitoring device 310-1 of the monitoring device 300-1. The control device 500-1 generates and outputs instruction information depending on the information regarding a component. The instruction information is information for changing an operation condition or adding a pH adjuster, a growth factor, or the like depending on a medium composition.

### <<Stirring device 600-1>>

The stirring device 600-1 stirs the medium contained in the culture vessel 110-1 of the culture device 100-1. By stirring the medium, cells are easily brought into contact with each other, and a cell aggregate is grown. As described above, the predetermined stirring condition varies depending on a stage of culture. The predetermined stirring condition is a condition for increasing a possibility of contact between cells, dispersing formed cell aggregates so as not to adhere to each other (enlarge), and preventing shear stress applied to the formed cell aggregates from increasing. By setting the predetermined stirring condition in this way, the possibility of contact between cells can be increased, whereby cell aggregates can be easily formed. By appropriately dispersing the formed cell aggregates, it is possible to prevent adhesion between the cell aggregates (enlargement). By preventing the shear stress applied to the cell aggregates from increasing, the formed cell aggregates can be prevented from being divided.

### <<<<Details of second embodiment>>>>

Hereinafter, a second embodiment will be described with reference to the drawings.

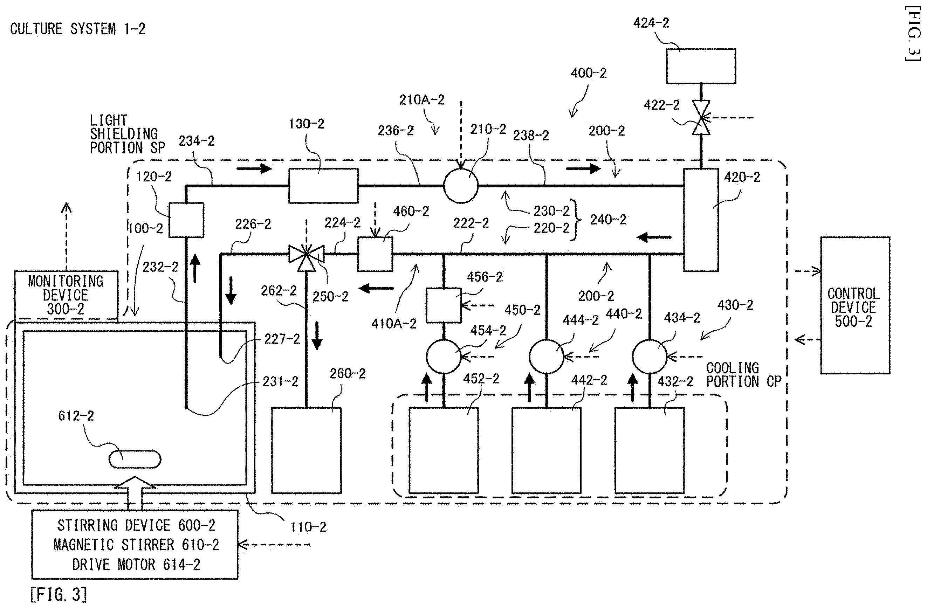

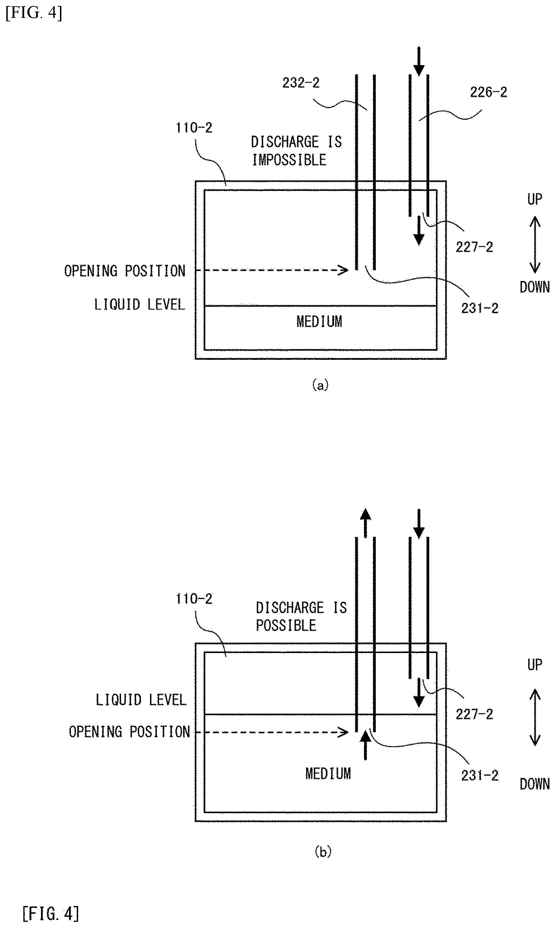

Fig. 3 is a block diagram illustrating details of a culture system 1-2 according to the second embodiment. Fig. 4 is a schematic diagram illustrating a relationship between positions of an inlet 227-2 and an outlet 231-2 in the culture vessel 110-2. Note that a broken line having an arrow illustrated in Fig. 3 indicates input or output of a control-related signal. Furthermore, two regions surrounded by broken lines in Fig. 3 are a light shielding portion SP indicating a constituent element to be shielded from light and a cooling portion CP indicating a cooled constituent element.

### <<<<Configuration of culture system 1-2>>>

The culture system 1-2 mainly includes:
a culture device 100-2;
a path structure 200-2;
a monitoring device 300-2;
an adjustment device 400-2;
a control device 500-2; and
a stirring device 600-2.

### <<Path structure 200-2>>

The path structure 200-2 includes:
a supply system 220-2; and
a circulation feed system 230-2.

### <Supply system 220-2>

The supply system 220-2 includes:
a supply pipe 222-2;
a supply pipe 224-2; and
a supply pipe 226-2. A medium is introduced into the culture device 100-2 by the supply system 220-2.

### <Circulation feed system 230-2>

The circulation feed system 230-2 includes:
a circulation feed pipe 232-2;
a circulation feed pipe 234-2;
a circulation feed pipe 236-2; and
a circulation feed pipe 238-2. The medium contained in the culture device 100-2 is led out to the circulation feed system 230-2. The medium led out to the circulation feed system 230-2 is returned to the supply system 220-2 (fed in a circulation manner).

### <Circulation system 240-2>

The supply system 220-2 and the circulation feed system 230-2 constitute a circulation system 240-2.

### <<New medium and circulation feed medium>>

The medium includes a new medium and a circulation feed medium.

### <New medium>

The new medium is a new medium stored in a medium tank 452-2 described later. The new medium is supplied from the medium tank 452-2 to the culture device 100-2.

### <Circulation feed medium>

The circulation feed medium is a medium already stored in the culture device 100-2 and used for cell culture. The circulation feed medium is led out from the medium tank 452-2 to the circulation feed system 230-2 and fed in a circulation manner to the supply system 220-2. The circulation feed medium is returned to the medium tank 452-2 again (circulated) or discarded to a waste liquid tank 260-2 described later.

### <<Culture device 100-2>>

The culture device 100-2 includes a culture vessel 110-2. The culture vessel 110-2 is a vessel for containing a cell suspension. The culture vessel 110-2 functions as a bioreactor for culturing cells. In the culture vessel 110-2, the cells divide to increase the number of the cells. In the culture vessel 110-2, a cell aggregate grows due to contact between the cells.

### <Supply pipe 226-2 and circulation feed pipe 232-2>

As illustrated in Fig. 4, a supply pipe 226-2 and a circulation feed pipe 232-2 are disposed in the culture vessel 110-2.

### <Inlet 227-2>

The supply pipe 226-2 has an inlet 227-2. The inlet 227-2 opens toward the culture vessel 110-2. A medium supplied to the culture vessel 110-2 flows through the supply pipe 226-2. Both a new medium and a circulation feed medium flow in the supply pipe 226-2. The medium flowing through the supply pipe 226-2 is introduced into the culture vessel 110-2 from the inlet 227-2. The introduced medium is stored in the culture vessel 110-2.

### <Outlet 231-2>

The circulation feed pipe 232-2 has an outlet 231-2. The outlet 231-2 opens toward the culture vessel 110-2. The medium stored in the culture vessel 110-2 is led out to the circulation feed pipe 232-2 via the outlet 231-2. The medium led out from the culture vessel 110-2 flows through the circulation feed pipe 232-2.

As described later, when a liquid level of a cell suspension is controlled so as to be lower than the outlet 231-2, the medium (circulation feed medium) and the cells are not led out to the circulation feed pipe 232-2. On the other hand, when the liquid level of the cell suspension is made higher than the outlet 231-2, a circulation pump 210-2 is operated to such an extent that the cell aggregates are not led out to the circulation feed pipe 232-2. With such control, it is possible to make it difficult for the cells to be led out to the circulation feed pipe 232-2. Note that, when there are cells near the outlet 231-2 of the circulation feed pipe 232-2, there is a possibility that the cells are led out from the outlet 231-2. Therefore, a cell-medium separation filter 120-2 described later is disposed to prevent the cells from flowing out to the circulation feed pipe 234-2 and subsequent pipes even when the cells are led out to the circulation feed pipe 232-2. The medium led out to the circulation feed pipe 232-2 is circulated or discarded.

### <Positions of inlet 227-2 and outlet 231-2>

As illustrated in Fig. 4, the outlet 231-2 is located at a position lower than the inlet 227-2.

### <Height of liquid level of cell suspension>

When a liquid level of the cell suspension is higher than the outlet 231-2, the medium can be led out from the outlet 231-2 by driving a circulation pump 210-2 described later. During a medium exchange circulation culture treatment described later, the liquid level of the cell suspension can be made higher than the outlet 231-2, and the medium can be circulated.

On the other hand, when the liquid level of the cell suspension is lower than the outlet 231-2, the medium cannot be led out from the outlet 231-2. In an intermittent stirring culture treatment and a medium addition culture treatment described later, the liquid level of the cell suspension is made lower than the outlet 231-2 such that the medium and the cells are not led out from the outlet 231-2.

As described above, the lead-out of the medium from the culture vessel 110-2 can be controlled by driving of the circulation pump 210-2 and the height of the liquid level of the cell suspension.

The inlet 227-2 is located at an upper portion of the culture vessel 110-2. Even when the liquid level of the cell suspension is the highest, the inlet 227-2 does not come into contact with the cell suspension.

As a result, the inlet 227-2 can be kept clean.

### <<Stirring device 600-2>>

### <Configuration of stirring device 600-2>

The stirring device 600-2 stirs the medium stored in the culture vessel 110-2. The stirring device 600-2 includes a magnetic stirrer 610-2. The magnetic stirrer 610-2 includes a stirrer 612-2 and a drive motor 614-2. The stirrer 612-2 rotates in response to rotational operation of the drive motor 614-2.

### <Stirring by stirring device 600-2>

The medium is stirred by rotation of the stirrer 612-2. By the medium being stirred, cells and cell aggregates in the cell suspension are displaced. By the cells being displaced, the cells easily come into contact with each other, and formation of a cell aggregate is promoted by the contact between the cells. By the cell aggregate being displaced, the cell aggregate easily comes into contact with the cells, and the cell aggregate becomes larger due to contact with the cells. Note that the cell itself also proliferates by cell division to form a cell aggregate.

### <Rotation speed of drive motor 614-2>

A rotation speed of the drive motor 614-2 is controlled by the control device 500-2. The medium can be caused to flow by rotation of the drive motor 614-2.

A minimum rotation speed of the drive motor 614-2 (corresponding to a minimum flow speed of the medium) is preferably set to such an extent that cells and cell aggregates do not come into contact with a wall surface of the culture vessel 110-2. The wall surface of the culture vessel 110-2 includes all surfaces with which cells and cell aggregates may come into contact. For example, when the culture vessel 110-2 has a cylindrical shape or a square cylindrical shape, the wall surface includes a bottom surface and an inner surface of a side surface. On the other hand, a maximum rotation speed of the drive motor 614-2 (corresponding to a maximum flow speed of the medium) is preferably such an extent that cells displaced in the culture vessel 110-2 float and are not led out from the outlet 231-2 by a flow due to operation of the circulation pump 210-2. When the cell aggregate becomes large, the cell aggregate becomes heavy and easily sinks, and easily comes into contact with a bottom surface of the culture vessel 110-2. Therefore, the control device 500-2 preferably performs control so as to increase the rotation speed of the drive motor 614-2 depending on growth of the cell aggregate (the size of the cell aggregate or the like).

### <Rotation direction of drive motor 614-2>

The drive motor 614-2 rotates in a constant direction. For example, the drive motor 614-2 rotates clockwise or counterclockwise in plan view. Note that the drive motor 614-2 may be appropriately reversed. By reversing the rotation direction, a possibility that cells come into contact with each other and a possibility that the cells come into contact with cell aggregates can be increased.

### <Modification of stirring device 600-2>

In the example described above, the stirring device 600-2 includes the magnetic stirrer 610-2. Without being limited thereto, the stirring device 600-2 may be a device that performs stirring by putting a shaft to which an impeller is attached into the culture vessel 110-2 and rotating the shaft or reciprocating the shaft up and down (not illustrated). In addition, the stirring device 600-2 may be a device using a rotation/shaking machine (not illustrated) such as a roller bottle, a shaking flask, or a culture bag. The stirring device 600-2 may be constituted by a spinner flask (including the culture vessel 110-2 and a stirrer (stirring blade)) or the like. The stirring device 600-2 only needs to be a device that causes the medium to flow. The stirring device 600-2 only needs to be a device that can apply a force to a cell suspension to cause the medium to flow.

### <<Adjustment device 400-2>>

The adjustment device 400-2 supplies a new medium or a circulation feed medium to the culture device 100-2. The adjustment device 400-2 includes a circulation pump drive portion 210A-2 and a supply pump drive portion 410A-2.

### <Circulation pump drive portion 210A-2>

The circulation pump drive portion 210A-2 includes the circulation pump 210-2 and the circulation feed pipes 232-2 to 238-2 (circulation feed system 230-2).

### <Circulation pump 210-2>

The circulation pump 210-2 leads out the medium stored in the culture device 100-2 from the outlet 231-2 to the circulation feed pipe 232-2. The circulation feed medium led out to the circulation feed pipe 232-2 is guided to an oxygen supply module 420-2 via the circulation feed pipes 234-2 to 238-2 by the circulation pump 210-2. The circulation feed medium is guided to the supply pump drive portion 410A-2 after passing through the oxygen supply module 420-2. By operating the circulation pump 210-2, the circulation feed medium is guided as follows: the outlet 231-2 → the circulation feed pipe 232-2 → the circulation feed pipe 234-2 → the circulation feed pipe 236-2 → the circulation pump 210-2 → the circulation feed pipe 238-2 → the oxygen supply module 420-2 → ....

The circulation pump 210-2 is operated to such an extent that the cell aggregates stored in the culture device 100-2 are not led out to the circulation feed pipe 232-2. For example, by appropriately reducing the rotation speed of the circulation pump 210-2, a flow speed of the circulation feed medium flowing through the circulation feed pipes 232-2 to 238-2 is reduced. By reducing the flow speed in the circulation feed pipes 232-2 to 238-2, a gravity sedimentation rate of the cell aggregates exceeds the flow speed, and the cell aggregates stored in the culture device 100-2 can be prevented from being led out to the circulation feed pipe 232-2.

### <Cell-medium separation filter 120-2>

The circulation pump drive portion 210A-2 includes the cell-medium separation filter 120-2. The cell-medium separation filter 120-2 is disposed between the circulation feed pipe 232-2 and the circulation feed pipe 234-2. As described above, the circulation pump 210-2 is operated to such an extent that the cell aggregates are not led out to the circulation feed pipe 232-2. However, there is also a possibility that cells that have not formed cell aggregates float and are led out to the circulation feed pipe 232-2. Therefore, by disposing the cell-medium separation filter 120-2, it is possible to prevent the cells from flowing out to the circulation feed pipe 234-2 and subsequent pipes.

### <Sampling port 130-2>

The circulation pump drive portion 210A-2 has sampling port 130-2. A flowing circulation feed medium is sampled at the sampling port 130-2. The sampled medium is analyzed by various analysis devices and the like, and a state of the medium can be acquired.

### <Supply pump drive portion 410A-2>

The supply pump drive portion 410A-2 includes the oxygen supply module 420-2, a pH adjuster supply portion 430-2, a glucose concentrate supply portion 440-2, a medium supply portion 450-2, a circulation heater 460-2, and supply pipes 222-2 to 226-2 (supply system 220-2).

### <Oxygen supply module 420-2>

The oxygen supply module 420-2 adds oxygen to the circulating circulation feed medium. The oxygen supply module 420-2 is connected to an oxygen cylinder 424-2 via a pressure regulator (not illustrated) and a valve 422-2. The valve 422-2 is opened or closed depending on a control signal of the control device 500-2. When the valve 422-2 is opened, oxygen is supplied from the oxygen cylinder 424-2 to the circulation feed medium via the oxygen supply module 420-2. When the valve 422-2 is closed, oxygen to the circulation feed medium is blocked.

### <pH adjuster supply portion 430-2>

The pH adjuster supply portion 430-2 includes a pH adjuster tank 432-2 and a supply pump 434-2. A pH adjuster is stored in the pH adjuster tank 432-2. The supply pump 434-2 operates depending on a control signal of the control device 500-2. When the supply pump 434-2 operates, the pH adjuster stored in the pH adjuster tank 432-2 is led out to the supply pipe 222-2. The led-out pH adjuster is added to the circulation feed medium flowing in the supply pipe 222-2.

### <Glucose concentrate supply portion 440-2>

The glucose concentrate supply portion 440-2 includes a glucose concentrate tank 442-2 and a supply pump 444-2. The glucose concentrate tank 442-2 stores a glucose concentrate. The supply pump 444-2 operates depending on a control signal of the control device 500-2. When the supply pump 444-2 operates, the glucose concentrate stored in the glucose concentrate tank 442-2 is led out to the supply pipe 222-2. The led-out glucose concentrate is added to the circulation feed medium flowing in the supply pipe 222-2.

### <Medium supply portion 450-2>

The medium supply portion 450-2 includes the medium tank 452-2, a supply pump 454-2, and a medium heater 456-2. A new medium is stored in the medium tank 452-2. The supply pump 454-2 operates depending on a control signal of the control device 500-2. When the supply pump 454-2 operates, the new medium stored in the medium tank 452-2 is led out to the supply pipe 222-2 via the medium heater 456-2.

The medium heater 456-2 heats the new medium to a desired temperature. Note that the new medium is also heated by the circulation heater 460-2 described later. The medium tank 452-2 stores the new medium at a low temperature. Therefore, only the circulation heater 460-2 is likely to heat the new medium insufficiently for supplying the new medium to the culture vessel 110-2. The medium heater 456-2 preliminarily heats the new medium to a certain temperature. The heated new medium is added to the circulation feed medium flowing in the supply pipe 222-2.

### <Circulation heater 460-2>

The circulation heater 460-2 heats a circulation feed medium, a new medium, a pH adjuster, and a glucose concentrate (hereinafter, for convenience, the circulation feed medium and the new medium are not distinguished from each other, and are simply referred to as a medium. In addition, the medium including the pH adjuster and the glucose concentrate is simply referred to as a medium.). The circulation heater 460-2 heats a passing medium to a temperature suitable for supply to the culture vessel 110-2. Specifically, the circulation heater 460-2 heats the passing medium so as to have a temperature suitable for cell culture in the culture vessel 110-2.

The circulation heater 460-2 operates depending on a control signal of the control device 500-2. The circulation heater 460-2 heats the passing medium depending on the control signal.

### <<Three-way valve 250-2 and waste liquid tank 260-2>>

A three-way valve 250-2 controls a flow of a medium that has passed through the circulation heater 460-2. The three-way valve 250-2 can be switched to a medium supply state or a medium disposal state.

### <Medium supply state>

The medium supply state is a state in which a medium is supplied to the culture vessel 110-2. The medium supply state is a state in which the supply pipe 224-2 and the supply pipe 226-2 communicate with each other. When the three-way valve 250-2 is in the medium supply state, a medium is supplied to the culture vessel 110-2 via the supply pipes 224-2 and 226-2.

### <Medium disposal state>

The medium disposal state is a state in which a medium is discharged to the waste liquid tank 260-2. The medium disposal state is a state in which the supply pipe 224-2 and the disposal pipe 262-2 communicate with each other. When the three-way valve 250-2 is in the medium disposal state, a medium is discarded to the waste liquid tank 260-2 via the supply pipe 224-2 and the disposal pipe 262-2. The discarded medium is guided to the waste liquid tank 260-2.

The three-way valve 250-2 operates depending on a control signal of the control device 500-2. The three-way valve 250-2 is in the medium supply state or the medium disposal state depending on the control signal.

### <<Light shielding portion SP>>

As illustrated in Fig. 3, the culture vessel 110-2, the circulation feed pipe 232-2, the circulation feed pipe 234-2, the circulation feed pipe 236-2, the circulation feed pipe 238-2, the cell-medium separation filter 120-2, the sampling port 130-2, the oxygen supply module 420-2, the supply pipe 222-2, the supply pipe 224-2, the supply pipe 226-2, the pH adjuster supply portion 430-2, the glucose concentrate supply portion 440-2, and the medium supply portion 450-2 are shielded from light by a light shielding member (light shielding portion SP). By shielding these portions from light, photodegradation and decomposition of an additive or the like can be prevented, and accuracy of a monitoring result by the monitoring device 300-2, for example, accuracy of measurement of a medium component, imaging, or the like can be improved.

### <<Cooling portion CP>>

The pH adjuster tank 432-2, the glucose concentrate tank 442-2, and the medium tank 452-2 are cooled by a low temperature thermostatic bath (cooling portion CP). By cooling the pH adjuster supply portion 430-2, the glucose concentrate supply portion 440-2, and the medium supply portion 450-2, degradation and decomposition of the additive and the like described above can be prevented.

### <<Monitoring device 300-2>>

The monitoring device 300-2 includes detection devices such as various sensors. A detection signal emitted from the detection device is transmitted to the control device 500-2.

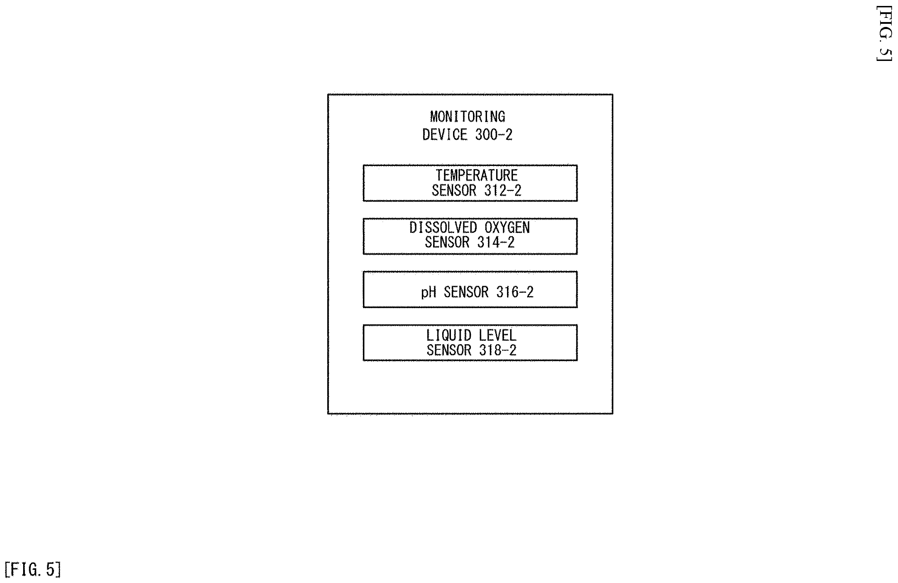

Fig. 5 is a block diagram illustrating various sensors included in the monitoring device 300-2. As illustrated in Fig. 5, the monitoring device 300-2 includes:
a temperature sensor 312-2;
a dissolved oxygen sensor 314-2;
a pH sensor 316-2; and
a liquid level sensor 318-2.

### <Temperature sensor 312-2>

The temperature sensor 312-2 detects a temperature of the medium stored in the culture vessel 110-2. The temperature sensor 312-2 outputs a detection signal indicating the detected temperature.

### <Dissolved oxygen sensor 314-2>

The dissolved oxygen sensor 314-2 detects the amount of dissolved oxygen in the medium stored in the culture vessel 110-2. The dissolved oxygen sensor 314-2 outputs a detection signal indicating the detected amount of dissolved oxygen.

### <pH sensor 316-2>

The pH sensor 316-2 detects a pH value of the medium stored in the culture vessel 110-2. The pH sensor 316-2 outputs a detection signal indicating the detected pH value.

### <Liquid level sensor 318-2>

The liquid level sensor 318-2 detects a liquid level of the cell suspension stored in the culture vessel 110-2. The liquid level sensor 318-2 outputs a detection signal indicating presence or absence of a liquid level (whether or not a liquid level is located).

For example, a plurality of liquid level sensors are disposed as the liquid level sensor 318-2 at different liquid level positions of the culture vessel 110-2 (not illustrated). Among the plurality of liquid level sensors, only a liquid level sensor at which a liquid level is located outputs a signal indicating that the liquid level is present. Among the plurality of liquid level sensors, a liquid level sensor at which a liquid level is not located outputs a signal indicating that the liquid level is not present. In this way, the position of the liquid level of the cell suspension in the culture vessel 110-2 can be acquired.

### <Camera 320-2>

The monitoring device 300-2 may include a camera 320-2. The camera 320-2 images cells and cell aggregates cultured in the culture vessel 110-2. The camera 320-2 outputs a detection signal indicating a captured image. An image to be captured may be a still image or a moving image. The image only needs to be an image that can acquire a state (sizes and the like) of cells or cell aggregates.

### <<Control device 500-2>>

The control device 500-2 performs various types of arithmetic processing, data processing, and determination processing on the basis of various detection signals emitted from the monitoring device 300-2, and controls a pump and the like.

The control device 500-2 mainly includes a processor (such as a central processing unit (CPU)), a read only memory (ROM), a random access memory (RAM), an interface device (I/F), an auxiliary storage device (such as a hard disk drive (HDD) or a solid state drive (SSD)), an input operation device (a keyboard, a mouse, a touch panel, or the like), and the like. For example, the control device 500-2 can be a personal computer, a tablet computer, a portable terminal device, or a device similar thereto.

The control device 500-2 transmits a control signal, via an interface device (I/F), to each of
the stirring device 600-2,
the valve 422-2,
the three-way valve 250-2,
the circulation pump 210-2,
the supply pump 434-2,
the supply pump 444-2,
the supply pump 454-2,
the medium heater 456-2, and
the circulation heater 460-2.

### <Control of magnetic stirrer 610-2>

The control device 500-2 transmits a control signal indicating a rotation speed of the drive motor 614-2 to the stirring device 600-2.

The rotation speed is a rotation speed at which cells easily come into contact with each other in the culture vessel 110-2, the cells do not come into contact with a wall portion, and the cells do not float up to the outlet 231-2. The wall surface of the culture vessel 110-2 includes all surfaces with which cells and cell aggregates may come into contact. For example, when the culture vessel 110-2 has a cylindrical shape, the wall surface is a bottom surface and an inner surface of a side surface. This rotation speed is determined by a preliminary experiment or the like, and is stored in advance in an auxiliary storage device or the like.

In addition, as described above, the cell aggregate becomes heavy and easily sinks as it grows, and easily comes into contact with a bottom surface of the culture vessel 110-2. This rotation speed is also determined by a preliminary experiment or the like, and is stored in advance in an auxiliary storage device or the like in association with an elapsed time from start of culture. The control device 500-2 reads a rotation speed corresponding to an elapsed time from start of culture from the auxiliary storage device or the like, and transmits the rotation speed to the magnetic stirrer 610-2.

### <Control of valve 422-2>

The control device 500-2 transmits a control signal indicating an opened state or a closed state of the valve 422-2 to the valve 422-2. When the control device 500-2 transmits a control signal indicating an opened state to the valve 422-2, the valve 422-2 is opened. When the valve 422-2 is opened, the oxygen cylinder 424-2 and the oxygen supply module 420-2 communicate with each other. Oxygen is supplied to the circulation feed medium via the oxygen supply module 420-2.

When the control device 500-2 transmits a control signal indicating a closed state to the valve 422-2, the valve 422-2 is closed. When the valve 422-2 is closed, the oxygen cylinder 424-2 and the oxygen supply module 420-2 do not communicate with each other, and the supply of oxygen to the circulation feed medium is stopped.

The control device 500-2 acquires the amount of dissolved oxygen in the medium of the culture vessel 110-2 from the detection signal output from the dissolved oxygen sensor 314-2. The control device 500-2 controls the valve 422-2 so as to have a predetermined target dissolved oxygen concentration. For example, the control device 500-2 controls the valve 422-2 so as to have a target dissolved oxygen concentration on the basis of the acquired amount of dissolved oxygen by proportional-integral-differential control (PID control). Note that oxygen is supplied in a medium exchange circulation culture treatment described later.

### <Control of three-way valve 250-2>

The control device 500-2 transmits a control signal indicating a medium supply state or a medium disposal state to the three-way valve 250-2. When the control device 500-2 transmits a control signal indicating a medium supply state to the three-way valve 250-2, the supply pipes 224-2 and 226-2 are allowed to communicate with each other. By the communication between the supply pipes 224-2 and 226-2, a medium is supplied to the culture vessel 110-2 via the supply pipes 224-2 and 226-2.

When the control device 500-2 transmits a control signal indicating a medium disposal state to the three-way valve 250-2, the supply pipe 224-2 and the disposal pipe 262-2 are allowed to communicate with each other. By the communication between the supply pipe 224-2 and the disposal pipe 262-2, a medium is discarded to the waste liquid tank 260-2 via the supply pipe 224-2 and the disposal pipe 262-2.

### <Control of circulation pump 210-2>

The control device 500-2 transmits a control signal indicating operation or stop to the circulation pump 210-2. When the control device 500-2 transmits a control signal indicating operation to the circulation pump 210-2, the circulation pump 210-2 operates. As a result, the medium can be circulated. When the control device 500-2 transmits a control signal indicating stop to the circulation pump 210-2, the circulation pump 210-2 stops. As a result, the circulation of the medium can be stopped.

The control device 500-2 can also transmit a control signal including information indicating a rotation speed of the circulation pump 210-2 to the circulation pump 210-2. The circulation pump 210-2 operates at a rotation speed indicated by the control signal. A flow rate (flow speed) of the medium per unit time can be adjusted by changing the rotation speed of the circulation pump 210-2. The rotation speed of the circulation pump 210-2 is determined by a preliminary experiment or the like, and is stored in advance in an auxiliary storage device or the like in association with the flow rate per unit time.

When the control device 500-2 transmits a control signal indicating a medium supply state to the three-way valve 250-2 and transmits a control signal indicating operation to the circulation pump 210-2, the three-way valve 250-2 becomes the medium supply state, and the circulation pump 210-2 operates. With such control, the medium can be circulated as follows: the culture vessel 110-2 → the outlet 231-2 → the circulation feed pipe 232-2 → the circulation feed pipe 234-2 → the circulation feed pipe 236-2 → the circulation pump 210-2 → the circulation feed pipe 238-2 → the oxygen supply module 420-2 → the supply pipe 222-2 → the circulation heater 460-2 → the supply pipe 224-2 → the supply pipe 226-2 → the inlet 227-2 → the culture vessel 110-2.

When the control device 500-2 transmits a control signal indicating a medium disposal state to the three-way valve 250-2 and transmits a control signal indicating operation to the circulation pump 210-2, the three-way valve 250-2 becomes the medium disposal state, and the circulation pump 210-2 operates. With such control, the medium can be discarded as follows: the culture vessel 110-2 → the outlet 231-2 → the circulation feed pipe 232-2 → the circulation feed pipe 234-2 → the circulation feed pipe 236-2 → the circulation pump 210-2 → the circulation feed pipe 238-2 → the oxygen supply module 420-2 → the supply pipe 222-2 → the circulation heater 460-2 → the supply pipe 224-2 → the disposal pipe 262-2 → the waste liquid tank 260-2.

### <Control of supply pump 434-2>

The control device 500-2 transmits a control signal indicating operation or stop to the supply pump 434-2. When the control device 500-2 transmits a control signal indicating operation to the supply pump 434-2, the supply pump 434-2 operates. By the operation of the supply pump 434-2, the pH adjuster stored in the pH adjuster tank 432-2 is led out to the supply pipe 222-2.

When the control device 500-2 transmits a control signal indicating stop to the supply pump 434-2, the supply pump 434-2 stops. By the stop of the supply pump 434-2, the lead-out of the pH adjuster to the supply pipe 222-2 is stopped.

The control device 500-2 can also transmit a control signal including information indicating a rotation speed of the supply pump 434-2 to the supply pump 434-2. The supply pump 434-2 operates at a rotation speed indicated by the control signal. A flow rate (flow speed) of the pH adjuster per unit time can be adjusted by changing the rotation speed of the supply pump 434-2. The rotation speed of the supply pump 434-2 is determined by a preliminary experiment or the like, and is stored in advance in an auxiliary storage device or the like in association with the flow rate per unit time.

### <Control of supply pump 444-2>

The control device 500-2 transmits a control signal indicating operation or stop to the supply pump 444-2. When the control device 500-2 transmits a control signal indicating operation to the supply pump 444-2, the supply pump 444-2 operates. By the operation of the supply pump 444-2, the glucose concentrate stored in the glucose concentrate tank 442-2 is led out to the supply pipe 222-2.

When the control device 500-2 transmits a control signal indicating stop to the supply pump 444-2, the supply pump 444-2 stops. By the stop of the supply pump 444-2, the lead-out of the glucose concentrate to the supply pipe 222-2 is stopped.

The control device 500-2 can also transmit a control signal including information indicating a rotation speed of the supply pump 444-2 to the supply pump 444-2. The supply pump 444-2 operates at a rotation speed indicated by the control signal. A flow rate (flow speed) of the glucose concentrate per unit time can be adjusted by changing the rotation speed of the supply pump 444-2. The rotation speed of the supply pump 444-2 is determined by a preliminary experiment or the like, and is stored in advance in an auxiliary storage device or the like in association with the flow rate per unit time.

### <Control of supply pump 454-2>

The control device 500-2 transmits a control signal indicating operation or stop to the supply pump 454-2. When the control device 500-2 transmits a control signal indicating operation to the supply pump 454-2, the supply pump 454-2 operates. By the operation of the supply pump 454-2, the new medium stored in the medium tank 452-2 is led out to the supply pipe 222-2.

When the control device 500-2 transmits a control signal indicating stop to the supply pump 454-2, the supply pump 454-2 stops. By the stop of the supply pump 454-2, the lead-out of the new medium to the supply pipe 222-2 is stopped.

The control device 500-2 can also transmit a control signal including information indicating a rotation speed of the supply pump 454-2 to the supply pump 454-2. The supply pump 454-2 operates at a rotation speed indicated by the control signal. A flow rate (flow speed) of the new medium per unit time can be adjusted by changing the rotation speed of the supply pump 454-2. The rotation speed of the supply pump 454-2 is determined by a preliminary experiment or the like, and is stored in advance in an auxiliary storage device or the like in association with the flow rate per unit time.

### <Control of medium heater 456-2>

The control device 500-2 transmits a control signal indicating operation or stop to the medium heater 456-2. When the control device 500-2 transmits a control signal indicating operation to the medium heater 456-2, the medium heater 456-2 operates. By the operation of the medium heater 456-2, the new medium stored in the medium tank 452-2 is heated by the medium heater 456-2 and led out to the supply pipe 222-2.

When the control device 500-2 transmits a control signal indicating stop to the medium heater 456-2, the medium heater 456-2 stops.

The control device 500-2 can also transmit a control signal including information indicating a current value of the medium heater 456-2 to the medium heater 456-2. The medium heater 456-2 operates at a current value indicated by the control signal. By change of the current value of the medium heater 456-2, a temperature at which the new medium is heated can be adjusted. The current value of the medium heater 456-2 is determined by a preliminary experiment or the like, and is stored in advance in an auxiliary storage device or the like in association with the temperature.

### <Control of circulation heater 460-2>

The control device 500-2 transmits a control signal indicating operation or stop to the circulation heater 460-2. When the control device 500-2 transmits a control signal indicating operation to the circulation heater 460-2, the circulation heater 460-2 operates. By the operation of the circulation heater 460-2, the medium passing through the circulation heater 460-2 is heated by the circulation heater 460-2.

When the control device 500-2 transmits a control signal indicating stop to the circulation heater 460-2, the circulation heater 460-2 stops.

The control device 500-2 can also transmit a control signal including information indicating a current value of the circulation heater 460-2 to the circulation heater 460-2. The circulation heater 460-2 operates at a current value indicated by the control signal. By change of the current value of the circulation heater 460-2, a temperature of the new medium passing through the circulation heater 460-2 can be adjusted. The current value of the circulation heater 460-2 is determined by a preliminary experiment or the like, and is stored in advance in an auxiliary storage device or the like in association with the temperature.

### <<<Treatment of culture system 1-2>>>

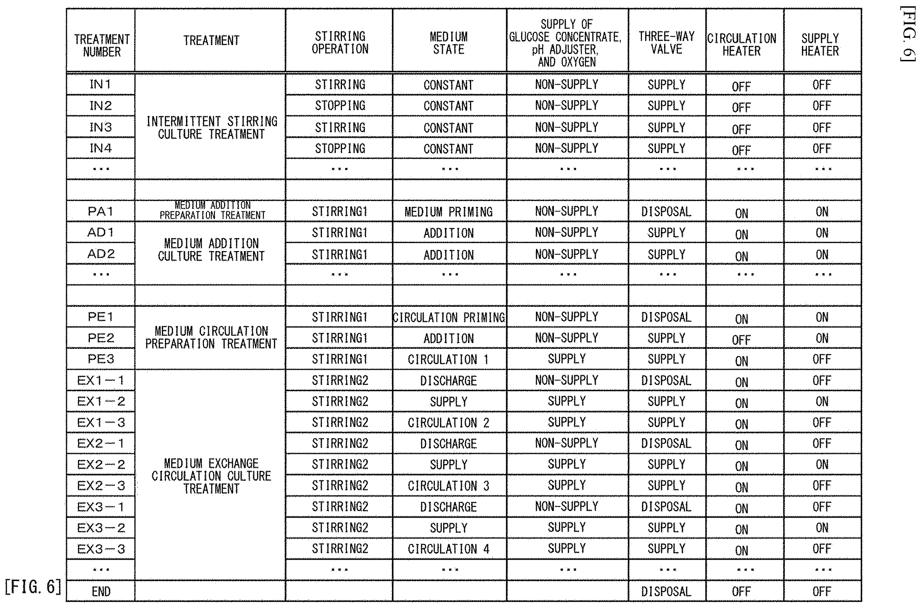

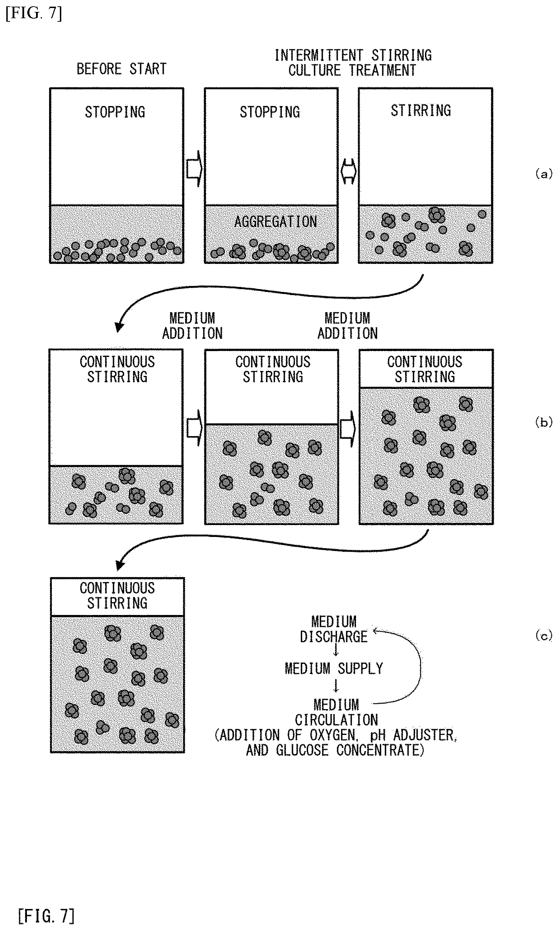

Fig. 6 is a time chart illustrating an outline of treatment of the culture system 1-2. Fig. 7 is a schematic diagram illustrating a state of cell aggregates that grow by the treatment of the culture system 1-2.

The treatment of the culture system 1-2 includes:
a seeding treatment;
an intermittent stirring culture treatment;
a medium addition culture treatment; and
a medium exchange circulation culture treatment.

### <<Seeding treatment>>

The seeding treatment is a treatment for preparing a cell suspension in the culture vessel 110-2. By the seeding treatment, a cell suspension in which cells are dispersed in a medium can be generated. The seeding treatment may be performed by the control device 500-2 or may be performed manually by an operator. In order to make it easy for cells to come into contact with each other, it is only required to make preparation such that a liquid level of the cell suspension in the culture vessel 110-2 is lower than the outlet 231-2.

The intermittent stirring culture treatment, the medium addition culture treatment, and the medium exchange circulation culture treatment will be described along the time chart illustrated in Fig. 6.

### <<Intermittent stirring culture treatment>>

The intermittent stirring culture treatment is a cell aggregation treatment in an initial stage. Cell aggregates are generated by the intermittent stirring culture treatment. In the intermittent stirring culture treatment, stirring and stopping are performed at least once as illustrated in Fig. 7 (a). In the intermittent stirring culture treatment, stirring and stopping are preferably repeated a plurality of times. By stirring a cell suspension, cells are easily brought into contact with each other to promote aggregation thereof. By stopping stirring, a state in which cells are in contact with each other is maintained, and a stable cell aggregate is generated.

In the example illustrated in Fig. 6, the intermittent stirring culture treatment has treatment numbers IN1 to IN4, etc. In the example illustrated in Fig. 6, stirring and stopping are repeated a plurality of times. In the intermittent stirring culture treatment, the amount of the medium (the amount of the cell suspension) in the culture vessel 110-2 is constant.

### <Control of supply pumps 434-2 and 444-2, valve 422-2, medium heater 456-2, and circulation heater 460-2>

In the intermittent stirring culture treatment, the control device 500-2 transmits a control signal indicating stop to the supply pumps 434-2 and 444-2. As a result, in the intermittent stirring culture treatment, a pH adjuster and a glucose concentrate are not supplied to the medium.

In addition, in the intermittent stirring culture treatment, the control device 500-2 transmits a control signal indicating a closed state to the valve 422-2. As a result, in the intermittent stirring culture treatment, oxygen is not supplied to the medium.

In the intermittent stirring culture treatment, the control device 500-2 transmits a control signal indicating a medium supply state to the three-way valve 250-2. As a result, in the intermittent stirring culture treatment, the three-way valve 250-2 becomes the medium supply state.

In the intermittent stirring culture treatment, the control device 500-2 transmits a control signal indicating stop to the medium heater 456-2 and the circulation heater 460-2. As a result, in the intermittent stirring culture treatment, the medium heater 456-2 and the circulation heater 460-2 do not operate and stop.

### <Stirring control (treatment numbers IN1, IN3, ...)>

The control device 500-2 reads a rotation speed stored in an auxiliary storage device or the like, and transmits the rotation speed to the magnetic stirrer 610-2. The control device 500-2 operates the drive motor 614-2 of the magnetic stirrer 610-2 to rotate the stirrer 612-2. As a result, the stirrer 612-2 rotates, and stirring of the medium is started.

### <Stopping control (treatment numbers IN2, IN4, ...)>

The control device 500-2 transmits a stopping control signal to the magnetic stirrer 610-2 after a stirring time has elapsed from start of stirring. As a result, the drive motor 614-2 stops, and the stirrer 612-2 stands still. By the standstill of the stirrer 612-2, the medium is gradually decelerated and stops.

### <Repetition of second and subsequent stirring and stopping>

The second and subsequent stirring (for example, treatment number IN3 in Fig. 6) starts after a stop time has elapsed from stop of stirring. Stirring is started by performing the stirring control described above.

### <Main characteristics of intermittent stirring culture treatment>

In the intermittent stirring culture treatment, stirring and stopping are performed at least once. Stirring and stopping are preferably repeated a plurality of times. By repeating stirring and stopping, a stable cell aggregate can be gradually grown (see Fig. 7(a)).

### <Maximum number of repetitions of repeating stirring and stopping>

A maximum number of repetitions of repeating stirring and stopping is the number of times until a cell aggregate reaches a desired size and increases to a desired number by the intermittent stirring culture treatment. The maximum number of repetitions is based on the type of cell and the like. This maximum number of repetitions is determined by a preliminary experiment or the like, and is stored in advance in an auxiliary storage device or the like. The control device 500-2 repeats stirring and stopping until the maximum number of repetitions is reached.

### <Stirring time and stopping time>

A stirring time during which the drive motor 614-2 is rotated and a stopping time during which the drive motor 614-2 is stopped are also based on the type of cell, the desired size of cell aggregate, and the like. The stirring time and the stopping time are determined by a preliminary experiment or the like, and are stored in advance in an auxiliary storage device or the like. The control device 500-2 reads the stirring time and the stopping time, and determines whether or not the stirring time and the stopping time have been reached.

### <Others>

When stirring and stopping are repeated, the stirring times may be the same as or different from each other. Similarly, when stirring and stopping are repeated, the stopping times may be the same as or different from each other.

The sizes and number of cell aggregates may be acquired on the basis of an image captured by the camera 320-2, and the stirring time, the stopping time, and whether to repeat stirring and stopping may be determined. It is preferable to convert the sizes and number of cell aggregates into an easily obtainable image by performing various types of image processing on the captured image.

In addition, the drive motor 614-2 may be rotated slowly without being completely stopped, and the medium may be displaced at a slow flow speed. Shear stress applied to cells and cell aggregates can be prevented from changing suddenly.

When stirring is started or when stirring is stopped, a speed of the drive motor 614-2 of the magnetic stirrer 610-2 may be gradually changed. Shear stress applied to cells and cell aggregates can be gently changed.

A rotation direction of the drive motor 614-2 may be reversed during stirring. In addition, the rotation direction of the drive motor 614-2 may be reversed every time stirring is performed. A possibility that cells come into contact with each other and a possibility that the cells come into contact with cell aggregates can be changed or increased.

### <<Medium addition culture treatment and medium addition preparation treatment>>

The medium addition culture treatment is a cell aggregation treatment in an intermediate stage. By the medium addition culture treatment, as illustrated in Fig. 7(b), cell aggregates are grown by sequentially adding a medium.

By sequentially adding the medium, cells are divided to increase the number of cells, and cell aggregates are enlarged. When cells not contained in a cell aggregate are divided, the divided cells newly come into contact with the cell aggregate to enlarge the cell aggregate. When cells contained in a cell aggregate are divided, the cell aggregate in which the cells are contained is enlarged.

At the time of start of the medium addition culture treatment, cell aggregates are still small. For this reason, when the medium is circulated, there is a possibility that cell aggregates enter the circulation feed pipe 232-2 or the like, and the medium is not circulated in the medium addition culture treatment. By the medium addition culture treatment, cell aggregates are grown to such an extent that the cell aggregates do not enter the circulation feed pipe 232-2.

In the example illustrated in Fig. 6, the medium addition culture treatment has treatment numbers AD1, AD2, etc. Note that, as illustrated in Fig. 6, before the medium addition culture treatment, a medium addition preparation treatment (treatment number PA1) is performed. The medium addition preparation treatment is a medium priming treatment.

Note that, in the medium addition preparation treatment and the medium addition culture treatment, a pH adjuster, a glucose concentrate, and oxygen are not supplied to the medium following the intermittent stirring culture treatment.

### <Medium addition preparation treatment (treatment number PA1)>

The control device 500-2 transmits a control signal indicating operation to the medium heater 456-2 and the circulation heater 460-2. As a result, the medium heater 456-2 and the circulation heater 460-2 operate before addition of the medium. When the medium is added, the medium can be accurately heated.

The control device 500-2 reads a rotation speed stored in an auxiliary storage device or the like, and transmits the rotation speed to the magnetic stirrer 610-2. The control device 500-2 operates the drive motor 614-2 of the magnetic stirrer 610-2 to rotate the stirrer 612-2. As a result, the stirrer 612-2 rotates at a predetermined rotation speed, and stirring is started again.

The control device 500-2 transmits a control signal indicating a medium disposal state to the three-way valve 250-2. As a result, the three-way valve 250-2 allows the supply pipe 224-2 and the disposal pipe 262-2 to communicate with each other.

The control device 500-2 transmits a control signal indicating operation to the supply pump 454-2. As a result, the new medium stored in the medium tank 452-2 is heated by the medium heater 456-2 and led out to the supply pipe 222-2.

The new medium led out to the supply pipe 222-2 passes through the circulation heater 460-2, flows through the supply pipe 224-2, and reaches the three-way valve 250-2. The new medium that has reached the three-way valve 250-2 is guided toward the disposal pipe 262-2 and discarded to the waste liquid tank 260-2. As a result, the supply pipe 222-2 to which the circulation heater 460-2 is attached, the supply pipe 224-2 and the three-way valve 250-2 can be cleaned (medium priming) with the new medium.

The medium priming is performed as follows. The new medium stored in the medium tank 452-2 is caused to flow to the supply pipe 222-2 using the supply pump 454-2. The flowing medium passes through the disposal pipe 262-2 via supply paths of the medium heater 456-2 and the circulation heater 460-2 and the three-way valve 250-2, and reaches the waste liquid tank 260-2. Cleaning these pipes and the like with the flowing medium is referred to as medium priming.

The control device 500-2 transmits a control signal indicating stop to the supply pump 454-2 after a predetermined time has elapsed. As a result, the lead-out of the new medium to the supply pipe 222-2 stops, and the medium priming ends. The predetermined time is based on the type of cell and a rotation speed of the supply pump 454-2. The predetermined time is determined by a preliminary experiment or the like, and is stored in advance in an auxiliary storage device or the like.

### <Medium addition culture treatment>

After the medium addition preparation treatment (PA1), the medium addition culture treatment is performed. The medium addition culture treatment has treatment numbers AD1, AD2, ... illustrated in Fig. 6.

### <Medium addition culture treatment (treatment number AD1)>

The control device 500-2 transmits a control signal indicating a medium supply state to the three-way valve 250-2. As a result, the three-way valve 250-2 becomes the medium supply state. Note that, as described above, the medium heater 456-2 and the circulation heater 460-2 are already in operation.

The control device 500-2 transmits a control signal indicating operation to the supply pump 454-2. As a result, the new medium stored in the medium tank 452-2 is heated by the medium heater 456-2 and led out to the supply pipe 222-2.

The new medium led out to the supply pipe 222-2 is heated by the circulation heater 460-2, flows through the supply pipe 224-2, and reaches the three-way valve 250-2. The new medium that has reached the three-way valve 250-2 is guided toward the supply pipe 226-2 and introduced into the culture vessel 110-2 from the inlet 227-2. In this way, the new medium is added to the culture vessel 110-2.

The control device 500-2 transmits a control signal indicating stop to the supply pump 454-2 after a predetermined time has elapsed. As a result, the first addition of the new medium to the culture vessel 110-2 is completed.

The predetermined time is a time corresponding to the amount of medium to be added. The predetermined time is based on the type of cell, a rotation speed of the supply pump 454-2, and the like. The predetermined time is determined by a preliminary experiment or the like, and is stored in advance in an auxiliary storage device or the like.

The control device 500-2 transmits a control signal indicating stop to the medium heater 456-2 and the circulation heater 460-2. As a result, the supply pump 454-2, the medium heater 456-2, and the circulation heater 460-2 stop.

### <Medium addition culture treatment (treatment number AD2)>

The medium addition culture treatment (treatment number AD2) is a treatment in which a medium is added again after a predetermined time has elapsed from addition of the medium by the above-described medium addition culture treatment (treatment number AD1).

The control device 500-2 transmits a control signal indicating operation to the medium heater 456-2 and the circulation heater 460-2 after a predetermined time has elapsed from completion of addition of the new medium to the culture vessel 110-2. As a result, the medium heater 456-2 and the circulation heater 460-2 operate before next addition of the medium. When the medium is added, the medium can be accurately heated.

Note that the three-way valve 250-2 is already in a medium disposal state, and the supply pipe 224-2 and the disposal pipe 262-2 communicate with each other.

The control device 500-2 transmits a control signal indicating operation to the supply pump 454-2. As a result, the new medium stored in the medium tank 452-2 is heated by the medium heater 456-2 and led out to the supply pipe 222-2.

The new medium led out to the supply pipe 222-2 is heated by the circulation heater 460-2, flows through the supply pipe 224-2, passes through the three-way valve 250-2, and is introduced into the culture vessel 110-2 from the inlet 227-2 of the supply pipe 226-2. In this way, the new medium is added to the culture vessel 110-2 again.

The control device 500-2 transmits a control signal indicating stop to the supply pump 454-2 after a predetermined time has elapsed. As a result, the second addition of the new medium to the culture vessel 110-2 is completed.

The predetermined time is a time corresponding to the amount of medium to be added. The predetermined time is based on the type of cell, a rotation speed of the supply pump 454-2, and the like. The predetermined time is determined by a preliminary experiment or the like, and is stored in advance in an auxiliary storage device or the like. Note that the predetermined time may be the same as or different from the predetermined time for the first addition of the new medium.

### <Medium addition culture treatment (after treatment number AD2)>

Similarly to the treatment numbers AD1 and AD2, addition of a medium can be repeated a plurality of times.

### <Position of liquid level and end of medium addition culture treatment>

Each time a new medium is added to the culture vessel 110-2, a liquid level of the cell suspension stored in the culture vessel 110-2 gradually increases. When the liquid level of the cell suspension becomes higher than the outlet 231-2 due to the addition of the medium, the medium addition culture treatment is ended.

By making the liquid level of the cell suspension higher than the outlet 231-2, the medium can be led out from the outlet 231-2 to the circulation feed pipe 232-2.

<Characteristics of medium addition culture treatment>

In the medium addition culture treatment, cells are further grown and divided by adding a medium, and cell aggregates are enlarged. In the medium addition culture treatment, the medium is added at least once. In the medium addition culture treatment, it is preferable to add the medium a plurality of times.

The number of times of adding the medium and the amount of the medium to be added are based on the type of cell, the desired size of aggregate, and the like. The number of times of adding the medium and the amount of the medium to be added are also determined by a preliminary experiment or the like, and are stored in advance in an auxiliary storage device or the like.

### <Others>

The amount of the medium to be added may be the same for each addition or may be different among additions.

The sizes and number of cell aggregates may be acquired on the basis of an image captured by the camera 320-2, and the amount of the medium to be added, the number of times of adding the medium, and the like may be determined. It is preferable to convert the sizes and number of cell aggregates into an easily obtainable image by performing various types of image processing on the captured image.

### <<Medium exchange circulation culture treatment and medium circulation preparation treatment>>

The medium exchange circulation culture treatment is a cell aggregation treatment in a final stage. Cell aggregates are grown (enlarged) to a desired final stage by the medium exchange circulation culture treatment. By sequentially exchanging the medium, a new medium is supplied, cells are further divided to increase the number of cells, and cell aggregates are enlarged. Furthermore, by circulating the medium and adding a pH adjuster or the like, not only the medium but also nutrients necessary for growth of cells are supplied, the cells are further divided to increase the number of cells, and cell aggregates are enlarged.

In a final stage of the medium addition culture treatment, the cell aggregates have already grown to such an extent that the cell aggregates do not enter the circulation feed pipe 232-2 or the like. For this reason, in the medium exchange circulation culture treatment, a pH adjuster, a glucose concentrate, and the amount of dissolved oxygen are added by circulating the medium, which are insufficient by supplying the medium. Finally, a cell aggregate having a desired size can be formed.

In the example illustrated in Fig. 6, the medium exchange circulation culture treatment has treatment numbers EX1-1 to EX1-3, EX2-1 to EX2-3, EX3-1 to EX3-3, .... In the medium exchange circulation culture treatment, discharge, supply, and circulation of the medium are repeated a plurality of times.

Note that, as illustrated in Fig. 6, before the medium exchange circulation culture treatment, a medium circulation preparation treatment (treatment numbers PE1 to PE3) is performed. The medium circulation preparation treatment is a circulation priming treatment.

### <Medium circulation preparation treatment (treatment numbers PE1 to PE3)>

As described above, the medium heater 456-2 and the circulation heater 460-2 are already in operation. In addition, in the medium circulation preparation treatment, a pH adjuster, a glucose concentrate, and oxygen are not supplied to the medium following the medium addition culture treatment.

### <Circulation priming (treatment number PE1)>

The control device 500-2 transmits a control signal indicating a medium disposal state to the three-way valve 250-2. As a result, the three-way valve 250-2 becomes the medium disposal state.

The control device 500-2 transmits a control signal indicating operation to the circulation pump 210-2. As a result, the circulation pump 210-2 operates. By the operation of the circulation pump 210-2, the medium stored in the culture vessel 110-2 is led out to the circulation feed pipe 232-2 via the outlet 231-2. The medium led out to the circulation feed pipe 232-2 is discarded to the waste liquid tank 260-2 as follows: the culture vessel 110-2 → the outlet 231-2 → the circulation feed pipe 232-2 → the circulation feed pipe 234-2 → the circulation feed pipe 236-2 → the circulation pump 210-2 → the circulation feed pipe 238-2 → the oxygen supply module 420-2 → the supply pipe 222-2 → the circulation heater 460-2 → the supply pipe 224-2 → the disposal pipe 262-2.

The control device 500-2 transmits a control signal indicating stop to the circulation pump 210-2 when a liquid level of a cell suspension becomes lower than the outlet 231-2 from the detection signal output from the liquid level sensor 318-2. As a result, the circulation pump 210-2 stops.

As a result, the circulation priming ends. By the circulation priming, the circulation feed pipes 232-2 to 238-2, the circulation pump 210-2, the oxygen supply module 420-2, the supply pipes 222-2 to 224-2, the circulation heater 460-2, and the three-way valve 250-2 can be cleaned with the medium (circulation feed medium) stored in the culture vessel 110-2.

### <Addition (treatment number PE2)>

The control device 500-2 transmits a control signal indicating a medium supply state to the three-way valve 250-2. As a result, the three-way valve 250-2 becomes the medium supply state.

The control device 500-2 transmits a control signal indicating stop to the circulation heater 460-2. As a result, the circulation heater 460-2 stops. Note that the medium heater 456-2 is already in operation.

The control device 500-2 transmits a control signal indicating operation to the supply pump 454-2. As a result, the new medium stored in the medium tank 452-2 is heated by the medium heater 456-2 and led out to the supply pipe 222-2.

The new medium led out to the supply pipe 222-2 is heated by the circulation heater 460-2, flows through the supply pipe 224-2, and reaches the three-way valve 250-2. The new medium that has reached the three-way valve 250-2 is guided toward the supply pipe 226-2 and introduced into the culture vessel 110-2 from the inlet 227-2. In this way, the new medium is added to the culture vessel 110-2.

The control device 500-2 transmits a control signal indicating stop to the supply pump 454-2 when a liquid level of a cell suspension becomes higher than the outlet 231-2 from the detection signal output from the liquid level sensor 318-2. As a result, the supply pump 454-2 stops.

The control device 500-2 transmits a control signal indicating stop to the medium heater 456-2. As a result, the medium heater 456-2 stops.

In this way, the new medium can be added to the culture vessel 110-2.

### <Circulation 1 (treatment number PE3)>

The control device 500-2 transmits a control signal indicating operation to the circulation heater 460-2. As a result, the circulation heater 460-2 operates. As a result, the circulation heater 460-2 operates before the medium is circulated. When the medium is circulated, the medium can be accurately heated. Note that the medium heater 456-2 is stopping.

The control device 500-2 transmits a control signal indicating an opened state of the valve 422-2 to the valve 422-2. By opening the valve 422-2, oxygen is supplied from the oxygen supply module 420-2 to the circulating medium.

The control device 500-2 transmits a control signal indicating operation to the supply pump 434-2. By the operation of the supply pump 434-2, the pH adjuster stored in the pH adjuster tank 432-2 is supplied to the circulating medium.

The control device 500-2 transmits a control signal indicating operation to the supply pump 444-2. By the operation of the supply pump 444-2, the glucose concentrate stored in the glucose concentrate tank 442-2 is supplied to the circulating medium.

The control device 500-2 transmits a control signal indicating operation to the circulation pump 210-2. As a result, the circulation pump 210-2 operates. Note that the three-way valve 250-2 is in the medium supply state by the above-described addition (treatment number PE2).

By the operation of the circulation pump 210-2, the medium stored in the culture vessel 110-2 is led out to the circulation feed pipe 232-2 via the outlet 231-2. The medium (circulation feed medium) led out to the circulation feed pipe 232-2 circulates as follows: the culture vessel 110-2 → the outlet 231-2 → the circulation feed pipe 232-2 → the circulation feed pipe 234-2 → the circulation feed pipe 236-2 → the circulation pump 210-2 → the circulation feed pipe 238-2 → the oxygen supply module 420-2 → the supply pipe 222-2 → the circulation heater 460-2 → the supply pipe 224-2 → the supply pipe 226-2 → the inlet 227-2 → the culture vessel 110-2.

In a treatment subsequent thereto, supply of oxygen, supply of the pH adjuster, and supply of the glucose concentrate to the circulating medium are continued.

The control device 500-2 transmits a control signal indicating stop to the circulation pump 210-2 when a liquid level of a cell suspension becomes lower than the outlet 231-2 from the detection signal output from the liquid level sensor 318-2. As a result, the circulation pump 210-2 stops.

### <Medium exchange circulation culture treatment>

After the medium circulation preparation treatment by the above-described (treatment numbers PE1 to PE3), a medium exchange circulation culture treatment is performed.

### <Discharge (treatment number EX1-1)>

The control device 500-2 transmits a control signal indicating operation to the medium heater 456-2 before discharge of the medium. The medium heater 456-2 operates. As a result, the medium heater 456-2 operates before the medium is circulated. When the medium is circulated, the medium can be accurately heated. Note that the circulation heater 460-2 is already in operation. Note that the medium heater 456-2 is stopping.

Next, the control device 500-2 transmits a control signal indicating stop to the supply pumps 434-2 and 444-2. As a result, a pH adjuster and a glucose concentrate are not supplied to the medium. Furthermore, the control device 500-2 transmits a control signal indicating a closed state to the valve 422-2. As a result, oxygen is not supplied to the medium.

Next, the control device 500-2 transmits a control signal indicating a medium disposal state to the three-way valve 250-2. As a result, the three-way valve 250-2 becomes the medium disposal state.

Next, the control device 500-2 transmits a control signal indicating operation to the circulation pump 210-2. As a result, the circulation pump 210-2 operates. By the operation of the circulation pump 210-2, the medium stored in the culture vessel 110-2 is led out to the circulation feed pipe 232-2 via the outlet 231-2. The medium led out to the circulation feed pipe 232-2 is discarded to the waste liquid tank 260-2 via the culture vessel 110-2 → the outlet 231-2 → the circulation feed pipe 232-2 → the circulation feed pipe 234-2 → the circulation feed pipe 236-2 → the circulation pump 210-2 → the circulation feed pipe 238-2 → the oxygen supply module 420-2 → the supply pipe 222-2 → the circulation heater 460-2 → the supply pipe 224-2 → the disposal pipe 262-2. That is, the medium used for growth of cells is discarded.

Next, the control device 500-2 transmits a control signal indicating stop to the circulation pump 210-2 when a liquid level of a cell suspension becomes lower than the outlet 231-2 from the detection signal output from the liquid level sensor 318-2. As a result, the circulation pump 210-2 stops.

### <Supply (treatment number EX1-2)>

The control device 500-2 transmits a control signal indicating a medium supply state to the three-way valve 250-2. As a result, the three-way valve 250-2 becomes the medium supply state.

The control device 500-2 transmits a control signal indicating operation to the medium heater 456-2. As a result, the medium heater 456-2 operates. Note that the circulation heater 460-2 is already in operation.

Next, the control device 500-2 transmits a control signal indicating operation to the supply pump 454-2. As a result, the new medium stored in the medium tank 452-2 is heated by the medium heater 456-2 and led out to the supply pipe 222-2.

The new medium led out to the supply pipe 222-2 passes through the circulation heater 460-2, flows through the supply pipe 224-2, and reaches the three-way valve 250-2. The new medium that has reached the three-way valve 250-2 is guided toward the supply pipe 226-2 and introduced into the culture vessel 110-2.

The control device 500-2 stops the supply pump 454-2 and the medium heater 456-2 when a liquid level of a cell suspension becomes higher than the outlet 231-2 from the detection signal output from the liquid level sensor 318-2. In this way, the new medium can be supplied to the culture vessel 110-2.

### <Circulation 2 (treatment number EX1-3)>

The control device 500-2 transmits a control signal indicating operation to the circulation pump 210-2. As a result, the circulation pump 210-2 operates. Note that the three-way valve 250-2 is in the medium supply state by the above-described addition (treatment number PE2). By the operation of the circulation pump 210-2, the medium stored in the culture vessel 110-2 is led out to the circulation feed pipe 232-2 via the outlet 231-2. The medium led out to the circulation feed pipe 232-2 circulates as follows: the culture vessel 110-2 → the outlet 231-2 → the circulation feed pipe 232-2 → the circulation feed pipe 234-2 → the circulation feed pipe 236-2 → the circulation pump 210-2 → the circulation feed pipe 238-2 → the oxygen supply module 420-2 → the supply pipe 222-2 → the circulation heater 460-2 → the supply pipe 224-2 → the supply pipe 226-2 → the inlet 227-2 → the culture vessel 110-2.

### <Treatment after discharge (treatment number EX1-1), supply (treatment number EX1-2), and circulation 2 (treatment number EX1-3)>

Similarly to the discharge (treatment number EX1-1), the supply (treatment number EX1-2), and the circulation 2 (treatment number EX1-3), the treatments of discharge, supply, and circulation are repeated a plurality of times (EX2-1 to EX2-3, EX3-1 to EX3-3, ...).

By repeating the treatments of discharge, supply, and circulation a plurality of times, the cell aggregate can be grown (enlarged) to a desired final stage.

### <Repetition of discharge of medium, supply of medium, and circulation of medium>

Discharge of the medium, supply of the medium, and circulation of the medium are repeated. The number of repetitions is based on the finally desired sizes and number of cell aggregates. The number of repetitions can be determined by a preliminary experiment or the like.

### <Flow speed by circulation pump 210-2>

A flow rate (flow speed) of the circulating medium per unit time is determined by a rotation speed of the circulation pump 210-2. In the medium exchange circulation culture treatment, cell aggregates are grown to a desired final stage. The cell aggregates become heavy and easily sink to a bottom of the culture vessel 110-2. Therefore, the cell aggregates can be floated by increasing the rotation speed of the circulation pump 210-2 to increase the flow speed of the medium. By floating the cell aggregates, contact thereof with the bottom surface of the culture vessel 110-2 can be prevented.

The rotation speed of the circulation pump 210-2 depending on the size of the cell aggregate can be determined by a preliminary experiment or the like. Furthermore, the size of the cell aggregate depending on an elapsed time from start of culture can also be determined by a preliminary experiment or the like. From these relationships, the rotation speed of the circulation pump 210-2 can be stored in advance in a storage portion such as an auxiliary storage device in association with the elapsed time from the start of culture.

The control device 500-2 reads the rotation speed of the circulation pump 210-2 depending on the elapsed time from the start of culture or the like, and controls the circulation pump 210-2. A drive motor (not illustrated) is rotated depending on growth of the cell aggregate (such as the size of the cell aggregate). The cell aggregate can be accurately grown while being maintained in a floating state.

A pH adjuster, a glucose concentrate, and the amount of dissolved oxygen can be determined depending on the type of cell, the desired size of aggregate, and the like. The pH adjuster, the glucose concentrate, and the amount of dissolved oxygen can also be determined by a preliminary experiment or the like.

The sizes and number of cell aggregates may be acquired by image analysis or the like of data captured by the camera 320-2, and it may be determined whether or not to end the treatment. It is preferable to convert the sizes and number of cell aggregates into an easily obtainable image by performing various types of image processing on the captured image.

### <<<<Details of third embodiment>>>>

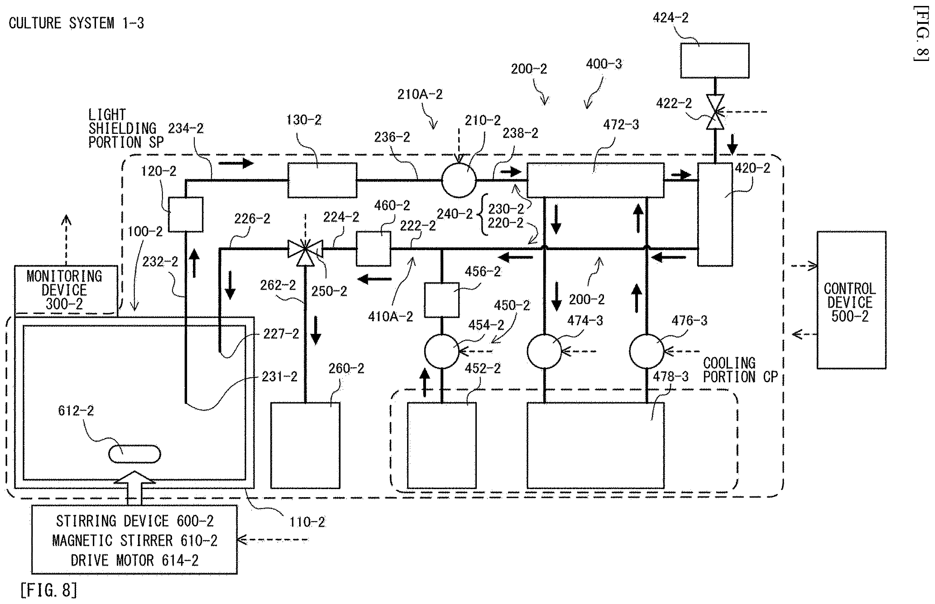

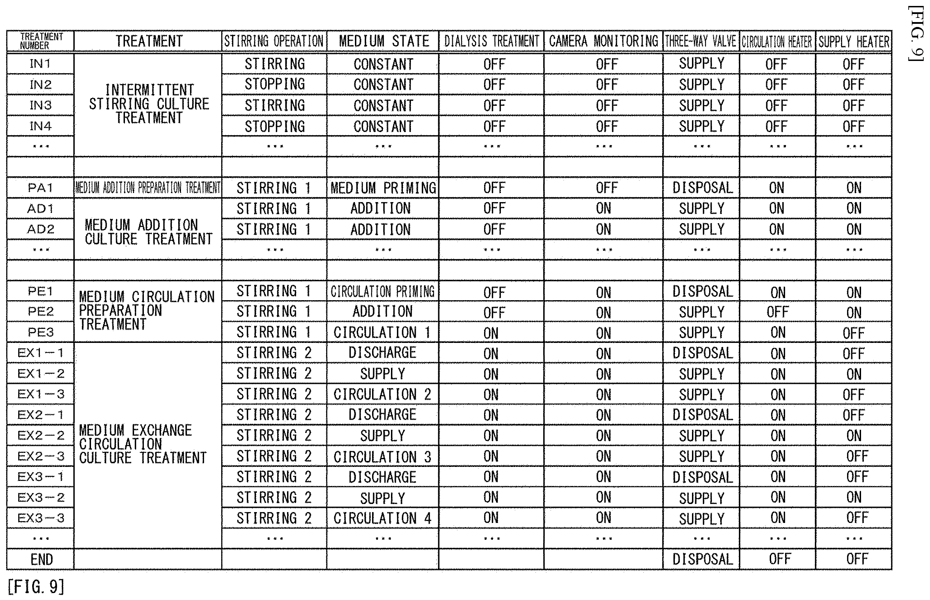

Hereinafter, a third embodiment will be described with reference to the drawings. Fig. 8 is a block diagram illustrating details of a culture system 1-3 according to the third embodiment. Fig. 9 is a time chart illustrating an outline of treatment of the culture system 1-3. Note that a broken line having an arrow illustrated in Fig. 8 indicates input or output of a control-related signal. Two regions surrounded by broken lines in Fig. 8 are a light shielding portion SP indicating a constituent element to be shielded from light and a cooling portion CP indicating a cooled constituent element.

### <<<<Configuration of culture system 1-3>>>

In Fig. 8, constituent elements similar to those of the culture system 1-2 according to the second embodiment illustrated in Fig. 3 are denoted by the same reference numerals. A constituent element different from the culture system 1-2 according to the second embodiment will be described.

The culture system 1-2 according to the second embodiment includes, as the adjustment device 400-2, the pH adjuster supply portion 430-2 (the pH adjuster tank 432-2 and the supply pump 434-2) and the glucose concentrate supply portion 440-2 (the glucose concentrate tank 442-2 and the supply pump 444-2). On the other hand, the culture system 1-3 according to the third embodiment includes an alternative device to the pH adjuster supply portion 430-2 and the glucose concentrate supply portion 440-2.

In addition, the culture system 1-2 according to the second embodiment is a system that performs control under a condition predetermined in a preliminary experiment or the like. On the other hand, in the culture system 1-3 according to the third embodiment, cells and cell aggregates cultured in the culture vessel 110-2 are captured by the camera 320-2. The culture system 1-3 is a system that acquires the size of a cell aggregate or the like from imaging data, and determines a timing of ending culture. Note that culture conditions, particularly a stirring condition and a stirring rate may be determined depending on the size of the acquired cell aggregate.

### <<Adjustment device 400-3>>

An adjustment device 400-3 includes: instead of the pH adjuster supply portion 430-2 and the glucose concentrate supply portion 440-2,
a dialysis module 472-3;
a dialysis circulation feed pump 474-3;
a dialysis supply pump 476-3; and
a dialysate tank 478-3.

Note that the adjustment device 400-3 also includes:
a medium supply portion 450-2;
a medium tank 452-2;
a supply pump 454-2;
a medium heater 456-2; and
a circulation heater 460-2. These have configurations similar to those of the culture system 1-2 according to the second embodiment and function in a similar manner.

### <Dialysis module 472-3>

The dialysis module 472-3 filters and removes waste products and the like discharged along with growth of cells in the culture vessel 110-2, and supplies components (for example, glucose, amino acid, a vitamin, an inorganic salt, and the like) necessary for growth of cells to the medium. The dialysis module 472-3 is disposed in the middle of the circulation feed pipe 238-2.

### <Dialysis circulation feed pump 474-3>

The dialysis circulation feed pump 474-3 leads out a liquid from which waste products and the like have been removed by the dialysis module 472-3 to the dialysate tank 478-3.

### <Dialysis supply pump 476-3>

The dialysis supply pump 476-3 supplies a dialysate stored in the dialysate tank 478-3 to the dialysis module 472-3. As a result, components necessary for growth of cells can be supplied to the medium.

### <Dialysate tank 478-3>

A dialysate is stored in the dialysate tank 478-3. The dialysate sufficiently contains glucose, an amino acid, a vitamin, an inorganic salt, and the like. Examples of the dialysate include DMEM, DMEMHG, EMEM, Iscove's Modified Dulbecco's Medium (IMDM), Glasgow's MEM (GMEM), RPMI-1640, α-MEM, Ham's Medium F-12, Ham's Medium F-10, Ham's Medium F12K, etc.which are the basal media described above. Furthermore, an amino acid, a vitamin, an inorganic salt, a protein (growth factor), glucose, an antibiotic, a signal transduction inhibitor, a reducing agent, a buffer, and the like, which are additives, may be added.

In the culture system 1-2 according to the second embodiment, a pH adjuster and a glucose concentrate are added to the circulating medium as components necessary for growth of cells. On the other hand, in the culture system 1-3 according to the third embodiment, the dialysis circulation feed pump 474-3 and the dialysis supply pump 476-3 are operated using the dialysis module 472-3. As a result, nutrients and the like contained in the dialysate can be added to the medium while waste products and the like contained in the medium are removed via the dialysis module.

### <<Light shielding portion SP>>

As illustrated in Fig. 8, the culture vessel 110-2, the circulation feed pipe 232-2, the circulation feed pipe 234-2, the circulation feed pipe 236-2, the circulation feed pipe 238-2, the cell-medium separation filter 120-2, the sampling port 130-2, the dialysis module 472-3, the oxygen supply module 420-2, the supply pipe 222-2, the supply pipe 224-2, the supply pipe 226-2, the dialysate tank 478-3, and the medium supply portion 450-2 are shielded from light by a light shielding member (light shielding portion SP). By shielding these portions from light, photodegradation and decomposition of an additive or the like can be prevented, and accuracy of a monitoring result by the monitoring portion 30, for example, accuracy of measurement of a medium component, imaging, or the like can be improved.

### <<Cooling portion CP>>

The dialysate tank 478-3 and the medium tank 452-2 are cooled by a low temperature thermostatic bath (cooling portion CP). By cooling the dialysate tank 478-3 and the medium tank 452-2, degradation and decomposition of an additive or the like can be prevented.

### <<<Treatment of culture system 1-3>>

Fig. 9 is a time chart illustrating an outline of treatment of the culture system 1-3. In the treatment of the culture system 1-3, the dialysis module 472-3 and the camera 320-2 are used. Note that, hereinafter, a treatment similar to those of the culture system 1-2 will be omitted.

As described above, the camera 320-2 images cells and cell aggregates cultured in the culture vessel 110-2. The size of the cell aggregate and the like can be acquired from imaging data captured by the camera 320-2.

As illustrated in Fig. 9, the intermittent stirring culture treatment (treatment number IN1 and subsequent numbers) and the medium addition preparation treatment (PA1) are performed in a similar manner to the culture system 1-2.

### <<Treatment by camera monitoring>>

From the medium addition preparation treatment (AD1 and subsequent numbers) to an end, the control device 500-2 performs a treatment by camera monitoring. The treatment by camera monitoring is a treatment using the size of a cell aggregate acquired from imaging data captured by the camera 320-2.

The control device 500-2 determines whether or not the size of the cell aggregate has become equal to or larger than a predetermined size, and when the size of the cell aggregate has become equal to or larger than the predetermined size, the control device 500-2 controls a rotation speed (stirring rate) of the drive motor 614-2 of the stirring device 600-2 and a flow rate of the circulation pump 210-2 depending on the size of the cell aggregate. In this way, suitable control can be performed according to growth of the cell aggregate.

### <<<Treatment using dialysis module 472-3>>>

From the medium circulation preparation treatment (PE2 and subsequent numbers) to an end, the control device 500-2 causes the dialysis circulation feed pump 474-3 and the dialysis supply pump 476-3 to operate using the monitored glucose concentration, lactic acid concentration, and pH as indices. For example, the control device 500-2 causes the dialysis circulation feed pump 474-3 and the dialysis supply pump 476-3 to operate depending on a flow rate of the circulation pump 210-2, a glucose concentration, a lactic acid concentration, and pH. In this way, glucose, an amino acid, a vitamin, an inorganic salt, and the like can be added to the medium depending on the flow rate of the medium.

### <<<<Details of fourth embodiment>>>>

The conventional cell culture system is on the basis of use of a filter that allows a cell culture solution to pass therethrough and does not allow a cell or a cell aggregate to pass therethrough. For this reason, there is a possibility that the cells or the cell aggregates are locally pressed against the filter, and the cells or the cell aggregates are damaged or divided. The culture system 1-1 according to the first embodiment, the culture system 1-2 according to the second embodiment, and the culture system 1-3 according to the third embodiment are made in view of such a point. An object of the culture systems 1-1, 1-2, and 1-3 is to provide a culture system capable of accurately growing a cell aggregate.

### <<<<Details of fourth embodiment>>>>

By accurately growing cell aggregates by the culture systems 1-1, 1-2, and 1-3, cells can be enlarged and cultured. The enlarged and cultured cell aggregates include cell aggregates having various particle sizes and have a particle size distribution. The particle size distribution varies depending on the type or lot of cell, culture conditions, and the like. A required particle size range varies depending on an application of cells. For example, when cells are used for cell therapy, the particle size of cell aggregate largely affects the quality and productivity of cells.

In consideration of these, it is necessary to set an optimum range of the particle size of the cell aggregate. In addition, in a case where a cell aggregate is transplanted into a tissue using an injection needle, it is necessary to make the particle size of cell aggregate smaller than at least the diameter of the injection needle. Therefore, it is desired to provide a system capable of recovering cell aggregates in a necessary particle size range by classifying the cell aggregates in a closed system after enlarging and culturing the cell aggregates.

An object of the fourth embodiment is to provide a system that classifies a cell aggregates and recovers cell aggregates having a particle size in a desired range. Note that provision of only a system that classifies cell aggregates and only a system that recovers cell aggregates are also included in the object of the fourth embodiment.

### <<Typical procedure of classifying and recovering cell aggregates>>

A sedimentation rate of a cell aggregate in a liquid varies depending on a particle size and a density, that is, a mass. Note that not only the particle size and density related to the mass of the cell aggregate but also a parameter affecting the sedimentation rate of the cell aggregate may be considered. For example, a parameter capable of directly or indirectly deriving the mass of the cell aggregate or a parameter related to a medium, such as a viscosity or a temperature of the medium may also be considered. The cell aggregates can be taken out (extracted) from the culture vessel by suctioning the cell aggregates at a flow speed against the sedimentation rate. The flow speed is controlled by a flow rate of suction and a cross-sectional area of a suction pipe nozzle to adjust the mass of a cell aggregate that can be taken out from the culture vessel. For example, when the enlarged and cultured cell aggregates have a particle size distribution of dA to dB, cell aggregates in a typical range indicated in the following (a) to (c) can be classified and recovered. Note that it is assumed that dA ≤ dX <dY ≤ dB is satisfied.
(a) Cell aggregates having a particle size of less than dX or less than dY
(b) Cell aggregates having a particle size of dX or more and less than dY
(c) Cell aggregates having a particle size of dX or more or dY or more

Furthermore, a cross-sectional area of the suction pipe nozzle is represented by A, and a flow rate of suction is represented by Qx, Qy, or Qall. Qx/A is a flow speed at which cell aggregates having a particle size of less than dX can be sucked into the suction pipe nozzle. Qy/A is a flow speed at which cell aggregates having a particle size of less than dY can be sucked into the suction pipe nozzle. Qall/A is a flow speed at which cell aggregates having a particle size of dA to dB can be sucked into the suction pipe nozzle.

### <(a) Cell aggregates having a particle size of less than dX or less than dY>

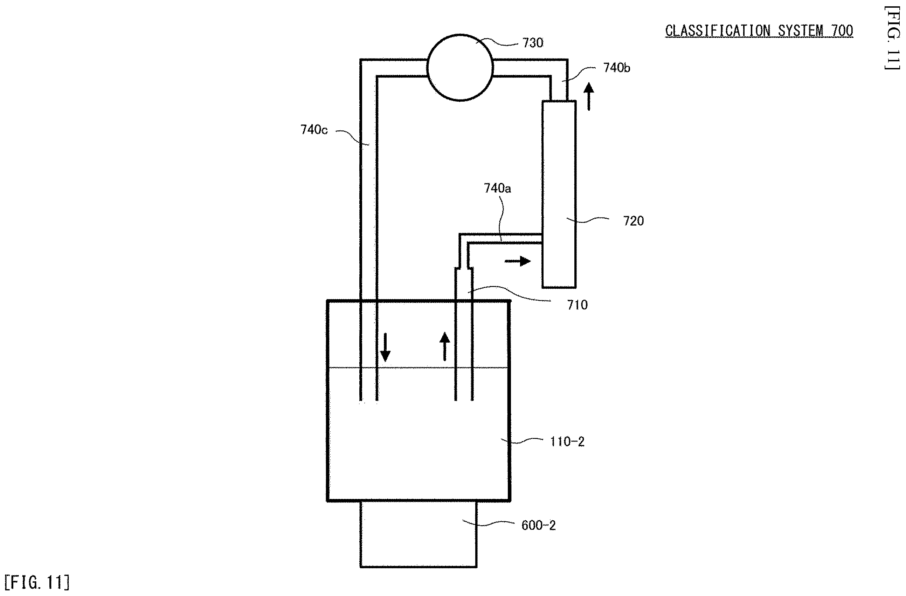

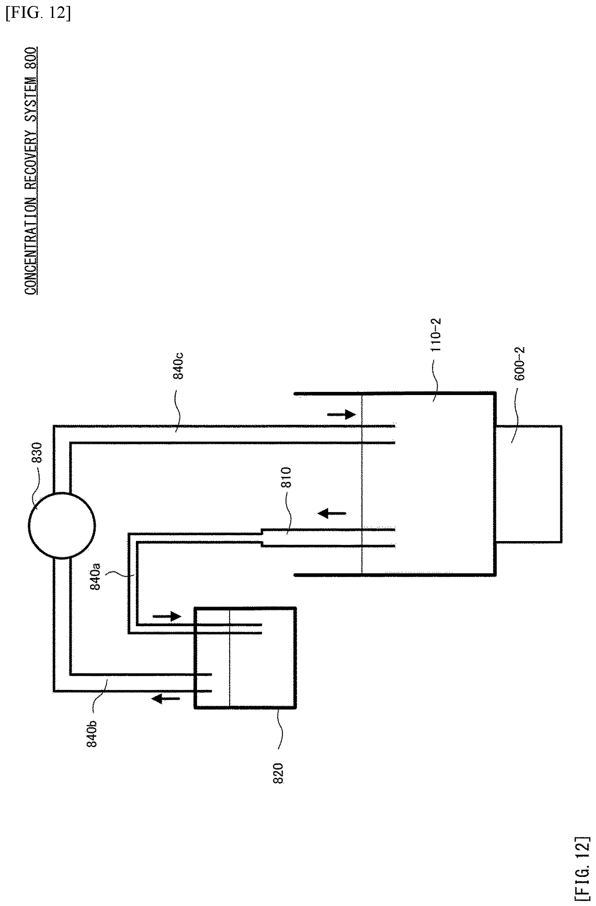

That is, this is a case of extracting cell aggregates having a small particle size (light cell aggregates). In this case, by performing suction at a flow rate Qx or Qy using a suction pipe nozzle having a cross-sectional area A, cell aggregates having a desired particle size of less than dX or less than dY are taken out from the culture vessel, and can be thereby immediately recovered in a recovery vessel. Note that a specific configuration including the culture vessel, the recovery vessel, and the like will be described with reference to Figs. 11 and 12 and the like.

### <(b) Cell aggregates having a particle size of dX or more and less than dY>

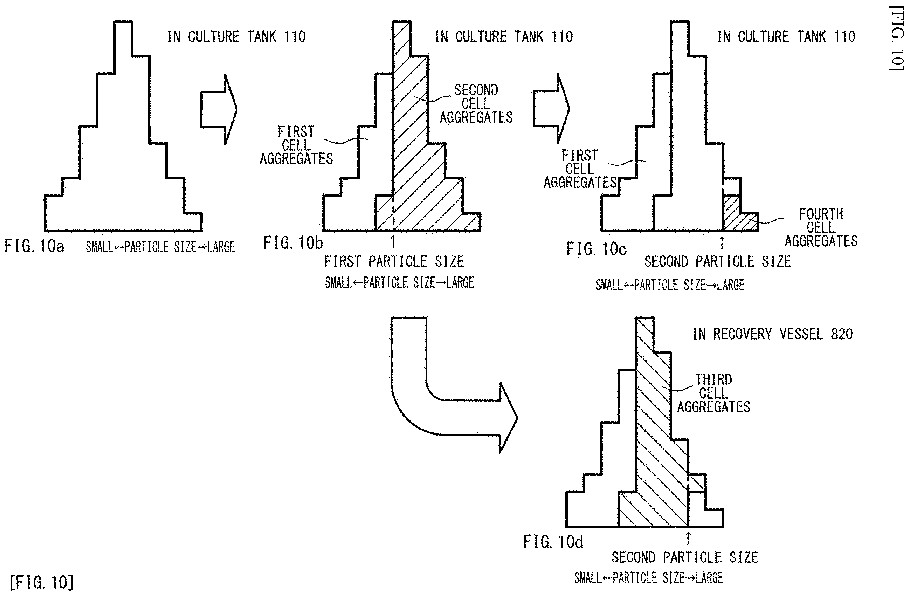

That is, this is a case of extracting cell aggregates having a medium particle size (cell aggregates having a medium weight). In this case, by first performing suction at a flow rate Qx using a suction pipe nozzle having a cross-sectional area A, cell aggregates having a particle size of less than dX are taken out from the culture vessel and classified (collected). Subsequently, by performing suction at a flow rate Qy, cell aggregates having a desired particle size of dX or more and less than dY, that is, a particle size of dX to dY can be taken out from the culture vessel and recovered in the recovery vessel. Note that details of the procedure of the classification treatment and the recovery treatment are illustrated in Fig. 10. Note that a specific configuration including the culture vessel, the recovery vessel, and the like will be described with reference to Figs. 11 and 12 and the like.

### <(c) Cell aggregates having a particle size of dX or more or dY or more>

That is, this is a case of extracting cell aggregates having a large particle size (heavy cell aggregates). First, cell aggregate having a particle size of dX or more can be taken out and recovered from the culture vessel to the recovery vessel by performing suction at Qx using a suction pipe nozzle having a cross-sectional area A to classify cell aggregates having a particle size of less than dX, and then performing suction at Qall. Furthermore, for cell aggregates having a particle size of dY or more, the following two procedures are possible. As a first procedure, cell aggregates having a particle size of less than dX are classified by performing suction at Qx using a suction pipe nozzle having a cross-sectional area A, and then suction is further performed at Qy, whereby cell aggregates having a particle size of less than dY can be classified. In addition, as a second procedure, initially, cell aggregates having a particle size of less than dY are classified by performing suction at Qy using a suction pipe nozzle having a cross-sectional area A, and then suction is performed at Qall, whereby cell aggregates having a particle size of dY or more can be taken out and recovered from the culture vessel to the recovery vessel. In this way, cell aggregates having a desired particle size of dX or more or dY or more can be taken out and recovered from the culture vessel. Note that a specific configuration including the culture vessel, the recovery vessel, and the like will be described with reference to Figs. 11 and 12 and the like.

In any one of the cases (a) to (c), the cell aggregates taken out from the culture vessel are recovered in the recovery vessel. A liquid amount can be reduced by using a vessel having a smaller volume than the culture vessel as the recovery vessel for recovering the cell aggregates. Depending on the volume of the recovery vessel, the cell aggregates are recovered while being concentrated.

### <<<Process of classification treatment and concentration recovery treatment>>

A process of the classification treatment and the concentration recovery treatment will be described with reference to Figs. 10, 11, and 12. The process of the classification treatment and the concentration recovery treatment here is a process for extracting (b) cell aggregates having a particle size of dX or more and less than dY. Note that, hereinafter, the particle size of dX is referred to as a first particle size, and the particle size of dY is referred to as a second particle size.

Fig. 10 is a graph illustrating a process of subjecting cell aggregates to a classification treatment and then subjecting the cell aggregates to a concentration recovery treatment by a change in a particle size distribution of the cell aggregates. The horizontal axis of the graph in Fig. 10 indicates a particle size, and the particle size increases as going rightward and decreases as going leftward. The vertical axis of the graph in Fig. 10 indicates the number of cell aggregates.

Fig. 11 is a schematic diagram illustrating a configuration of a classification system 700 for classifying cell aggregates. Fig. 12 is a schematic diagram illustrating a configuration of a concentration recovery system 800 for concentrating and recovering cell aggregates. In Figs. 11 and 12, components common to those of the culture system 1-2 illustrated in Fig. 3 are denoted by the same reference numerals.

### <<State before treatment>>

The classification treatment and the concentration recovery treatment are started from a state where the cell aggregates after completion of the culture treatment are contained in the culture vessel 110-2 (Fig. 10a).

### <<Classification treatment>>

The classification treatment is a treatment of distributing the cell aggregates into first cell aggregates having a size smaller than the first particle size and second cell aggregates having a size equal to or larger than the first particle size in an environment containing the medium.

### <Configuration of classification system 700>

By the classification treatment by the classification system 700 illustrated in Fig. 11, the cell aggregates contained in the culture vessel 110-2 (see Fig. 11) are distributed to the first cell aggregates and the second cell aggregates (Fig. 10b).

As illustrated in Fig. 11, the classification system 700 mainly includes a suction pipe nozzle 710, a classification module 720, and a pump 730. The classification system 700 forms a circuit that circulates a liquid from the culture vessel 110-2 containing the cell aggregates with the pump 730.

The suction pipe nozzle 710 is connected to the classification module 720 via a pipe 740a. The size of a cross-sectional area of the suction pipe nozzle 710 is equal to or larger than the size of a cross-sectional area of the pipe 740a. With this configuration, it is possible to prevent the cell aggregates from staying in a region including a portion where the suction pipe nozzle 710 and the pipe 740a are connected to each other. The classification module 720 is connected to the pump 730 via a pipe 740b. Furthermore, a pipe 740c is connected to the pump 730.

The suction pipe nozzle 710 has a substantially cylindrical shape having a constant diameter (cross-sectional area). The suction pipe nozzle 710 has a constant cross-sectional area A1. The suction pipe nozzle 710 is disposed such that an axial direction thereof is along the vertical direction. The suction pipe nozzle 710 has an elongated shape, and is disposed such that a longitudinal direction thereof is along the vertical direction.

The classification module 720 has a substantially cylindrical shape having a constant diameter (cross-sectional area). The classification module 720 has a constant cross-sectional area A2. The classification module 720 is disposed such that an axial direction thereof is along the vertical direction. The classification module 720 has an elongated shape, and is disposed such that a longitudinal direction thereof is along the vertical direction.

The cross-sectional area A1 of the suction pipe nozzle 710 is smaller than the cross-sectional area A2 of the classification module 720. Since the diameter (cross-sectional area) of the suction pipe nozzle 710 is different from the diameter (cross-sectional area) of the classification module 720, a flow speed generated in the suction pipe nozzle 710 can be made different from a flow speed generated in the classification module 720 using the single pump 730.

### <Classification treatment by classification system 700>

First, the cell aggregates in the culture vessel 110-2 are dispersed by the stirring device 600-2.

The cell aggregates to be classified (collected) are taken out from the culture vessel 110-2 via the suction pipe nozzle 710 having the cross-sectional area A1 by a circulation flow rate Q1 by driving of the pump 730. The cross-sectional area A2 of the classification module 720 is larger than the cross-sectional area A1 of the suction pipe nozzle 710 (A1 < A2). Therefore, the flow speed Q1/A2 in the classification module 720 is smaller than the flow speed Q1/A1 in the suction pipe nozzle 710. With this configuration, a liquid moves in the classification module 720, and the cell aggregates can be caused to stay in the classification module 720.

The circulation flow rate Q1 only needs to be determined in advance depending on a range of the particle size (mass) of the cell aggregate to be taken out from the culture vessel 110-2 via the suction pipe nozzle 710 having the cross-sectional area A1. Note that, when the classification treatment is performed, the circulation flow rate Q1 may be constant or may be changed stepwise.

By driving the pump 730, the cell aggregates are sucked into the classification module 720 together with the liquid from the culture vessel 110-2 via the suction pipe nozzle 710. The classification treatment is a treatment of distributing the cell aggregates by causing the cell aggregates to stay in the classification module 720 along with flow of the liquid against gravity sedimentation of the cell aggregates sucked into the classification module 720.

The cell aggregates sucked into the classification module 720 can be stored at a bottom of the classification module 720 depending on the circulation flow rate Q1. Note that the cell aggregates sucked into the classification module 720 need not be stored at the bottom of the classification module 720 as long as they can be caused to stay in the classification module 720.

By the classification treatment, the first cell aggregates having a size smaller than the first particle size move from the culture vessel 110-2 to the classification module 720 together with the liquid, and stay in the classification module 720. On the other hand, the second cell aggregates having a size equal to or larger than the first particle size do not move to the classification module 720 and stay in the culture vessel 110-2 (Fig. 10b). In this way, the cell aggregates can be distributed to the first cell aggregates and the second cell aggregates.

Note that the liquid sucked into the classification module 720 by driving of the pump 730 is discharged from the classification module 720 and returned to the culture vessel 110-2 via the pipes 740b and 740c.

### <<Concentration recovery treatment>>

The concentration recovery treatment is a treatment of further distributing the second cell aggregates (Fig. 10b) staying in the culture vessel 110-2 after the classification treatment to third cell aggregates (Fig. 10d) having a size smaller than the second particle size and fourth cell aggregates (Fig. 10c) having a size equal to or larger than the second particle size in an environment containing the medium. The second particle size is larger than the first particle size of the classification treatment.

### <<Configuration of concentration recovery system 800>>

The second cell aggregates (Fig. 10b) staying in the culture vessel 110-2 by the classification treatment by the classification system 700 are distributed to the third cell aggregates (Fig. 10d) and the fourth cell aggregates (Fig. 10c) by the concentration recovery treatment by the concentration recovery system 800.

As illustrated in Fig. 12, the concentration recovery system 800 mainly includes a suction pipe nozzle 810, a recovery vessel 820, and a pump 830. The concentration recovery system 800 forms a circuit that circulates a liquid from the culture vessel 110-2 containing the cell aggregates with the pump 830.

The suction pipe nozzle 810 is connected to the recovery vessel 820 via a pipe 840a. The size of a cross-sectional area of the suction pipe nozzle 810 is equal to or larger than the size of a cross-sectional area of the pipe 840a. With this configuration, it is possible to prevent the cell aggregates from staying in a region including a portion where the suction pipe nozzle 810 and the pipe 840a are connected to each other. The recovery vessel 820 is connected to the pump 830 via a pipe 840b. A pipe 840c is connected to the pump 830. Note that the size of the cross-sectional area of the pipe 840a, the size of a cross-sectional area of the pipe 840b, and the size of a cross-sectional area of the pipe 840c are not particularly limited.

The suction pipe nozzle 810 has a substantially cylindrical shape having a constant diameter (cross-sectional area). Similarly to the suction pipe nozzle 710, the suction pipe nozzle 810 has the constant cross-sectional area A1. The suction pipe nozzle 810 is disposed such that an axial direction thereof is along the vertical direction. The suction pipe nozzle 810 has an elongated shape, and is disposed such that a longitudinal direction thereof is along the vertical direction.

The recovery vessel 820 has a substantially cylindrical shape having a constant diameter (cross-sectional area). The recovery vessel 820 has a constant cross-sectional area A3. The recovery vessel 820 is disposed such that an axial direction thereof is along the vertical direction.

The diameter (cross-sectional area) of the suction pipe nozzle 810 is different from the diameter (cross-sectional area) of the recovery vessel 820. The cross-sectional area A1 of the suction pipe nozzle 810 is smaller than the cross-sectional area A3 of the recovery vessel 820. Since the diameter (cross-sectional area) of the suction pipe nozzle 810 is different from the diameter (cross-sectional area) of the recovery vessel 820, a flow speed generated in the suction pipe nozzle 810 can be made different from a flow speed generated in the recovery vessel 820 using the single pump 830.

### <Concentration recovery treatment by concentration recovery system 800>

First, the second cell aggregates in the culture vessel 110-2 are dispersed by the stirring device 600-2.

The cell aggregates to be concentrated and recovered are taken out from the culture vessel 110-2 via the suction pipe nozzle 810 having the cross-sectional area A1 by a circulation flow rate Q2 by driving of the pump 830. The cross-sectional area A3 of the recovery vessel 820 is larger than the cross-sectional area A1 of the suction pipe nozzle 810 (A1 < A3). Therefore, the flow speed Q1/A3 in the recovery vessel 820 is smaller than the flow speed Q1/A1 in the suction pipe nozzle 810. With this configuration, a liquid moves in the recovery vessel 820, and the cell aggregates can be caused to stay in the recovery vessel 820.

The circulation flow rate Q2 only needs to be determined depending on a range of the particle size (mass) of the cell aggregate to be taken out from the culture vessel 110-2 via the suction pipe nozzle 810 having the cross-sectional area A1. Note that, when concentration and recovery are performed, the stirring rate and the circulation flow rate Q2 may be constant or may be changed depending on a concentration of the cell aggregates in the culture vessel 110-2 or the like.

By driving the pump 830, the cell aggregates are sucked into the recovery vessel 820 together with the liquid from the culture vessel 110-2 via the suction pipe nozzle 810. The concentration recovery treatment is a treatment of distributing the cell aggregates by causing the cell aggregates to stay in the recovery vessel 820 along with flow of the liquid against gravity sedimentation of the cell aggregates sucked into the recovery vessel 820.

By the concentration recovery treatment, the third cell aggregates having a size smaller than the second particle size move from the culture vessel 110-2 to the recovery vessel 820 together with the liquid, and stay in the recovery vessel 820 (Fig. 10d). On the other hand, the fourth cell aggregates having a size equal to or larger than the second particle size do not move to the recovery vessel 820 and stay in the culture vessel 110-2 (Fig. 10c). In this way, the cell aggregates can be distributed to the third cell aggregates and the fourth cell aggregates.

Note that the liquid sucked into the recovery vessel 820 by driving of the pump 830 is discharged from the recovery vessel 820 and returned to the culture vessel 110-2 via the pipes 840b and 840c.

### <Other treatment 1 of classification treatment and concentration recovery treatment>

Note that Fig. 10 is a diagram for illustrating a concept of the classification treatment and the concentration recovery treatment. As illustrated in Fig. 10a, the cell aggregates after culture are initially contained in the culture vessel 110-2, and then, as illustrated in Fig. 10b, the cell aggregates are subjected to the classification treatment. However, in the culture classification system 4-1, the culture classification concentration recovery system 4-2, and the like, the classification treatment can be sequentially performed during the culture treatment.

### <Other treatment 2 of classification treatment and concentration recovery treatment>

In addition, the configuration in which the classification treatment is performed and then the concentration recovery treatment is subsequently performed has been described, but a configuration in which only the classification treatment is performed or a configuration in which only the concentration recovery treatment is performed can be adopted depending on a purpose.

### <Particle size and mass (weight) of cell aggregate>

In the description of Fig. 10, for clarity, the description has been given in which the cell aggregates are distributed depending on the particle size of the cell aggregate. However, in the classification treatment and the concentration recovery treatment, gravity sedimentation of the cell aggregates is used. Therefore, the first particle size corresponds to a first mass (weight), and the second particle size corresponds to a second mass (weight). Therefore, the classification treatment and the concentration recovery treatment can also be said to be a treatment of distributing the cell aggregates depending on the mass (weight) of the cell aggregate.

### <<<Culture classification system 4-1>>>

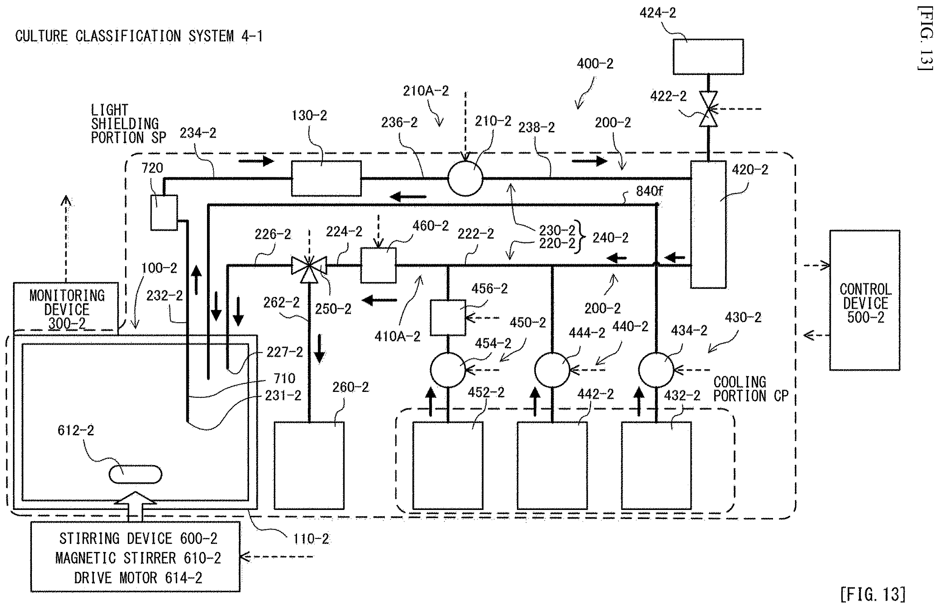

Fig. 13 is a block diagram illustrating a culture classification system 4-1 in which the classification module 720 for classifying cell aggregates is incorporated into the culture system 1-2 (Fig. 3). Note that, in the culture classification system 4-1 illustrated in Fig. 13, components common to those of the culture system 1-2 illustrated in Fig. 3 and the classification system 700 illustrated in Fig. 11 are denoted by the same reference numerals.

Note that the monitoring device 300-2 of the culture classification system 4-1 includes a sensor and a device (not illustrated) for measuring a particle size distribution in addition to the temperature sensor 312-2, the dissolved oxygen sensor 314-2, the pH sensor 316-2, and the liquid level sensor 318-2. As the cell aggregates are cultured, a particle size distribution of the cell aggregates can be acquired.

### <<Configuration of culture classification system 4-1>>

In the culture classification system 4-1, as illustrated in Fig. 13, the suction pipe nozzle 710 and the classification module 720 of the classification system 700 are used instead of the cell-medium separation filter 120-2 in Fig. 3. As described in Fig 11, the suction pipe nozzle 710 has the constant cross-sectional area A1, and the classification module 720 has the constant cross-sectional area A2. The cross-sectional area A1 of the suction pipe nozzle 710 is smaller than the cross-sectional area A2 of the classification module 720.

Note that, in the culture classification system 4-1, the classification module 720 is used instead of the cell-medium separation filter 120-2, but the cell-medium separation filter 120-2 may be used together with the classification module 720. Whether or not to use the cell-medium separation filter 120-2 can be appropriately selected.

Note that, in the culture classification system 4-1, the circulation pump 210-2 is used instead of the pump 730 of the classification system 700 in Fig. 11. By appropriately setting a rotation speed of a motor (not illustrated) constituting the circulation pump 210-2, flow speeds in the suction pipe nozzle 710 and the classification module 720 can be adjusted.

As described in Fig 11, the first cell aggregates having a size smaller than the first particle size move from the culture vessel 110-2 to the classification module 720 together with a liquid via the suction pipe nozzle 710, and stay in the classification module 720. In addition, the second cell aggregates having a size equal to or larger than the first particle size do not move to the classification module 720 and stay in the culture vessel 110-2. In this way, the cell aggregates can be classified into the first cell aggregates and the second cell aggregates. In the culture classification system 4-1, by using the culture classification system 4-1, by adjusting flow speeds in the suction pipe nozzle 710 and the classification module 720, it is possible to distribute the cell aggregates to the first cell aggregates and the second cell aggregates while culturing the cell aggregates in the culture vessel 110-2 in the process of culturing the cell aggregates.

### <<Classification treatment in culture classification system 4-1>>

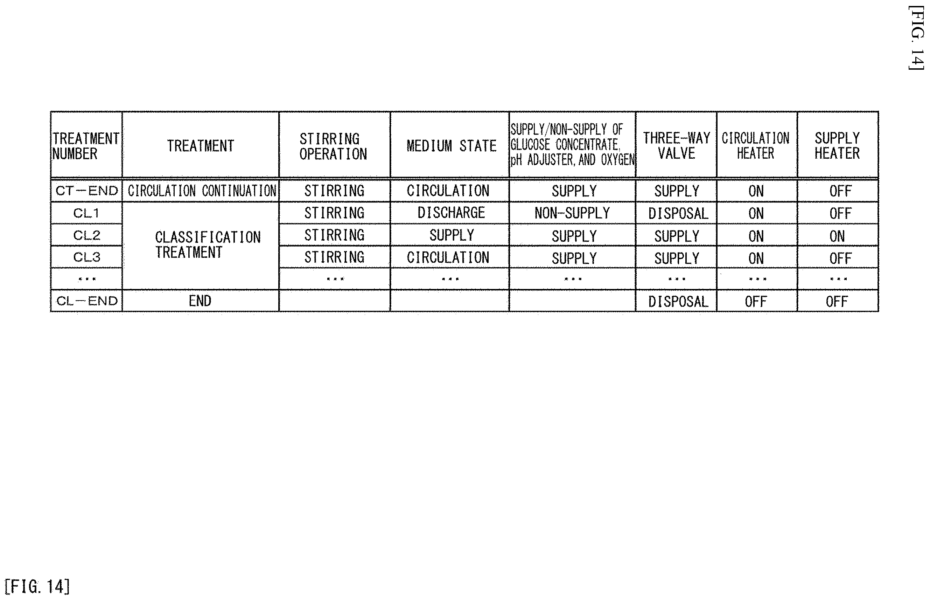

Fig. 14 is a time chart illustrating a classification treatment in the culture classification system 4-1 illustrated in Fig. 13. Hereinafter, a classification treatment in the culture classification system 4-1 will be described with reference to the time chart illustrated in Fig. 14.

Note that, when all the steps of culturing the cell aggregates are completed, the process proceeds to "circulation continuation" of CT-END illustrated in Fig. 14. Subsequently, the process proceeds to classification treatments CL1, CL2, CL3, .... Transition from the "circulation continuation" of CT-END to the classification treatment CL1 may be performed under control of the control device 500-2 or manually by an operator. As soon as the classification treatment CL1 is started, medium exchange is started. The medium exchange is performed at set time intervals. When all the classification treatments (CL1, ...) are completed, the process proceeds to a recovery treatment. Transition from the classification treatment to the recovery treatment may be performed under control of the control device 500-2 or manually by an operator. Hereinafter, details of each step will be described.

### <CT-END (circulation continuation)>

The control device 500-2 transmits a control signal indicating operation to the circulation heater 460-2.

As a result, the circulation heater 460-2 operates. The circulation heater 460-2 operates before the medium is circulated. When the medium is circulated, the medium can be accurately heated. Note that the medium heater 456-2 is stopping.

The control device 500-2 transmits a control signal indicating an opened state of the valve 422-2 to the valve 422-2. By opening the valve 422-2, oxygen is supplied from the oxygen supply module 420-2 to the circulating medium.

The control device 500-2 transmits a control signal indicating operation to the supply pump 434-2. By the operation of the supply pump 434-2, the pH adjuster stored in the pH adjuster tank 432-2 is supplied to the circulating medium.

The control device 500-2 transmits a control signal indicating operation to the supply pump 444-2. By the operation of the supply pump 444-2, the glucose concentrate stored in the glucose concentrate tank 442-2 is supplied to the circulating medium.

The control device 500-2 transmits a control signal indicating operation to the circulation pump 210-2 and the three-way valve 250-2. As a result, the circulation pump 210-2 and the three-way valve 250-2 operate.

By the operation of the circulation pump 210-2, the medium stored in the culture vessel 110-2 is led out to the suction pipe nozzle 710 via the outlet 231-2. The medium (circulation feed medium) led out to the suction pipe nozzle 710 circulates as follows: the culture vessel 110-2 → the outlet 231-2 → the suction pipe nozzle 710 → the circulation feed pipe 232-2 → the classification module 720 → the circulation feed pipe 234-2 → the circulation feed pipe 236-2 → the circulation pump 210-2 → the circulation feed pipe 238-2 → the oxygen supply module 420-2 → the supply pipe 222-2 → the circulation heater 460-2 → the supply pipe 224-2 → the supply pipe 226-2 → the inlet 227-2 → the culture vessel 110-2.

The control device 500-2 can also transmit a control signal including information indicating a rotation speed of the circulation pump 210-2 to the circulation pump 210-2. The circulation pump 210-2 operates at a rotation speed indicated by the control signal. The control device 500-2 transmits a control signal to the circulation pump 210-2 such that the circulation pump 210-2 operates at a predetermined rotation speed.

The predetermined rotation speed is a rotation speed at which a flow speed in the suction pipe nozzle 710 and a flow speed in the classification module 720 are each a predetermined flow speed. The flow speed in the suction pipe nozzle 710 and the flow speed in the classification module 720 are each a flow speed at which the first cell aggregates having a size of a cell aggregate smaller than the first particle size (first mass) stay in the classification module 720 and the second cell aggregates having a size equal to or larger than the first particle size (first mass) are not sucked by the suction pipe nozzle 710. The rotation speed of the circulation pump 210-2 can be determined by a preliminary experiment or the like, and is stored in advance in an auxiliary storage device or the like in association with the flow rate per unit time.

The control device 500-2 controls a flow rate of the circulation pump 210-2 so as to be a predetermined flow rate. By controlling the flow rate, among the cell aggregates stored in the culture vessel 110-2, the first cell aggregates having a size of a cell aggregate smaller than the first particle size (first mass) can be moved from a culture tank 110 to the classification module 720 together with the liquid via the suction pipe nozzle 710, and can be caused to stay in the classification module 720. Furthermore, the second cell aggregates having a size equal to or larger than the first particle size (first mass) can be caused to stay in the culture tank 110 without being moved to the classification module 720.

### <CL1 (classification treatment)>

The control device 500-2 transmits a control signal indicating operation to the medium heater 456-2 before discharge of the medium. The medium heater 456-2 operates. As a result, the medium heater 456-2 operates before the medium is circulated. When the medium is circulated, the medium can be accurately heated. Note that the circulation heater 460-2 is already in operation. In addition, the medium heater 456-2 is stopping.

Next, the control device 500-2 transmits a control signal indicating stop to the supply pumps 434-2 and 444-2. As a result, a pH adjuster and a glucose concentrate are not supplied to the medium. Furthermore, the control device 500-2 transmits a control signal indicating a closed state to the valve 422-2. As a result, oxygen is not supplied to the medium.

Next, the control device 500-2 transmits a control signal indicating a medium disposal state to the three-way valve 250-2. As a result, the three-way valve 250-2 becomes the medium disposal state.

Next, the control device 500-2 transmits a control signal indicating operation to the circulation pump 210-2. As a result, the circulation pump 210-2 operates. By the operation of the circulation pump 210-2, the medium stored in the culture vessel 110-2 is led out to the suction pipe nozzle 710 via the outlet 231-2. The medium led out to the suction pipe nozzle 710 is discarded to the waste liquid tank 260-2 via the culture vessel 110-2 → the outlet 231-2 → the suction pipe nozzle 710 → the circulation feed pipe 232-2 → the classification module 720 → the circulation feed pipe 234-2 → the circulation feed pipe 236-2 → the circulation pump 210-2 → the circulation feed pipe 238-2 → the oxygen supply module 420-2 → the supply pipe 222-2 → the circulation heater 460-2 → the supply pipe 224-2 → the disposal pipe 262-2. That is, the medium used for growth of cells is discarded.

Also in CL1 (classification treatment), similarly to CT-END (circulation continuation), the control device 500-2 controls the flow rate of the circulation pump 210-2 so as to be a predetermined flow rate. By controlling the flow rate, also in CL1 (classification treatment), among the cell aggregates stored in the culture vessel 110-2, the first cell aggregates having a size of a cell aggregate smaller than the first particle size (first mass) can be moved from the culture tank 110 to the classification module 720 together with the liquid via the suction pipe nozzle 710, and can be caused to stay in the classification module 720. Furthermore, the second cell aggregates having a size equal to or larger than the first particle size (first mass) can be caused to stay in the culture tank 110 without being moved to the classification module 720.

### <CL2 (classification treatment)>

The control device 500-2 transmits a control signal indicating a medium supply state to the three-way valve 250-2. As a result, the three-way valve 250-2 becomes the medium supply state.

The control device 500-2 transmits a control signal indicating operation to the medium heater 456-2. As a result, the medium heater 456-2 operates. Note that the circulation heater 460-2 is already in operation.

The control device 500-2 transmits a control signal indicating an opened state of the valve 422-2 to the valve 422-2. By opening the valve 422-2, oxygen is supplied from the oxygen supply module 420-2 to the circulating medium.

The control device 500-2 transmits a control signal indicating operation to the supply pump 434-2. By the operation of the supply pump 434-2, the pH adjuster stored in the pH adjuster tank 432-2 is supplied to the circulating medium.

The control device 500-2 transmits a control signal indicating operation to the supply pump 444-2. By the operation of the supply pump 444-2, the glucose concentrate stored in the glucose concentrate tank 442-2 is supplied to the circulating medium.

Next, the control device 500-2 transmits a control signal indicating operation to the supply pump 454-2. As a result, the new medium stored in the medium tank 452-2 is heated by the medium heater 456-2 and led out to the supply pipe 222-2.

The new medium led out to the supply pipe 222-2 passes through the circulation heater 460-2, flows through the supply pipe 224-2, and reaches the three-way valve 250-2. The new medium that has reached the three-way valve 250-2 is guided toward the supply pipe 226-2 and introduced into the culture vessel 110-2.

### <CL3 (classification treatment)>

The control device 500-2 transmits a control signal indicating operation to the circulation pump 210-2. As a result, the circulation pump 210-2 operates. Note that the three-way valve 250-2 is in the medium supply state by CL2 (classification treatment).

By the operation of the circulation pump 210-2, the medium stored in the culture vessel 110-2 is led out to the suction pipe nozzle 710 via the outlet 231-2. The medium led out to the suction pipe nozzle 710 circulates as follows: the culture vessel 110-2 → the outlet 231-2 → the suction pipe nozzle 710 → the circulation feed pipe 232-2 → the classification module 720 → the circulation feed pipe 234-2 → the circulation feed pipe 236-2 → the circulation pump 210-2 → the circulation feed pipe 238-2 → the oxygen supply module 420-2 → the supply pipe 222-2 → the circulation heater 460-2 → the supply pipe 224-2 → the supply pipe 226-2 → the inlet 227-2 → the culture vessel 110-2.

Also in CL3 (classification treatment), similarly to CT-END (circulation continuation) and CL1 (classification treatment), the control device 500-2 controls the flow rate of the circulation pump 210-2 so as to be a predetermined flow rate. By controlling the flow rate, also in CL3 (classification treatment), among the cell aggregates stored in the culture vessel 110-2, the first cell aggregates having a size of a cell aggregate smaller than the first particle size (first mass) can be moved from the culture tank 110 to the classification module 720 together with the liquid via the suction pipe nozzle 710, and can be caused to stay in the classification module 720. Furthermore, the second cell aggregates having a size equal to or larger than the first particle size (first mass) can be caused to stay in the culture tank 110 without being moved to the classification module 720.

### <CL-END (classification end)>

The control device 500-2 transmits a control signal indicating stop to the circulation heater 460-2. As a result, the circulation heater 460-2 stops.

Furthermore, the control device 500-2 transmits a control signal indicating a medium disposal state to the three-way valve 250-2. As a result, the three-way valve 250-2 becomes the medium disposal state.

The time chart illustrated in Fig. 14 illustrates an example in which the classification treatment is started following the culture treatment after all the steps of the culture treatment of the cell aggregates are completed. The classification treatment may be performed not only by this procedure but also during the culture treatment. That is, the classification treatment can be performed in parallel with the culture treatment. Note that it is not necessary to perform the classification treatment over the all the steps of the culture treatment, and the classification treatment only needs to be performed in an overlapping manner with at least some of the steps of the culture treatment. As described above, by performing the classification treatment in parallel with at least a part of the culture treatment, time required for the entire treatment related to the culture of the cell aggregates can be shortened.

### <<<Culture classification concentration recovery system 4-2>>

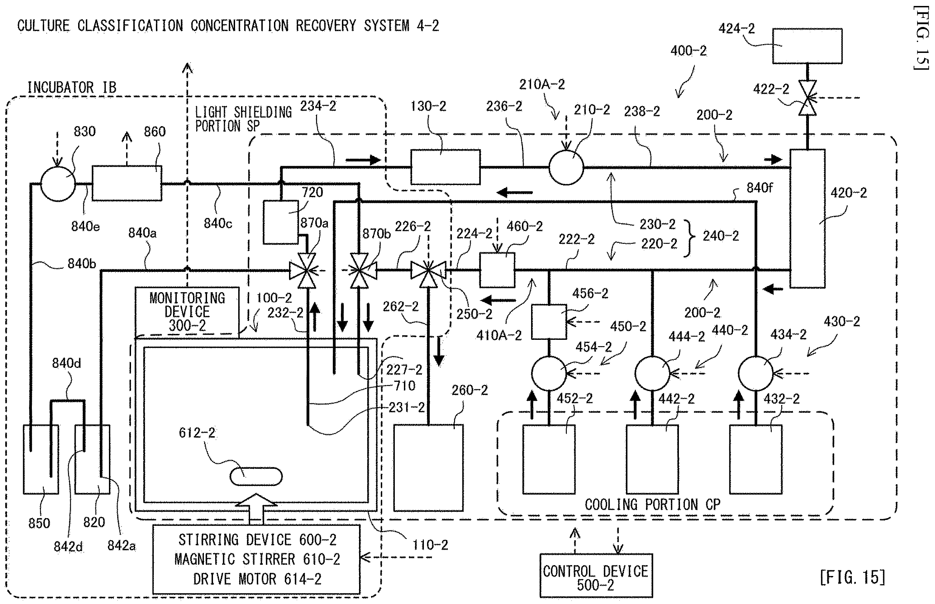

Fig. 15 is a block diagram illustrating a configuration of the culture classification concentration recovery system 4-2. The culture classification concentration recovery system 4-2 has a configuration in which the classification system 700 and the concentration recovery system 800 are incorporated into the culture system 1-2. In the culture classification concentration recovery system 4-2 illustrated in Fig. 15, components common to those of the culture system 1-2 illustrated in Fig. 3, the classification system 700 illustrated in Fig. 11, and the concentration recovery system 800 illustrated in Fig. 12 are denoted by the same reference numerals.

When the classification treatment is performed, similarly to the culture classification system 4-1, the classification treatment only needs to be performed according to the time chart illustrated in Fig. 14.

### <<Supply of pH adjuster>>

In the culture classification concentration recovery system 4-2, unlike the culture system 1-2 illustrated in Fig. 3, the pH adjuster tank 432-2 and the supply pump 434-2 are connected to a pipe 840f. The pH adjuster is directly supplied to the culture vessel 110-2 via the pipe 840f. Addition of the pH adjusters largely affects the pH of the medium in the circulation circuit, especially the pH of the new medium. Therefore, denaturation and deactivation of medium components (a protein and the like) may affect proliferation of cells. Therefore, by adopting a configuration in which the pH adjuster can be directly supplied to the culture vessel 110-2, the influence can be suppressed.

Note that, also in the culture system 1-2 illustrated in Fig. 3, similarly to the culture classification concentration recovery system 4-2, the pH adjuster tank 432-2 and the supply pump 434-2 may be connected to the pipe 840f using the pipe 840f, and the pH adjuster may be directly supplied to the culture vessel 110-2 via the pipe 840f.

### <Classification system>

Similarly to the culture classification system 4-1, also in the culture classification concentration recovery system 4-2, the suction pipe nozzle 710 and the classification module 720 are used instead of the cell-medium separation filter 120-2 in Fig. 3.

Note that, in the culture classification concentration recovery system 4-2, the classification module 720 is used instead of the cell-medium separation filter 120-2, but the cell-medium separation filter 120-2 may be used together with the classification module 720. Whether or not to use the cell-medium separation filter 120-2 can be appropriately selected.

In the culture classification concentration recovery system 4-2, the classification module 720 is connected to the suction pipe nozzle 710 via a three-way valve 870a. The three-way valve 870a is controlled by the control device 500-2. The control device 500-2 transmits a control signal indicating a culture state to the three-way valve 870a when culturing cell aggregates. As a result, the classification module 720 communicates with the suction pipe nozzle 710 and the circulation feed pipe 232-2 via the three-way valve 870a.

The control device 500-2 controls a flow rate of the circulation pump 210-2 so as to be a predetermined flow rate. By controlling the flow rate, among the cell aggregates stored in the culture vessel 110-2, the first cell aggregates having a size of a cell aggregate smaller than the first particle size (first mass) can be moved from the culture vessel 110-2 to the classification module 720 together with the liquid via the suction pipe nozzle 710, and can be caused to stay in the classification module 720. Furthermore, the second cell aggregates having a size equal to or larger than the first particle size (first mass) can be caused to stay in the culture vessel 110-2 without being moved to the classification module 720.

### <Concentration recovery system>

The concentration recovery system in the culture classification concentration recovery system 4-2 mainly includes the suction pipe nozzle 710, the recovery vessel 820, a new medium vessel 850, the pump 830, and a mass flow meter 860. Note that, in the concentration recovery system in the culture classification concentration recovery system 4-2, the suction pipe nozzle 710 is shared with the classification system.

The three-way valve 870a and the recovery vessel 820 are connected to each other by the pipe 840a. The recovery vessel 820 and the new medium vessel 850 are connected to each other by a pipe 840d. The new medium vessel 850 and the pump 830 are connected to each other by the pipe 840b. The pump 830 and the mass flowmeter 860 are connected to each other by a pipe 840e. The mass flowmeter 860 and a three-way valve 870b are connected to each other by the pipe 840c.

The pipe 840a is connected to the suction pipe nozzle 710 via the three-way valve 870a. When concentrating and recovering cell aggregates, the control device 500-2 transmits a control signal indicating a concentration recovery state to the three-way valve 870a. As a result, the classification module 720 and the pipe 840a communicate with each other via the three-way valve 870a.

In addition, the three-way valve 870b is also controlled by the control device 500-2. The control device 500-2 transmits a control signal indicating a culture state to the three-way valve 870b when culturing cell aggregates, and transmits a control signal indicating a concentration recovery state to the three-way valve 870b when concentrating and recovering the cell aggregates. When the control signal indicating a culture state is transmitted to the three-way valve 870b, the supply pipe 226-2 and the inlet 227-2 communicate with each other. When the control signal indicating a concentration recovery state is transmitted to the three-way valve 870b, the pipe 840c and the inlet 227-2 communicate with each other.

When the control device 500-2 transmits a control signal indicating operation to the pump 830, the pump 830 operates. The control device 500-2 controls a flow rate of the pump 830 so as to be a predetermined flow rate. By controlling the flow rate, among the cell aggregates stored in the culture vessel 110-2, the third cell aggregates having a size of a cell aggregate smaller than the second particle size (second mass) can be moved from the culture vessel 110-2 to the recovery vessel 820 together with the liquid via the suction pipe nozzle 710, and can be caused to stay in the recovery vessel 820. Furthermore, the fourth cell aggregates having a size equal to or larger than the second particle size (second mass) are caused to stay in the culture vessel 110-2 without being moved to the recovery vessel 820.

Furthermore, by operation of the pump 830, the medium is discharged from the recovery vessel 820 and moved to the new medium vessel 850. In the new medium vessel 850, the new medium is added and discharged from the new medium vessel 850. The medium to which the new medium is added is supplied to the culture vessel 110-2 via the pump 830 and the mass flowmeter 860, and further via the three-way valve 870b.

The mass flowmeter 860 detects a flow rate of a passing medium and transmits a signal indicating the detected flow rate to the control device 500-2. The control device 500-2 compares a preset set flow rate with the detected flow rate, and transmits a signal indicating a rotation speed of a motor of the pump 830 to the pump 830 when there is a difference between the preset set flow rate and the detected flow rate. As described above, the flow rate of the medium can be corrected by feedback control.

### <Incubator IB>

The culture classification concentration recovery system 4-2 includes an incubator IB. The incubator IB mainly includes the culture vessel 110-2, the stirring device 600-2, the classification module 720, the recovery vessel 820, and the new medium vessel 850. In this way, the classification treatment and the concentration recovery treatment can be performed in a desired temperature environment.

### <Classification (collection) treatment>

After the enlargement and culture, classification (collection) of cell aggregates using a classification system is performed. Note that, since medium circulation is performed via the classification module 720 also during the enlargement and culture, the classification (collection) is substantially performed also during the medium circulation at the time of enlargement and culture. The classified (collected) cell aggregates stay in the classification module 720, and many cell aggregates accumulate at a bottom of the classification module 720. Note that overflow occurs depending on the amount of cell aggregates to be collected, and thus it is necessary to dispose a storage portion in the classification module 720.

### <Concentration and recovery treatment>

After classification, concentration and recovery of cell aggregates using a concentration recovery system are performed. By switching the three-way valves 870a and 870b, the medium is circulated in the concentration system, and concentration and recovery are started. As the circulation starts, cell aggregates accumulate on a bottom surface of the recovery vessel 820. Note that, in order to prevent the aggregates recovered in the recovery vessel 820 from adhering to each other, a temperature of the incubator IB is set to be lower than the culture temperature to lower cell activity.

In order to supply nutrients to the cells, it is preferable to connect the new medium vessel 850 containing the new medium to the inside of the circuit of the concentration recovery system. The size of the new medium vessel 850 can be changed depending on the required amount of the new medium.

In addition, an end 842a of the pipe 840a for introducing the medium is located at a lower portion of the recovery vessel 820. On the other hand, an end 842d of the pipe 840d for leading out the medium is located at an upper portion of the recovery vessel 820. By locating the end 842a for introducing the medium and the end 842d for leading out the medium so as to be separated from each other, recovered cells can be prevented from flowing out of the recovery vessel 820. In addition, the amount of liquid after concentration is determined by the volume of the recovery vessel 820 and the position of the end 842d for leading out the medium.

### <<<Concentration recovery system 5-1>>

The culture classification concentration recovery system 4-2 has a configuration capable of continuously performing the culture treatment, the classification treatment, and the concentration recovery treatment in a single system. On the other hand, only the concentration recovery treatment may be performed alone after the classification treatment. That is, only the concentration recovery treatment may be performed in another system.

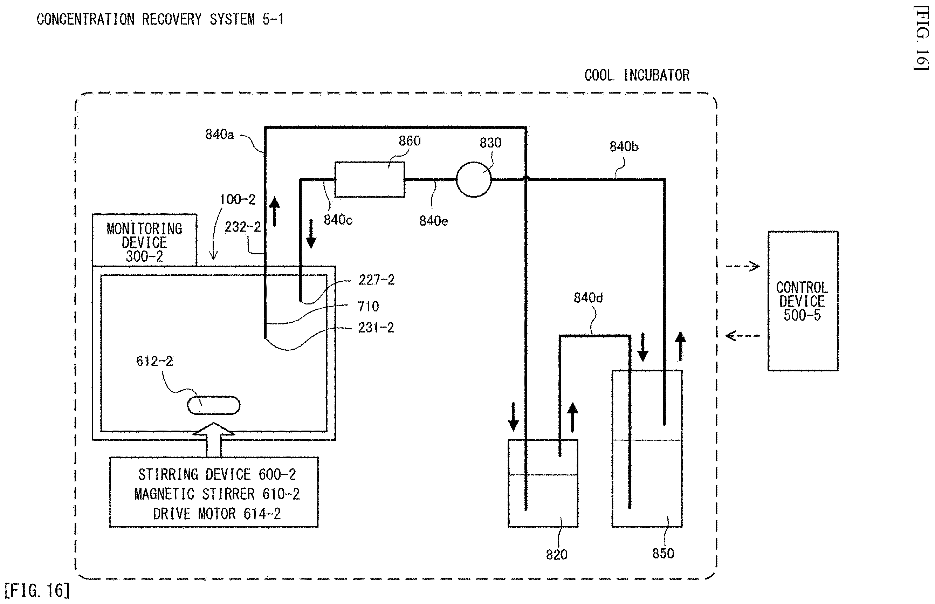

Fig. 16 is a block diagram illustrating a concentration recovery system 5-1 for culturing and classifying cell aggregates by the culture classification system 4-1 illustrated in Fig. 13, then moving the culture tank 110, and concentrating and recovering the cell aggregates. Note that components similar to those of the culture classification concentration recovery system 4-2 illustrated in Fig. 15 are denoted by the same reference numerals.

The concentration recovery system 5-1 also includes the suction pipe nozzle 810 and the recovery vessel 820.

The suction pipe nozzle 810 has a substantially cylindrical shape having a constant diameter (cross-sectional area). The suction pipe nozzle 810 has the constant cross-sectional area A1. The suction pipe nozzle 810 is disposed such that an axial direction thereof is along the vertical direction. The suction pipe nozzle 810 has an elongated shape, and is disposed such that a longitudinal direction thereof is along the vertical direction.

The recovery vessel 820 has a substantially cylindrical shape having a constant diameter (cross-sectional area). The recovery vessel 820 has a constant cross-sectional area A3. The recovery vessel 820 is disposed such that an axial direction thereof is along the vertical direction.

The diameter (cross-sectional area) of the suction pipe nozzle 810 is different from the diameter (cross-sectional area) of the recovery vessel 820. The cross-sectional area A1 of the suction pipe nozzle 810 is smaller than the cross-sectional area A3 of the recovery vessel 820. Since the diameter (cross-sectional area) of the suction pipe nozzle 810 is different from the diameter (cross-sectional area) of the recovery vessel 820, a flow speed generated in the suction pipe nozzle 810 can be made different from a flow speed generated in the recovery vessel 820 using the single pump 830.

In the concentration recovery system 5-1, a control device 500-5 controls a flow rate of the pump 830 so as to be a predetermined flow rate. By controlling the flow rate, among the cell aggregates stored in the culture vessel 110-2, the third cell aggregates having a size of a cell aggregate smaller than the second particle size (second mass) can be moved from the culture vessel 110-2 to the recovery vessel 820 together with the liquid via the suction pipe nozzle 710, and can be caused to stay in the recovery vessel 820. Furthermore, the fourth cell aggregates having a size equal to or larger than the second particle size (second mass) are caused to stay in the culture vessel 110-2 without being moved to the recovery vessel 820.

Like the concentration recovery system 5-1 illustrated in Fig. 16, only the concentration recovery system can be performed by a temperature control facility separate from an enlargement culture and classification system, for example, a cool incubator. In order to use the separate temperature control facility, it is necessary to make the culture vessel 110-2 attachable and detachable.

Therefore, in the culture classification system 4-1 illustrated in Fig. 13 and the like, it is necessary to dispose a branch portion (not illustrated) for concentration and recovery in the middle of the pipe 840a on the medium lead-out side, the pipe 840c on the medium introduction side, and the like. When the enlargement culture and classification treatment is performed, the branch portion is opened to the enlargement culture and classification side to communicate with the circuit, and the concentration recovery side is closed. On the other hand, in order to perform the concentration recovery treatment, the enlargement culture and classification side of the branch portion is closed, and the concentration recovery side is opened to communicate with the circuit. For example, after enlargement culture and classification are performed in the incubator IB of the culture classification system 4-1 illustrated in Fig. 13, the culture vessel 110-2 is removed from the circuit on the enlargement culture and classification side, and moved to the cool incubator of the concentration recovery system 5-1, and concentration and recovery can be performed.

Note that the culture vessel 110-2 may be moved to the cooling portion CP (culture classification system 4-1 in Fig. 13) in which the pH adjuster tank 432-2, the glucose concentrate tank 442-2, and the medium tank 452-2 are housed without preparing a separate cool incubator, and concentration and recovery may be performed in the cooling portion CP. The cooling portion CP of the culture classification system 4-1 can be shared, and a configuration can be simplified. In this case, the control device 500-2 can also be shared.

In addition, instead of the control device 500-5 of the concentration recovery system 5-1, the control device 500-2 of the culture classification system 4-1 may be used. The control device 500-2 can be shared, and a configuration can be simplified.

### <<Procedure of concentration recovery treatment in concentration recovery system 5-1>>

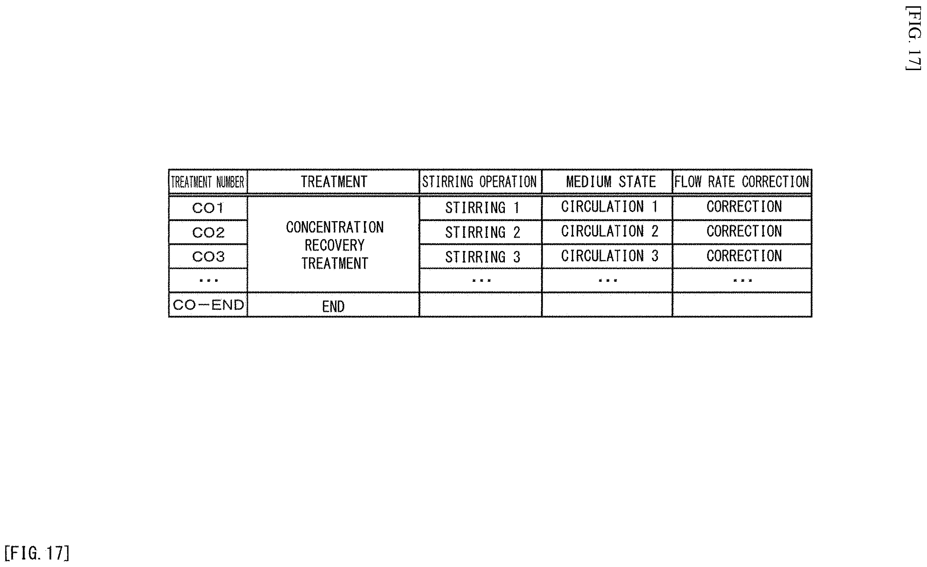

Fig. 17 is a time chart illustrating a procedure of the concentration recovery treatment. After classification by the culture classification system 4-1 illustrated in Fig. 13, stirring and circulation of the medium are performed for concentration and recovery in the concentration recovery system 5-1 illustrated in Fig. 16. Specifically, as illustrated in Fig. 17, in a step of recovery 1, stirring 1 and circulation 1 are performed, in a step of recovery 2, stirring 2 and circulation 2 are performed, and in a step of recovery 3, stirring 3 and circulation 3 are performed.

Furthermore, a flow rate is also corrected in each of steps of the recovery 1, the recovery 2, the recovery 3, .... The mass flowmeter 860 detects a flow rate of a passing medium and transmits a signal indicating the detected flow rate from the mass flowmeter 860 to the control device 500-5. The control device 500-5 compares a preset set flow rate with the detected flow rate, and transmits a signal indicating a rotation speed of a motor of the pump 830 to the pump 830 when there is a difference between the preset set flow rate and the detected flow rate. As described above, by performing the feedback control, the flow rate of the medium can be corrected in each of the steps of the recovery 1, the recovery 2, the recovery 3, ....

### <<<Culture classification concentration recovery system 6-1>>

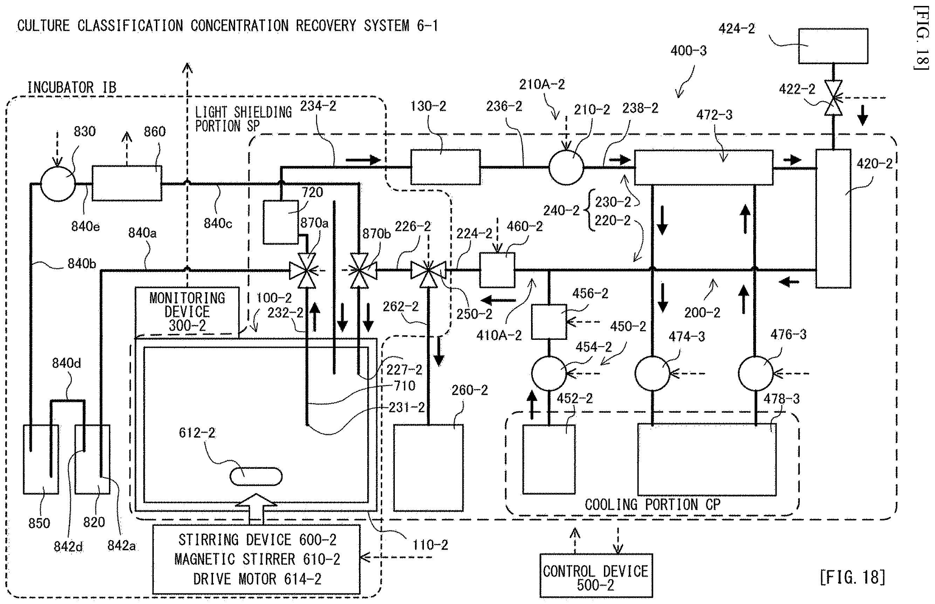

Fig. 18 is a block diagram illustrating a configuration of the culture classification concentration recovery system 6-1. The culture classification concentration recovery system 6-1 has a configuration in which the classification system 700 and the concentration recovery system 800 are incorporated into the culture system 1-3 (Fig. 8). In the culture classification concentration recovery system 6-1 illustrated in Fig. 18, components common to those of the culture system 1-3 illustrated in Fig. 8, the classification system 700 illustrated in Fig. 11, and the concentration recovery system 800 illustrated in Fig. 12 are denoted by the same reference numerals.

### <Culture treatment>

In the culture treatment of cell aggregates, similarly to the culture system 1-3, the dialysis circulation feed pump 474-3 and the dialysis supply pump 476-3 are provided. The control device 500-2 causes the dialysis circulation feed pump 474-3 and the dialysis supply pump 476-3 to operate depending on a flow rate of the circulation pump 210-2, a glucose concentration, a lactic acid concentration, and pH. Glucose, an amino acid, a vitamin, an inorganic salt, and the like can be added to the medium depending on the flow rate of the medium.

### <Classification treatment>

When the classification treatment is performed, similarly to the culture classification system 4-1 and the culture classification concentration recovery system 4-2, the classification treatment only needs to be performed according to the time chart illustrated in Fig. 14. Similarly to the culture classification system 4-1 and the culture classification concentration recovery system 4-2, also in the culture classification concentration recovery system 6-1, the classification treatment is performed using the suction pipe nozzle 710 and the classification module 720 instead of the cell-medium separation filter 120-2 in Fig. 3.

Note that, in the culture classification concentration recovery system 6-1, the classification module 720 is used instead of the cell-medium separation filter 120-2, but the cell-medium separation filter 120-2 may be used together with the classification module 720. Whether or not to use the cell-medium separation filter 120-2 can be appropriately selected.

Among the cell aggregates stored in the culture vessel 110-2, the first cell aggregates having a size of a cell aggregate smaller than the first particle size (first mass) can be moved from the culture vessel 110-2 to the classification module 720 together with the liquid via the suction pipe nozzle 710, and can be caused to stay in the classification module 720. Furthermore, the second cell aggregates having a size equal to or larger than the first particle size (first mass) can be caused to stay in the culture vessel 110-2 without being moved to the classification module 720.

### <Concentration recovery system>

The concentration recovery system in the culture classification concentration recovery system 6-1 also mainly includes the suction pipe nozzle 710, the recovery vessel 820, the new medium vessel 850, the pump 830, and the mass flow meter 860. Note that, also in the concentration recovery system in the culture classification concentration recovery system 6-1, the suction pipe nozzle 710 is shared with the classification system.

Among the cell aggregates stored in the culture vessel 110-2, the third cell aggregates having a size of a cell aggregate smaller than the second particle size (second mass) can be moved from the culture vessel 110-2 to the recovery vessel 820 together with the liquid via the suction pipe nozzle 710, and can be caused to stay in the recovery vessel 820. Furthermore, the fourth cell aggregates having a size equal to or larger than the second particle size (second mass) are caused to stay in the culture vessel 110-2 without being moved to the recovery vessel 820.

### <<<<Range of embodiments>>>>

As described above, the first embodiment and the fourth embodiment have been described. However, the description and drawings forming a part of this disclosure should not be understood as being limited. Various embodiments and the like not described herein are included.

### <<<<Aspects of invention>>>

### <<First aspect>>

A culture system according to a first aspect provides a culture system that forms a cell aggregate by liquid culture, the culture system including:
a culture portion that contains a liquid and has an outlet through which the liquid is led out and an inlet through which the liquid is introduced;
a path portion that allows the liquid to flow between the outlet and the inlet;
a monitoring portion capable of monitoring a component in the liquid and/or properties of the liquid in at least one of the culture portion and the path portion;
an adjustment portion capable of adjusting the component in the liquid and/or the properties of the liquid in at least one of the culture portion and the path portion; and
a control portion capable of controlling the adjustment portion on the basis of a monitoring result of the monitoring portion.

Since a component in a liquid or properties of the liquid is monitored and the component in the liquid or the properties of the liquid is adjusted on the basis of a monitoring result thereof, it is possible to accurately grow cells to form cell aggregates.

### <<Second aspect>>

A culture system according to a second aspect is the culture system according to the first aspect,
further including a stirring portion that stirs the liquid in the culture portion, in which
the control portion controls the stirring portion on the basis of a predetermined stirring condition.

### <<Third aspect>>

A culture system according to a third aspect is the culture system according to the second aspect, in which
the liquid culture is floating culture in which cells in the liquid are floated by stirring by the stirring portion.

### <<Fourth aspect>>

A culture system according to a fourth aspect is the culture system according to the second aspect, in which
the stirring portion includes a movable body that causes the liquid to flow.

### <<Fifth aspect>>

A culture system according to a fifth aspect is the culture system according to the second aspect, in which
the stirring portion includes a shaking mechanism that shakes the culture portion.

### <<Sixth aspect>>

A culture system according to a sixth aspect is the culture system according to the second aspect, in which
the predetermined stirring condition is a condition determined in advance on the basis of nucleation of cells and the size of the cell aggregate.

The monitoring portion monitors at least one of the component in the liquid and the properties of the liquid. As a result of monitoring, the monitoring portion outputs various types of information such as at least one piece of properties information, at least one piece of component information, and size information regarding the size of the cell aggregate. The predetermined stirring condition is determined on the basis of various types of information output from the monitoring portion.

For example, the monitoring portion includes an imaging portion that images a cell aggregate. The imaging portion outputs size information capable of determining the size of the cell aggregate. The control portion acquires the size information output from the monitoring portion. The control portion can determine the size of the cell aggregate from the size information. The control portion determines the predetermined stirring condition on the basis of the size of the cell aggregate. For example, a stirring rate is determined depending on the size of the cell aggregate.

Stirring can be performed to such an extent that shear stress applied to the cell aggregate does not increase (such an extent that a cell aggregate that has been once formed is not divided) while a possibility that cells come into contact with each other is increased.

### <<Seventh aspect>>

A culture system according to a seventh aspect is the culture system according to any one of the first to sixth aspects, further including
a thermostatic bath capable of housing at least a part of the culture portion and the path portion therein.

A cell aggregate can be accurately grown in a stable environment.

### <<Eighth aspect>>

A culture system according to an eighth aspect is the culture system according to any one of the first to seventh aspects, further including
an external heater capable of heating the liquids in the culture portion and the path portion from the outside.

By heating the liquid by the external heater, an environment suitable for forming the cell aggregate can be obtained.

### <<Ninth aspect>>

A culture system according to a ninth aspect is the culture system according to any one of the first to eighth aspects, in which
the outlet is higher than a liquid level of the liquid at the time of start of culture.

By a filter, a physical mechanism, or the like, it is possible to prevent ungrown cells and small cell aggregates from being discharged from the outlet.

### <<Tenth aspect>>

A culture system according to a tenth aspect is the culture system according to any one of the first to ninth aspects, in which
the monitoring portion is capable of outputting, as information regarding the properties of the liquid, at least one piece of properties information among information regarding a temperature of the liquid, information regarding a hydrogen ion concentration of the liquid, and information regarding a dissolved oxygen concentration in the liquid, and
the control portion acquires the at least one piece of properties information output from the monitoring portion.

The control portion can control the adjustment portion, the stirring portion, and the like using the acquired at least one piece of properties information.

### <<Eleventh aspect>>

A culture system according to an eleventh aspect is the culture system according to any one of the first to tenth aspects, in which
the monitoring portion is capable of outputting, as information regarding the component in the liquid, at least one piece of component information among information regarding an electrolyte, information regarding an amino acid, information regarding glucose, and information regarding lactic acid, and
the control portion acquires the at least one piece of component information output from the monitoring portion.

The control portion can control the adjustment portion, the stirring portion, and the like using the acquired at least one piece of component information.

### <<Twelfth aspect>>

A culture system according to a twelfth aspect is the culture system according to any one of the first to eleventh aspects, in which
the monitoring portion is capable of outputting size information regarding the size of the cell aggregate in the culture portion, and
the control portion acquires the size information output from the monitoring portion.

The control portion can control the adjustment portion, the stirring portion, and the like using the acquired size information.

### <<Thirteenth aspect>>

A culture system according to a thirteenth aspect is the culture system according to the twelfth aspect, in which
the monitoring portion includes an imaging portion that images the cell aggregate in the culture portion, and
the control portion acquires the size of the cell aggregate as the size information from imaging data of the cell aggregate.

### <<Fourteenth aspect>>

A culture system according to a fourteenth aspect is the culture system according to any one of the first to thirteenth aspects, in which
the adjustment portion includes a component adding portion that adds a necessary component to the liquid and/or a component removing portion that removes an unnecessary component from the liquid, and
the control portion controls the component adding portion and/or the component removing portion on the basis of a monitoring result of the monitoring portion to adjust the component in the liquid and/or the properties of the liquid.

The control portion controls the component adding portion and the component removing portion on the basis of the monitoring result output from the monitoring portion. By control of the component adding portion and the component removing portion, the component in the liquid or the properties of the liquid can be adjusted. By adjusting the component in the liquid or the properties of the liquid, an environment of the culture portion can be brought into a state appropriate for growth of the cell aggregate.

### <<Fifteenth aspect>>

A culture system according to a fifteenth aspect is the culture system according to any one of the first to fourteenth aspects, in which
the adjustment portion includes a gas exchange membrane capable of supplying a gas component necessary for culture to the liquid.

By supply of the gas component necessary for culture, an environment of the culture portion can be brought into a state appropriate for growth of the cell aggregate.

### <<Sixteenth aspect>>

A culture system according to a sixteenth aspect is the culture system according to the fifteenth aspect, in which
the gas exchange membrane is a hollow fiber gas exchange membrane containing any one of silicone, polypropylene, polytetrafluoroethylene, and polymethylpentene.

Since the hollow fiber gas exchange membrane is used, use for a long period of time is possible due to the homogeneous membrane that is less likely to be clogged, and the system can be downsized.

### <<Seventeenth aspect>>

A culture system according to a seventeenth aspect is the culture system according to any one of the first to sixteenth aspects, in which
the adjustment portion includes a dialysis membrane.

Since the adjustment portion includes the dialysis membrane, the medium can be continuously used for a long period of time.

### <<Eighteenth aspect>>

A culture system according to an eighteenth aspect is the culture system according to any one of the first to seventeenth aspects, in which
the control portion controls the adjustment portion on the basis of the monitoring result of the monitoring portion, and adjusts a hydrogen ion concentration of the liquid by adding a pH adjuster to the liquid.

A hydrogen ion concentration range appropriate for cell culture can be maintained.

### <<Nineteenth aspect>>

A culture system according to a nineteenth aspect is the culture system according to any one of the first to eighteenth aspects, in which
the monitoring portion is further capable of outputting information regarding a carbon dioxide concentration in a gas in contact with the liquid, and
the control portion controls the adjustment portion on the basis of the information regarding the carbon dioxide concentration of the monitoring portion, supplies carbon dioxide, and adjusts a hydrogen ion concentration of the liquid.

By also using the information regarding the carbon dioxide concentration, an environment in which cells are cultured can be accurately maintained.

### <<Twentieth aspect>>

A culture system according to a twentieth aspect is the culture system according to any one of the first to nineteenth aspects, in which
the adjustment portion includes a flow rate adjusting portion that adjusts a flow rate of each component for adjustment, and
the control portion controls the flow rate adjusting portion on the basis of a monitoring result of the monitoring portion.

Since the flow rate can be adjusted for each component, components necessary for growth of a cell aggregate can be supplied to the culture portion without excess or deficiency.

### <<Twenty-first aspect>>

A culture system according to a twenty-first aspect is the culture system according to any one of the first to twentieth aspects, in which
the adjustment portion is disposed on a panel that is attachable and detachable.

By disposing the adjustment portion on the panel that is attachable and detachable, for example, operation of exchanging a member, a component, or the like related to the adjustment portion can be easily performed.

### <<Twenty-second aspect>>

A culture system according to a twenty-second aspect is the culture system according to any one of the first to twenty-first aspects, further including a disposal portion capable of discharging the liquid from the path portion.

Since the liquid can be discharged to the disposal portion, a component in the liquid contained in the culture portion or properties of the liquid can be adjusted only to those necessary for growth of the cell aggregate. The amount of the liquid contained in the culture portion can be adjusted to an appropriate amount.

### <<Twenty-third aspect>>

A culture system according to a twenty-third aspect is the culture system according to the twenty-second aspect, further including a discharge mechanism that discharges the liquid from the path portion to the disposal portion, in which
the control portion controls the discharge mechanism to cause the liquid in the culture portion to flow to the path portion as circulation priming, and then to be discharged to the disposal portion.

Since the liquid of the culture portion is discharged by being caused to flow to the path portion as circulation priming, the liquid in the culture portion can be effectively utilized.

### <<Twenty-fourth aspect>>

A culture system according to a twenty-fourth aspect is the culture system according to any one of the first to twenty-third aspects, in which
the control portion controls the component in the liquid and/or the properties of the liquid to a predetermined value or a predetermined range on the basis of a monitoring result of the monitoring portion.

The cell aggregate can be continuously grown appropriately depending on the degree of growth of the cell aggregate and a state of the liquid.

### <<Twenty-fifth aspect>>

A culture system according to a twenty-fifth aspect is the culture system according to any one of the first to twenty-fourth aspects, in which
the control portion controls the adjustment portion to make the amount of the liquid in the culture portion larger than the amount of the liquid at the time of start of culture on the basis of progress of culture.

Since the amount of the liquid is determined depending on the size of the cell and the degree of growth of the cell aggregate, it is possible to prevent the cell and the cell aggregate from being led out from the culture portion, to cause the cell and the cell aggregate to stay in the culture portion, and to grow the cell aggregate.

### <<Twenty-sixth aspect>>

A culture system according to a twenty-sixth aspect is the culture system according to the twenty-fifth aspect, in which
the control portion performs
a first treatment of controlling the stirring portion by alternately switching between a first stirring control and a second stirring control different from the first stirring control when the amount of the liquid in the culture portion is the amount of the liquid at the time of start of culture.

By repeating both a state in which the cells are easily brought into contact with each other and the cell aggregate is easily formed and a state in which the formed cell aggregate is stabilized, the cell aggregate can be accurately grown in the culture portion.

### <<Twenty-seventh aspect>>

A culture system according to a twenty-seventh aspect is the culture system according to the twenty-sixth aspect, in which
the control portion
performs, after the first treatment, a second treatment of operating the stirring portion by the first stirring control and causing the adjustment portion to stepwise adjust the amount of the liquid in the culture portion.

The cell aggregate can be gradually grown depending on the size of the cell aggregate so as to approach a size that is not led out from the culture portion.

### <<Twenty-eighth aspect>>

A culture system according to a twenty-eighth aspect is the culture system according to the twenty-seventh aspect, in which
the control portion controls the adjustment portion, and
performs, after the second treatment, a third treatment of exchanging the liquid via the path portion when a liquid level of the liquid in the culture portion is higher than the outlet.

The cell aggregate can be grown to a size that is not led out from the culture portion, and the cell aggregate can be finally grown to a desired size by circulating the liquid.

### <<Twenty-ninth aspect>>

A classification system according to a twenty-ninth aspect provides
a classification system that classifies cell aggregates that have been subjected to culture treatment by a culture system, the classification system including:
a culture portion (for example, the culture vessel 110-2) that contains a liquid containing the cell aggregates; and
a first classification portion (for example, the suction pipe nozzle 710 or the classification module 720) that classifies the cell aggregates into first cell aggregates having a mass less than a first mass and second cell aggregates having a mass equal to or more than the first mass, and causes the second cell aggregates to stay in the culture portion.

Since the cell aggregates are classified into the first cell aggregates having a mass less than the first mass and the second cell aggregates having a mass equal to or more than the first mass, cell aggregates having a desired mass distribution (particle size distribution) can be extracted.

### <<Thirtieth aspect>>

A classification system according to a thirtieth aspect provides
a classification system that classifies cell aggregates that have been subjected to culture treatment by a culture system, the classification system including:
a culture portion (for example, the culture vessel 110-2) that contains a liquid containing the cell aggregates;
a second classification portion (for example, the suction pipe nozzle 710 or 810) that classifies the cell aggregates into third cell aggregates having a mass less than a second mass and fourth cell aggregates having a mass equal to or more than the second mass, and causes the fourth cell aggregates to stay in the culture portion; and
a recovery portion (for example, the recovery vessel 820) that recovers the third cell aggregates.

Since the cell aggregates are classified into the third cell aggregates having a mass less than the second mass and the fourth cell aggregates having a mass equal to or more than the second mass, cell aggregates having a desired mass distribution (particle size distribution) can be extracted.

Since the third cell aggregates are recovered to the recovery portion, the amount of the third cell aggregates that can be recovered can be stably increased.

### <<Thirty-first aspect>>

A classification system according to a thirty-first aspect is the classification system according to the twenty-ninth aspect, in which
the first classification portion is a classification module including a stay portion (for example, a bottom of the classification module 720) that makes stay of the first cell aggregates possible.

Since the first cell aggregates are recovered to the stay portion, the amount of the first cell aggregates that can be recovered can be stably increased.

### <<Thirty-second aspect>>

A classification system according to a thirty-second aspect is the classification system according to the twenty-ninth aspect,
further including a first return pipe (for example, the pipe 740c) that returns a liquid discharged from the first classification portion to the culture portion.

Since an environment in which a liquid stably flows is formed, classification into the first cell aggregates and the second cell aggregates can be stably performed.

### <<Thirty-third aspect>>

A classification system according to a thirty-third aspect is the classification system according to the thirtieth aspect,
further including a second return pipe (for example, the pipe 840c) that returns a liquid discharged from the second classification portion to the culture portion.

An environment in which a liquid stably flows is formed, and classification into the third cell aggregates and the fourth cell aggregates can be stably performed.

### <<Thirty-fourth aspect>>

A classification system according to a thirty-fourth aspect is the classification system according to the twenty-ninth aspect, in which
in the first classification portion, the cell aggregates can be displaced along with a flow of the liquid against gravity sedimentation of the cell aggregates.

Since a certain force is applied to the cell aggregates by using gravity sedimentation, classification can be stably performed.

### <<Thirty-fifth aspect>>

A classification system according to a thirty-fifth aspect is the classification system according to the thirtieth aspect, in which
in the second classification portion, the cell aggregates can be displaced along with a flow of the liquid against gravity sedimentation of the cell aggregates.

### <<Thirty-sixth aspect>>

A classification system according to a thirty-sixth aspect is the classification system according to the twenty-ninth aspect, in which
the cell aggregates are classified into the first cell aggregates and the second cell aggregates during culture of the cell aggregates.

Since classification is performed during culture, culture and classification can be performed in parallel, and classification can be performed efficiently and quickly.

### <<Thirty-seventh aspect>>

A classification system according to a thirty-seventh aspect is the classification system according to the twenty-ninth aspect, including:
a second classification portion (for example, the suction pipe nozzle 710 or 810) that classifies the cell aggregates into third cell aggregates having a mass less than a second mass and fourth cell aggregates having a mass equal to or more than the second mass after the first classification portion classifies the cell aggregates into the first cell aggregates and the second cell aggregates, and causes the fourth cell aggregates to stay in the culture portion; and
a recovery portion (for example, the recovery vessel 820) that recovers the third cell aggregates.

Following the classification into the first cell aggregates and the second cell aggregates, the classification into the third cell aggregates and the fourth cell aggregates can be performed continuously, and the classification can be performed quickly and smoothly.

### Reference Signs List

1, 1-1, 1-2, 1-3 Culture system
100-1, 100-2 Culture device
200-1, 200-2 Path structure
300-1, 300-2 Monitoring device
400-1, 400-2 Adjustment device
500-1, 500-2 Control device
600-1, 600-2 Stirring device
700 Classification system
710 Suction pipe nozzle
720 Classification module
800 Concentration recovery system
810 Suction pipe nozzle
820 Recovery vessel
850 New medium vessel

## Claims

1. A culture system that forms a cell aggregate by liquid culture, the culture system comprising:
a culture portion that contains a liquid and has an outlet through which the liquid is led out and an inlet through which the liquid is introduced;
a path portion that allows the liquid to flow between the outlet and the inlet;
a monitoring portion capable of monitoring a component in the liquid and/or properties of the liquid in at least one of the culture portion and the path portion;
an adjustment portion capable of adjusting the component in the liquid and/or the properties of the liquid in at least one of the culture portion and the path portion; and
a control portion capable of controlling the adjustment portion on a basis of a monitoring result of the monitoring portion, wherein
the control portion
performs a liquid adjustment amount treatment that causes the adjustment portion to stepwise adjust an amount of the liquid in the culture portion.

2. The culture system according to claim 1, wherein the outlet is higher than a liquid level of the liquid at a time of start of culture.

3. The culture system according to claim 2, wherein
the control portion controls the adjustment portion, and
performs, after the liquid adjustment amount treatment, a liquid exchange treatment of exchanging the liquid via the path portion when a liquid level of the liquid in the culture portion is higher than the outlet.

4. The culture system according to claim 2 or 3, further comprising a stirring portion that stirs the liquid in the culture portion, wherein
the control portion controls the stirring portion on a basis of a predetermined stirring condition.

5. The culture system according to claim 4, wherein
the control portion performs,
before performing the liquid adjustment amount treatment,
a stirring portion control treatment of controlling the stirring portion by alternately switching between a first stirring control and a second stirring control different from the first stirring control when an amount of the liquid in the culture portion is an amount of the liquid at a time of start of culture.

6. The culture system according to claim 4 or 5, wherein the liquid culture is floating culture in which cells in the liquid are floated by stirring by the stirring portion.

7. The culture system according to claim 4 or 5, wherein the stirring portion includes a movable body that causes the liquid to flow.

8. The culture system according to claim 4 or 5, wherein the stirring portion includes a shaking mechanism that shakes the culture portion.

9. The culture system according to claim 4 or 5, wherein the predetermined stirring condition is a condition determined in advance on a basis of nucleation of cells and a size of the cell aggregate.

10. The culture system according to any one of claims 2 to 5, further comprising a thermostatic bath capable of housing at least a part of the culture portion and the path portion therein.

11. The culture system according to any one of claims 2 to 5, further comprising an external heater capable of heating the liquids in the culture portion and the path portion from an outside.

12. The culture system according to any one of claims 2 to 5, wherein the monitoring portion is capable of outputting, as information regarding the properties of the liquid, at least one piece of properties information among information regarding a temperature of the liquid, information regarding a hydrogen ion concentration of the liquid, and information regarding a dissolved oxygen concentration in the liquid, and
the control portion acquires the at least one piece of properties information output from the monitoring portion.

13. The culture system according to any one of claims 2 to 5, wherein the monitoring portion is capable of outputting, as information regarding the component in the liquid, at least one piece of component information among information regarding an electrolyte, information regarding an amino acid, information regarding glucose, and information regarding lactic acid, and
the control portion acquires the at least one piece of component information output from the monitoring portion.

14. The culture system according to any one of claims 2 to 5, wherein the monitoring portion is capable of outputting size information regarding a size of the cell aggregate in the culture portion, and
the control portion acquires the size information output from the monitoring portion.

15. The culture system according to claim 15, wherein the monitoring portion includes an imaging portion that images the cell aggregate in the culture portion, and
the control portion acquires the size of the cell aggregate as the size information from imaging data of the cell aggregate.

16. The culture system according to any one of claims 2 to 5, wherein
the adjustment portion includes a component adding portion that adds a necessary component to a liquid and/or a component removing portion that removes an unnecessary component from the liquid, and
the control portion controls the component adding portion and/or the component removing portion on a basis of a monitoring result of the monitoring portion to adjust the component in the liquid and/or the properties of the liquid.

17. The culture system according to any one of claims 2 to 5, wherein the adjustment portion includes a gas exchange membrane capable of supplying a gas component necessary for culture to the liquid.

18. The culture system according to claim 18, wherein the gas exchange membrane is a hollow fiber gas exchange membrane containing any one of silicone, polypropylene, polytetrafluoroethylene, and polymethylpentene.

19. The culture system according to any one of claims 2 to 5, wherein the adjustment portion includes a dialysis membrane.

20. The culture system according to claim 13, wherein the control portion controls the adjustment portion on a basis of a monitoring result of the monitoring portion, and adjusts a hydrogen ion concentration of the liquid by adding a pH adjuster to the liquid.

21. The culture system according to claim 13, wherein the monitoring portion is further capable of outputting information regarding a carbon dioxide concentration in a gas in contact with the liquid, and
the control portion controls the adjustment portion on a basis of the information regarding the carbon dioxide concentration of the monitoring portion, supplies carbon dioxide, and adjusts a hydrogen ion concentration of the liquid.

22. The culture system according to any one of claims 2 to 5, wherein the adjustment portion includes a flow rate adjusting portion that adjusts a flow rate of each component for adjustment, and
the control portion controls the flow rate adjusting portion on a basis of a monitoring result of the monitoring portion.

23. The culture system according to any one of claims 2 to 5, further comprising a panel attachable to and detachable from the adjustment portion.

24. The culture system according to any one of claims 2 to 5, further comprising a disposal portion capable of discharging the liquid from the path portion.

25. The culture system according to claim 25, further comprising a discharge mechanism that discharges the liquid from the path portion to the disposal portion, wherein
the control portion controls the discharge mechanism to cause the liquid in the culture portion to flow to the path portion as circulation priming, and then to be discharged to the disposal portion.

26. The culture system according to any one of claims 2 to 5, wherein the control portion controls the component in the liquid and/or the properties of the liquid to a predetermined value or a predetermined range on a basis of a monitoring result of the monitoring portion.

27. The culture system according to any one of claims 2 to 5, wherein the control portion controls the adjustment portion to make an amount of the liquid in the culture portion larger than an amount of the liquid at a time of start of culture on a basis of progress of culture.

28. A classification system that classifies cell aggregates that have been subjected to culture treatment by the culture system according to any one of claims 1 to 5, the classification system comprising:
a culture portion that contains a liquid containing the cell aggregates; and
a first classification portion that classifies the cell aggregates into first cell aggregates having a mass less than a first mass and second cell aggregates having a mass equal to or more than the first mass, and causes the second cell aggregates to stay in the culture portion.

29. A classification system that classifies cell aggregates that have been subjected to culture treatment by the culture system according to any one of claims 1 to 5, the classification system comprising:
a culture portion that contains a liquid containing the cell aggregates;
a second classification portion that classifies the cell aggregates into third cell aggregates having a mass less than a second mass and fourth cell aggregates having a mass equal to or more than the second mass, and causes the fourth cell aggregates to stay in the culture portion; and
a recovery portion that recovers the third cell aggregates.

30. The classification system according to claim 29, wherein the first classification portion is a classification module including a stay portion that makes stay of the first cell aggregates possible.

31. The classification system according to claim 29, further comprising a first return pipe that returns a liquid discharged from the first classification portion to the culture portion.

32. The classification system according to claim 30, further comprising a second return pipe that returns a liquid discharged from the second classification portion to the culture portion.

33. The classification system according to claim 29, wherein in the first classification portion, the cell aggregates are capable of being displaced along with a flow of a liquid against gravity sedimentation of the cell aggregates.

34. The classification system according to claim 30, wherein in the second classification portion, the cell aggregates are capable of being displaced along with a flow of a liquid against gravity sedimentation of the cell aggregates.

35. The classification system according to claim 29, wherein the cell aggregates are classified into the first cell aggregates and the second cell aggregates during culture of the cell aggregates.

36. The classification system according to claim 29, comprising:
a second classification portion that classifies the cell aggregates into third cell aggregates having a mass less than a second mass and fourth cell aggregates having a mass equal to or more than the second mass after the first classification portion classifies the cell aggregates into the first cell aggregates and the second cell aggregates, and causes the fourth cell aggregates to stay in the culture portion; and
a recovery portion that recovers the third cell aggregates.
